(19) Europäisches Patentamt / European Patent Office / Office européen des brevets

(11) **EP 4 344 699 A2**

(12) **EUROPEAN PATENT APPLICATION**

(43) Date of publication:
**03.04.2024 Bulletin 2024/14**

(21) Application number: **23201047.0**

(22) Date of filing: **29.09.2023**

(51) International Patent Classification (IPC):
*A61K 9/00* (2006.01)   *A61K 38/00* (2006.01)
*A61K 39/00* (2006.01)   *A61K 47/10* (2017.01)
*A61K 47/18* (2017.01)   *A61K 47/26* (2006.01)
*A61K 47/32* (2006.01)   *A61K 47/36* (2006.01)
*A61K 47/40* (2006.01)

(52) Cooperative Patent Classification (CPC):
**A61K 9/0053; A61K 9/0019; A61K 9/0043;
A61K 38/00; A61K 39/00; A61K 47/10;
A61K 47/183; A61K 47/26; A61K 47/32;
A61K 47/36; A61K 47/40**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.09.2022 US 202263411460 P
12.05.2023 US 202363466053 P**

(71) Applicant: **The Board of Regents of the University of
Texas System
Austin, TX 78701 (US)**

(72) Inventors:
• **Doan, Trang
Austin, Texas, 78712 (US)**
• **Croyle, Maria
Austin, Texas, 78712 (US)**

(74) Representative: **Potter Clarkson
Chapel Quarter
Mount Street
Nottingham NG1 6HQ (GB)**

(54) **METHODS AND COMPOSITIONS FOR TRANSPORT, STORAGE, AND DELIVERY OF NUCLEIC ACIDS AND OTHER MOLECULES**

(57)   Aspects of the present disclosure are directed to liquid and substantially solid film compositions for storage, transport, and administration of agents, including nucleic acids, without the use of ultralow temperatures. Provided are compositions capable of long-term storage of nucleic acids at ambient temperatures. Also disclosed are injectable or oral compositions comprising nucleic acids, as well as methods for formulation and administration of such compositions.

**EP 4 344 699 A2**

## Description

## CROSS-REFERENCE TO RELATED APPLICATIONS

This application claims the benefit of priority to U.S. Provisional Patent Application Serial No. 63/466,053, filed May 12, 2023, and U.S. Provisional Patent Application Serial No. 63/411,460, filed September 29, 2022, each of which is hereby incorporated by reference in its entirety.

## BACKGROUND

### I. Technological Field

Aspects of the disclosure relate to at least the fields of biotechnology and pharmaceutical chemistry. More particularly, aspects relate to compositions and methods for storage, preservation, and delivery of biological materials, including agents such as nucleic acids.

### II. Background

Recombinant DNA (e.g., gene-containing plasmid DNA (pDNA)) and RNA (e.g., mRNA) are crucial research tools and potential delivery vectors in gene therapy and vaccination. However, many current nucleic acid products are formulated as liquid products that are stored and shipped at ultralow temperatures because such nucleic acids are prone to alteration and degradation which compromise their biological functions. Therefore, current approaches present significant logistic and economic issues with respect to global distribution and access to life-saving medicines.

As part of a project to improve global distribution of vaccines and other biological drugs, a process was previously developed to stabilize live viral vectors within a film matrix. The resulting film can be shipped and stored at ambient temperatures for significant periods of time without compromising potency, and solutions prepared from rehydrated film can be administered by injection and needle-free routes. However, initial experiments revealed that the film matrix optimized for recombinant viruses was not physically compatible with naked plasmid DNA or lipid nanoparticles comprising a nucleic acid (e.g., mRNA).

Accordingly, recognized is a need for compositions and methods for storage, maintenance, and delivery (e.g., *via* intravenous delivery) of stabilized agents, such as nucleic acids (e.g., plasmid DNA, mRNA), without the need for ultralow temperatures.

## SUMMARY

Aspects of the present disclosure address certain needs in the art by providing liquid and film compositions capable of long-term storage of agents (e.g., therapeutic agents), such as nucleic acids, at temperatures above freezing, including refrigeration (e.g., 4 °C) and ambient temperatures (e.g., 25 °C), while retaining biological function and efficacy. Accordingly, certain aspects are directed to compositions comprising an agent (e.g., a therapeutic agent) (e.g., a nucleic acid) and a carrier, where the carrier enables long-term storage of the agents (e.g., therapeutic agents). As disclosed herein, in some cases, compositions comprising a carrier including a sugar, a zwitterionic compound, and a base polymer may be particularly conducive for generation of stable DNA formulations. In some cases, compositions comprising a plasticizer and/or a surfactant and a base polymer may be conducive for generation of stable RNA formulations. Thus, some aspects of the disclosure include compositions comprising an agent in a substantially solid carrier comprising at least a sugar, a zwitterionic compound, and a base polymer, and some aspects of the disclosure include compositions comprising an agent in a substantially solid carrier comprising at least a plasticizer and/or a surfactant and a base polymer. Also disclosed are methods for generating such compositions, methods for storage of such compositions, and methods of administration of such compositions, e.g., administration to a patient for therapeutic purposes including gene therapy.

Disclosed herein, in some aspects, is a composition comprising an agent (e.g., a therapeutic agent) in a carrier comprising a sugar, a zwitterionic compound, and a base polymer. In some aspects, the sugar is glucose, dextrose, fructose, lactose, maltose, xylose, sucrose, corn sugar syrup, sorbitol, hexitol, maltitol, xylitol, mannitol, melezitose, raffinose, cyclodextrin or a combination thereof. Various cyclodextrins are disclosed herein and include alpha-cyclodextrin, beta-cyclodextrin, gamma-cyclodextrin, hydroxypropyl-beta-cyclodextrin, and methoxy-beta-cyclodextrin. Any one of the sugars disclosed herein may be excluded from a composition of the disclosure in certain aspects. In some aspects, the sugar is between 0.1% and 5.0%, e.g., at least, at most, equal to, or between any two of 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the carrier, or the carrier comprises at least, at most, equal to, or between any two of 0.1% and 5.0% of a sugar, e.g., 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the sugar. In some aspects, the carrier comprises at least, at most, equal to, or between 0.1% and 3.0% of a sugar. In specific aspects, the sugar is trehalose, and the carrier comprises about 1.0% trehalose. In specific aspects, the sugar is sucrose, and the carrier comprises about 0.5% sucrose. In specific aspects, the sugar is sorbitol, and the carrier comprises about 0.5% or about 1.0% sorbitol. In specific aspects, the sugar is a combination of trehalose and sucrose, and the carrier comprises about 1.0% trehalose and about 0.5% sucrose. In specific aspects, the sugar is a combination of trehalose and sorbitol, and the carrier comprises about 1.0% trehalose and about 0.5% sorbitol. In a preferred embodiment, a given % value in respect of the sugar component of the composition as described in this paragraph, and/or elsewhere in the present application, refers to a % weight to volume (w/v), that is the % weight of the sugar relative to the volume of the carrier, within the composition. For example, the value of 1% (w/v) of a sugar may correspond to 1g sugar per 100ml of the carrier in the case of a liquid composition, or 1 g sugar per $100cm^3$ of the carrier in the case of a solid (or substantially solid) composition. In a alternative embodiment, a given % value in respect of the sugar component of the composition as described in this paragraph, and/or elsewhere in the present application, refers to a % weight to weight (w/w), that is the % weight of the sugar relative to the weight of whole composition. For example, the value of 1% (w/w) of a sugar may correspond to 1g sugar per 100g of the composition.

In some aspects, the zwitterionic compound is arginine and/or ethylenediaminetetraacetic acid (EDTA). In some aspects, the zwitterionic compound is between 0.1% and 5.0%, e.g., at least, at most, equal to, or between any two of 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the carrier, or the carrier comprises at least, at most, equal to, or between any two of 0.1% and 5.0% of the zwitterionic compound, e.g., 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the zwitterionic compound. In some aspects, the carrier comprises at least, at most, equal to, or between 0.1% and 2.0% of the zwitterionic compound. In specific aspects, the zwitterionic compound is arginine, and the carrier comprises about 0.1% arginine or about 1.0% arginine. In specific aspects, the zwitterionic compound is EDTA, and the carrier comprises about 1 mM EDTA or 2 mM EDTA. In specific aspects, the zwitterionic compound is a combination of arginine and EDTA, and the carrier comprises about 0.1% arginine or about 1.0% arginine and about 1 mM EDTA or 2 mM EDTA. In a preferred embodiment, a given % value in respect of the zwitterionic compound component of the composition as described in this paragraph, and/or elsewhere in the present application, refers to a % weight to volume (w/v), that is the % weight of the zwitterionic compound relative to the volume of the carrier, within the composition. For example, the value of 1% (w/v) of a zwitterionic compound may correspond to 1g zwitterionic compound per 100ml of the carrier in the case of a liquid composition, or 1 g zwitterionic compound per $100cm^3$ of the carrier in the case of a solid (or substantially solid) composition. In a alternative embodiment, a given % value in respect of the zwitterionic compound component of the composition as described in this paragraph, and/or elsewhere in the present application, refers to a % weight to weight (w/w), that is the % weight of the zwitterionic compound component relative to the weight of whole composition. For example, the value of 1% (w/w) of a zwitterionic compound component may correspond to 1g of the zwitterionic compound component per 100g of the composition.

In some aspects, the base polymer is hydroxypropyl methylcellulose (HPMC), polyvinyl pyrrolidone (PVP), polyvinyl alcohol (PVA), gelatin, or a combination thereof. Any one of the base polymers disclosed herein may be excluded from a composition of the disclosure in certain aspects. In some aspects, the base polymer is between 0.1% and 5.0%, e.g., at least, at most, equal to, or between any two of 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the carrier, or the carrier comprises at least, at most, equal to, or between any two of 0.1% and 5.0% of the base polymer, e.g., 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the base polymer. In some aspects, the carrier comprises at least, at most, equal to, or between 0.5% and 3.0% of the base polymer. In specific aspects, the base polymer is HPMC, and the carrier comprises about 0.75% to 2.0% of the HPMC, e.g., at least, at most, equal to, or between any two of 0.75%, 0.875%, 1%, 1.125%, 1.25%, 1.375%, 1.5%, 1.625%, 1.75%, 1.875%, or 2% HPMC. In a preferred embodiment, a given % value in respect of the base polymer component of the composition as described in this paragraph, and/or elsewhere in the present application, refers to a % weight to volume (w/v), that is the % weight of the base polymer relative to the volume of the carrier, within the composition. For example, the value of 1% (w/v) of a base polymer may correspond to 1g base polymer per 100ml of the carrier in the case of a liquid composition, or 1 g base polymer per 100cm$^3$ of the carrier in the case of a solid (or substantially solid) composition. In a alternative embodiment, a given % value in respect of the base polymer component of the composition as described in this paragraph, and/or elsewhere in the present application, refers to a % weight to weight (w/w), that is the % weight of the base polymer component relative to the weight of whole composition. For example, the value of 1% (w/w) of a base polymer may correspond to 1g of the base polymer per 100g of the composition. Various HPMC grades are disclosed herein and include A4C, A4M, A15C, A15LV, E4M, E6LV, F4M, K4M, and K100LV. In specific aspects, the HPMC is K100LV. In specific aspects, the HPMC is K4M. Any one of the HPMC grades disclosed herein may be excluded from a composition of the disclosure in certain aspects. In certain aspects, the HPMC has a molecular weight that produces a viscosity that is less than 4000, 3500, 3000, 2500, 2000, 1800, 1500, 1200, 1000, 800, 600, 500, 400, 300, 200, 100, 50, 20, 15, 14, 13, 12, 11, 10, 9, 8, 7, 6, or 5 centipoise (cp) at a concentration of 2% in water, including any range or value derivable therein. As disclosed herein, HPMC viscosity is described in terms of viscosity at a concentration of the HPMC of 2% in water, unless otherwise indicated. In specific aspects, the base polymer is PVA, and the carrier comprises about 2.0% PVA. In specific aspects, the base polymer is gelatin, and the carrier comprises about 2.0% gelatin. The gelatin may have a pH of between 5.0 to 9.0, e.g., at least, at most, equal to, or between any two of 5.0, 6.0, 7.0, 8.0, or 9.0.

Aspects of the disclosure relate to a composition comprising between 0.001 mg/mL and 1 mg/mL of an agent (e.g., therapeutic agent) (e.g., DNA) formulated within from about 0.1% to about 3.0% of a sugar; from about 0.1% to about 2.0% of a zwitterionic compound; and from about 0.5% and 3.0% of a base polymer. Aspects of the disclosure relate to a composition comprising an agents (e.g., therapeutic agent) (e.g., DNA) in a substantially solid carrier comprising a sugar, a zwitterionic compound, and a base polymer, wherein: the sugar is melezatose, trehalose, raffinose, sucrose, dextrose, mannitol, sorbitol, cyclodextrin, or a combination thereof; the zwitterionic compound is arginine and/or ethylenediaminetetraacetic acid (EDTA); and the base polymer is hydroxypropyl methylcellulose (HPMC), polyvinyl pyrrolidone (PVP), polyvinyl alcohol (PVA), gelatin, or a combination thereof. Aspects of the disclosure relate to a pharmaceutical composition generated by combining a composition described herein and a pharmaceutically acceptable carrier (e.g., to dilute the composition and/or further prepare for administration to a subject).

Disclosed herein, in some aspects, is a composition comprising an agent (e.g., a therapeutic agent) in a carrier comprising a plasticizer and/or a surfactant and a base polymer. In some aspects, the plasticizer is between 0.1% and 5.0%, e.g., at least, at most, equal to, or between any two of 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the carrier, or the carrier comprises between 0.1% and 5.0% of the plasticizer, e.g., at least, at most, equal to, or between any two of 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the plasticizer. In a preferred embodiment, a given % value in respect of the plasticizer component of the composition as described in this paragraph, and/or elsewhere in the present application, refers to a % weight to volume (w/v), that is the % weight of the plasticizer relative to the volume of the carrier, within the composition. For example, the value of 1% (w/v) of a plasticizer may correspond to 1g plasticizer per 100ml of the carrier in the case of a liquid composition, or 1 g plasticizer per 100cm$^3$ of the carrier in the case of a solid (or substantially solid) composition. In a alternative embodiment, a given % value in respect of the plasticizer component of the composition as described in this paragraph, and/or elsewhere in the present application, refers to a % weight to weight (w/w), that is the % weight of the plasticizer component relative to the weight of whole composition. For example, the value of 1% (w/w) of a plasticizer may correspond to 1g of the plasticizer per 100g of the composition. Various plasticizers are contemplated for use in the compositions disclosed herein and generally include low molecular weight compounds that can be added to a polymer to improve its processability, flexibility, and/or stretchability by modifying the mechanical properties of a composition (e.g., improving flexibility or ductility and/or lowering melt viscosity of the composition without altering the fundamental chemical character of the composition). Any one of the plasticizers disclosed herein may be excluded from a composition of the disclosure in certain aspects. In some aspects, the plasticizer is glycerol, and the carrier comprises about 3.0% of the plasticizer.

In some aspects, the surfactant is a zwitterionic and/or a non-ionic surfactant. In some aspects, the zwitterionic surfactant is PMAL-C16. In some aspects, the zwitterionic surfactant is not PMAL-C16. In some aspects, the non-ionic surfactant a poloxamer. In some aspects, the surfactant is between 0.0001% and 3.0%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, or 3.0% of the carrier, or the carrier comprises at least, at most, equal to, or between any two of 0.0001% and 3.0% of the surfactant, e.g., 0.0001%, 0.0002%, 0.0003%, 0.0004%, 0.0005%, 0.0006%, 0.0007%, 0.0008%, 0.0009%, 0.001%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, or 3.0% of the surfactant. In a preferred embodiment, a given % value in respect of the surfactant component of the composition as described in this paragraph, and/or elsewhere in the present application, refers to a % weight to volume (w/v), that is the % weight of the surfactant relative to the volume of the carrier, within the composition. For example, the value of 1% (w/v) of a surfactant may correspond to 1g surfactant per 100ml of the carrier in the case of a liquid composition, or 1 g surfactant per 100cm$^3$ of the carrier in the case of a solid (or substantially solid) composition. In a alternative embodiment, a given % value in respect of the surfactant component of the composition as described in this paragraph, and/or elsewhere in the present application, refers to a % weight to weight (w/w), that is the % weight of the surfactant component relative to the weight of whole composition. For example, the value of 1% (w/w) of a surfactant may correspond to 1g of the surfactant per 100g of the composition. Various poloxamers are disclosed herein and include polyoxypropylenepolyoxyethylene copolymers, e.g., lineal (e.g., LUTROL® or PLURONIC® copolymers, e.g., LUTROL® F-127 and PLURONIC® F68, F108, F127, L61, L122, P85, P94, P105, or P123) or X-shaped (e.g., TETRONIC® copolymers, e.g., TETRONIC® 304, 904, 908, 1304, 1307) amphiphilic triblock copolymers, having the ability of to self-assemble into micelles in aqueous solution at concentrations higher than the critical micellar concentration (CMC). Any one of the poloxamers disclosed herein may be excluded from a composition of the disclosure in certain aspects. In specific aspects, the poloxamer is poloxamer 188 (i.e., PLURONIC® F-68) or poloxamer 407 (i.e., PLURONIC® F-127). In specific aspects, the surfactant is poloxamer 407, and the carrier comprises about 0.01% poloxamer 407. In specific aspects, the surfactant is poloxamer 188 and poloxamer 407, and the carrier comprises about 0.012% poloxamer (e.g., 0.006% poloxamer 188 and 0.006% poloxamer 407).

In some aspects, the base polymer is hydroxypropyl methylcellulose (HPMC), polyvinyl pyrrolidone (PVP), polyvinyl alcohol (PVA), gelatin, or a combination thereof. Any one of the base polymers disclosed herein may be excluded from a composition of the disclosure in certain aspects. In some aspects, the base polymer is between 0.1% and 5.0%, e.g., at least, at most, equal to, or between any two of 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the carrier, or the carrier comprises at least, at most, equal to, or between any two of 0.1% and 5.0% of the base polymer, e.g., 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9, or 5.0% of the base polymer. In some aspects, the carrier comprises at least, at most, equal to, or between 0.5% and 3.0% of the base polymer. In specific aspects, the base polymer is HPMC, and the carrier comprises about 0.75% to 2.0% of the HPMC, e.g., at least, at most, equal to, or between any two of 0.75%, 0.875%, 1%, 1.125%, 1.25%, 1.375%, 1.5%, 1.625%, 1.75%, 1.875%, or 2% HPMC. Various HPMC grades are disclosed herein and include A4C, A4M, A15C, A15LV, E4M, E6LV, F4M, K4M, and K100LV. In specific aspects, the HPMC is K100LV. In specific aspects, the HPMC is K4M. Any one of the HPMC grades disclosed herein may be excluded from a composition of the disclosure in certain aspects. In certain aspects, the HPMC has a molecular weight that produces a viscosity that is less than 4000, 3500, 3000, 2500, 2000, 1800, 1500, 1200, 1000, 800, 600, 500, 400, 300, 200, 100, 50, 20, 15, 14, 13, 12, 11, 10, 9, 8, 7, 6, or 5 centipoise (cp) at a concentration of 2% in water, including any range or value derivable therein. As disclosed herein, HPMC viscosity is described in terms of viscosity at a concentration of the HPMC of 2% in water, unless otherwise indicated. In specific aspects, the base polymer is PVA, and the carrier comprises about 2.0% PVA. In specific aspects, the base polymer is gelatin, and the carrier comprises about 2.0% gelatin. The gelatin may have a pH of between 5.0 to 9.0, e.g., at least, at most, equal to, or between any two of 5.0, 6.0, 7.0, 8.0, or 9.0.

In some aspects, the carrier further comprises a pegylated lipid. In some aspects, the pegylated lipid is between 0.0001% and 3.0%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, or 3.0% of the carrier, or the carrier comprises at least, at most, equal to, or between any two of 0.0001% and 3.0% of the pegylated lipid, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, or 3.0% of the pegylated lipid. In a preferred embodiment, a given % value in respect of the pegylated lipid component of the composition as described in this paragraph, and/or elsewhere in the present application, refers to a % weight to volume (w/v), that is the % weight of the pegylated lipid relative to the volume of the carrier, within the composition. For example, the value of 1% (w/v) of a pegylated lipid may correspond to 1g pegylated lipid per 100m1 of the carrier in the case of a liquid composition, or 1 g pegylated lipid per $100cm^3$ of the carrier in the case of a solid (or substantially solid) composition. In a alternative embodiment, a given % value in respect of the pegylated lipid component of the composition as described in this paragraph, and/or elsewhere in the present application, refers to a % weight to weight (w/w), that is the % weight of the pegylated lipid component relative to the weight of whole composition. For example, the value of 1% (w/w) of a pegylated lipid may correspond to 1g of the pegylated lipid per 100g of the composition. In specific aspects, the pegylated lipid comprises DMPE-PEG and/or DMG-PEG. Any one or more of the pegylated lipids disclosed herein may be excluded from a composition of the disclosure in certain aspects.

Aspects of the disclosure relate to a composition comprising between 0.001 mg/mL and 1 mg/mL of an agent (e.g., a therapeutic agent) (e.g., RNA) formulated within from about 1.0% to about 5.0% of a plasticizer; from about 0.0001% to about 3.0% of a surfactant; and/or from about 0.5% and 3.0% of a base polymer. Aspects of the disclosure relate to a composition comprising an agent (e.g., a therapeutic agent) (e.g., RNA) in a substantially solid carrier comprising a plasticizer and/or a surfactant and a base polymer, wherein: the plasticizer is glycerol; the surfactant is a poloxamer; and the base polymer is hydroxypropyl methylcellulose (HPMC), polyvinyl pyrrolidone (PVP), polyvinyl alcohol (PVA), gelatin, or a combination thereof. Aspects of the disclosure relate to a pharmaceutical composition generated by combining a composition described herein and a pharmaceutically acceptable carrier (e.g., to dilute the composition and/or further prepare for administration to a subject).

In some aspects, the compositions disclosed herein further comprise one or more excipients. In some aspects, the one or more excipients can include choline, phosphocholine, calcium D-heptagluconate dihydrate, additional surfactants, additional plasticizers, additional sugars, and/or additional polymers. In some aspects, the choline and/or the phosphocholine is between 0.0001% and 10%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the carrier, or the carrier comprises between 0.0001 % and 10% of the choline and/or the phosphocholine, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the choline and/or the phosphocholine. In a preferred embodiment, a given % value in respect of the choline and/or the phosphocholine component of the composition as described in this paragraph, and/or elsewhere in the present application, refers to a % weight to volume (w/v), that is the % weight of the choline and/or the phosphocholine relative to the volume of the carrier, within the composition. For example, the value of 1% (w/v) of choline and/or the phosphocholine may correspond to 1g choline and/or the phosphocholine per 100ml of the carrier in the case of a liquid composition, or 1 g choline and/or the phosphocholine per $100cm^3$ of the carrier in the case of a solid (or substantially solid) composition. In a alternative embodiment, a given % value in respect of the choline and/or the phosphocholine component of the composition as described in this paragraph, and/or elsewhere in the present application, refers to a % weight to weight (w/w), that is the % weight of the choline and/or the phosphocholine component relative to the weight of whole composition. For example, the value of 1% (w/w) of a choline and/or the phosphocholine may correspond to 1g of the choline and/or the phosphocholine per 100g of the composition.

In some aspects, the calcium D-heptagluconate dihydrate is between 0.0001% and 10%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the carrier, or the carrier comprises at least, at most, equal to, or between any two of 0.0001% and 10% of the calcium D-heptagluconate dihydrate, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the calcium D-heptagluconate dihydrate. In a preferred embodiment, a given % value in respect of the calcium D-heptagluconate dihydrate component of the composition as described in this paragraph, and/or elsewhere in the present application, refers to a % weight to volume (w/v), that is the % weight of the calcium D-heptagluconate dihydrate relative to the volume of the carrier, within the composition. For example, the value of 1% (w/v) of calcium D-heptagluconate dihydrate may correspond to 1g of calcium D-heptagluconate dihydrate per 100m1 of the carrier in the case of a liquid composition, or 1 g of calcium D-heptagluconate dihydrate per $100cm^3$ of the carrier in the case of a solid (or substantially solid) composition. In a alternative embodiment, a given % value in respect of the calcium D-heptagluconate dihydrate component of the composition as described in this paragraph, and/or elsewhere in the present application, refers to a % weight to weight (w/w), that is the % weight of the calcium D-heptagluconate dihydrate component relative to the weight of whole composition. For example, the value of 1% (w/w) of a calcium D-heptagluconate dihydrate may correspond to 1g of the calcium D-heptagluconate dihydrate per 100g of the composition.

In some aspects, the additional surfactant is between 0.0001% and 10.0%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the carrier, or the carrier comprises at least, at most, equal to, or between any two of 0.0001% and 10.0% of the surfactant, e.g., 0.0001%, 0.0002%, 0.0003%, 0.0004%, 0.0005%, 0.0006%, 0.0007%, 0.0008%, 0.0009%, 0.001%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, or 3.0% of the surfactant. In a preferred embodiment, a given % value in respect of the additional surfactant component of the composition as described in this paragraph, and/or elsewhere in the present application, refers to a % weight to volume (w/v), that is the % weight of the additional surfactant relative to the volume of the carrier, within the composition. For example, the value of 1% (w/v) of additional surfactant may correspond to 1g of additional surfactant per 100ml of the carrier in the case of a liquid composition, or 1 g of additional surfactant per 100cm$^3$ of the carrier in the case of a solid (or substantially solid) composition. In a alternative embodiment, a given % value in respect of the additional surfactant component of the composition as described in this paragraph, and/or elsewhere in the present application, refers to a % weight to weight (w/w), that is the % weight of the additional surfactant component relative to the weight of whole composition. For example, the value of 1% (w/w) of an additional surfactant may correspond to 1g of the additional surfactant per 100g of the composition. The additional surfactant may be any one or more of the various surfactants disclosed herein, each of which are contemplated for use in the compositions disclosed herein. Any one of the surfactants disclosed herein may be excluded as an additional surfactant from a composition of the disclosure in certain aspects.

In some aspects, the additional plasticizer is between 0.0001% and 10.0%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the carrier, or the carrier comprises at least, at most, equal to, or between any two of 0.0001% and 10.0% of the plasticizer, e.g., 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the plasticizer. In a preferred embodiment, a given % value in respect of the additional plasticizer component of the composition as described in this paragraph, and/or elsewhere in the present application, refers to a % weight to volume (w/v), that is the % weight of the additional plasticizer relative to the volume of the carrier, within the composition. For example, the value of 1% (w/v) of additional plasticizer may correspond to 1g of additional plasticizer per 100ml of the carrier in the case of a liquid composition, or 1 g of additional plasticizer per 100cm$^3$ of the carrier in the case of a solid (or substantially solid) composition. In a alternative embodiment, a given % value in respect of the additional plasticizer component of the composition as described in this paragraph, and/or elsewhere in the present application, refers to a % weight to weight (w/w), that is the % weight of the additional plasticizer component relative to the weight of whole composition. For example, the value of 1% (w/w) of an additional plasticizer may correspond to 1g of the additional plasticizer per 100g of the composition. The additional plasticizer may be any one or more of the various plasticizers disclosed herein, each of which are contemplated for use in the compositions disclosed herein. Any one of the plasticizers disclosed herein may be excluded as an additional plasticizer from a composition of the disclosure in certain aspects.

In some aspects, the additional sugar is between 0.0001% and 10.0%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the carrier, or the carrier comprises at least, at most, equal to, or between any two of 0.0001% and 10.0% of the sugar, e.g., 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the sugar. In a preferred embodiment, a given % value in respect of the additional sugar component of the composition as described in this paragraph, and/or elsewhere in the present application, refers to a % weight to volume (w/v), that is the % weight of the additional sugar relative to the volume of the carrier, within the composition. For example, the value of 1% (w/v) of additional sugar may correspond to 1g of additional sugar per 100ml of the carrier in the case of a liquid composition, or 1 g of additional sugar per 100cm$^3$ of the

carrier in the case of a solid (or substantially solid) composition. In a alternative embodiment, a given % value in respect of the additional sugar component of the composition as described in this paragraph, and/or elsewhere in the present application, refers to a % weight to weight (w/w), that is the % weight of the additional sugar component relative to the weight of whole composition. For example, the value of 1% (w/w) of an additional sugar may correspond to 1g of the additional sugar per 100g of the composition. The additional sugar may be any one or more of the various sugars disclosed herein, each of which are contemplated for use in the compositions disclosed herein. Any one of the sugars disclosed herein may be excluded as an additional sugar from a composition of the disclosure in certain aspects.

In some aspects, the additional polymer is between 0.0001% and 10.0%, e.g., at least, at most, equal to, or between any two of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the carrier, or the carrier comprises at least, at most, equal to, or between any two of 0.0001% and 10.0% of the polymer, e.g., 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0% of the polymer. In a preferred embodiment, a given % value in respect of the additional polymer component of the composition as described in this paragraph, and/or elsewhere in the present application, refers to a % weight to volume (w/v), that is the % weight of the additional polymer relative to the volume of the carrier, within the composition. For example, the value of 1% (w/v) of additional polymer may correspond to 1g of additional polymer per 100ml of the carrier in the case of a liquid composition, or 1 g of additional polymer per 100cm$^3$ of the carrier in the case of a solid (or substantially solid) composition. In a alternative embodiment, a given % value in respect of the additional polymer component of the composition as described in this paragraph, and/or elsewhere in the present application, refers to a % weight to weight (w/w), that is the % weight of the additional polymer component relative to the weight of whole composition. For example, the value of 1% (w/w) of an additional polymer may correspond to 1g of the additional sugar per 100g of the composition. The additional polymer may be any one or more of the various polymers disclosed herein, each of which are contemplated for use in the compositions disclosed herein. In specific aspects, the additional polymer is polyethylene glycol (PEG). PEGs having various molecular weights are disclosed herein and include PEGs having a molecular weight of 200-20,000 g/mol, e.g., PEG 200, PEG 300, PEG 400, PEG 600, PEG 1000, PEG 1500, PEG 4000, PEG 8000, PEG 10,000, 12,000, and PEG 20,000. Any one of the PEGs disclosed herein may be excluded from a composition of the disclosure in certain aspects. In specific aspects, the PEG is PEG 1500, and the carrier comprises about 0.5% PEG. In some aspects, the additional polymer is pullulan, and the carrier comprises about 0.5% pullulan. Any one of the polymers disclosed herein may be excluded as an additional polymer from a composition of the disclosure in certain aspects.

In some aspects, the agent (e.g., therapeutic agent) of the composition is a polypeptide, small molecule, or nucleic acid. In some aspects, the composition comprises between 0.001 mg/mL and 100 mg/mL of the agent (e.g., therapeutic agent), e.g., at least, at most, equal to, or between any two of 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2.0, 3.0, 4.0, 5.0, 6.0, 7.0, 8.0, 9.0, 10, 20, 30, 40, 50, 60, 70, 80, 90, and 100 mg/mL of the agent (e.g., therapeutic agent). In some aspects, the agent (e.g., therapeutic agent) is a nucleic acid, and the composition comprises between 0.001 mg/mL and 1 mg/mL of the nucleic acid. Various nucleic acids are disclosed herein and can include DNA (e.g., recombinant DNA or plasmid DNA or genomic DNA) and RNA (e.g., mRNA). In specific aspects, the nucleic acid is plasmid DNA, and the composition comprises between 0.05 mg/mL and 1 mg/mL, e.g., about 0.1 mg/mL, plasmid DNA. In specific aspects, the nucleic acid is genomic DNA, and the composition comprises between 0.05 mg/mL and 2 g/mL genomic DNA. In specific aspects, the nucleic acid is mRNA, and the composition comprises between 0.001 mg/mL and 1 mg/mL, e.g., about 0.025 mg/mL or about 0.1 mg/mL, mRNA. In some aspects, the nucleic acid is naked, e.g., nucleic acid that is not associated with proteins, lipids, or any other protective molecules. In some aspects, the nucleic acid is associated with one or more lipids, proteins, carbohydrates, and/or other organic compounds (e.g., squalene). In some aspects, the nucleic acid is associated with one or more transfection reagents (e.g., LIPOFECTAMINE® (a mixture of DOSPA (2,3-dioleoyloxy-N- [2(sperminecarboxamido)ethyl]-N,N-dimethyl-1-propaniminium trifluoroacetate) and DOPE (1,2-Dioleoyl-sn-glycerophosphoethanolamine)), calcium phosphate, polyethylenimine). In specific aspects, the nucleic acid is encapsulated in lipid nanoparticles (LNPs) (e.g., LNP-encapsulated mRNA). Any one of the nucleic acids disclosed herein may be excluded from a composition of the disclosure in certain aspects.

In certain aspects, the pH of one or more of the components of the composition is adjusted so that the final pH of the composition is from about pH 6.0 to 9.0. In some aspects, the composition has a pH from about 6.0 to about 9.0, e.g., at least, at most, equal to, or between any two of 6.0, 6.1, 6.2, 6.3, 6.4, 6.5, 6.6. 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0. In specific aspects, the composition has a pH of about 8.5. In specific aspects, the composition has a pH of about 8.0. In some aspects of the composition and methods disclosed herein, the composition is in liquid form. In some aspects of the composition and methods disclosed herein, the composition is in substantially solid film form. In some aspects of the compositions and methods disclosed herein, the composition is a liquid or substantially solid amorphous carrier.

Aspects of the disclosure relate to a method of storing/preserving an agent (e.g., therapeutic agent) comprising formulating the agent in a composition described herein. Further disclosed are methods for storing an agent (e.g., therapeutic agent) comprising formulating the agent in a composition of the disclosure and storing the composition, for example storing the composition at temperatures above 0 °C, above 4 °C, above 15 °C, above 25 °C, above 30 °C, or above 35 °C, for at least, at most, equal to, or between any two of 1 day, 7 days, 14 days, 30 days, 60 days, 90 days, 120 days, 150 days, 180 days, 210 days, 240 days, 270 days, 300 days, 330 days, or 360 days. In some aspects of the composition and methods disclosed herein, the film is stored for at least, at most, equal to, or about 1 year. In some aspects of the composition and methods disclosed herein, the film is stored for at least, at most, equal to, or between any two of 1 month, 2 months, 3 months, 4 months, 5 months, 6 months, 7 months, 8 months, 9 months, 10 months, 11 months, 12 months, 18 months, 24 months, or 36 months.

In some aspects, after storing, the agent (e.g., therapeutic agent) is preserved by at least, at most, equal to, or between any two of 50%, 55%, 60%, 65%, 70%, 75%, 80%, 85%, 90%, 91%, 92%, 93%, 94%, 95%, 96%, 97%, 98%, or 99% as measured by biological function and efficacy. It is believed that considerable value is obtained even with a composition described herein that results in 50% or less (e.g., about 45%, about 40%, about 35%, about 30%, about 25%, etc.) preservation of biological function and efficacy, in light of the ease of storage and shipment. In some aspects, the agent (e.g., therapeutic agent) is a nucleic acid, and after storing, the nucleic acid is preserved by at least, at most, equal to, or between any two of 50%, 55%, 60%, 65%, 70%, 75%, 80%, 85%, 90%, 91%, 92%, 93%, 94%, 95%, 96%, 97%, 98%, or 99% as measured by transduction efficiency and/or transfection efficiency. In some aspects, the nucleic acid is preserved by at least 50%, as measured by transduction efficiency and/or transfection efficiency, after storage. In some aspects, the nucleic acid is preserved by at least 55%, as measured by transduction efficiency and/or transfection efficiency, after storage. In some aspects, the nucleic acid is preserved by at least 60%, as measured by transduction efficiency and/or transfection efficiency, after storage. In some aspects, the nucleic acid is preserved by at least 65%, as measured by transduction efficiency and/or transfection efficiency, after storage. In some aspects, the nucleic acid is preserved by at least 70%, as measured by transduction efficiency and/or transfection efficiency, after storage. In some aspects, the nucleic acid is preserved by at least 75%, as measured by transduction efficiency and/or transfection efficiency, after storage. In some aspects, the nucleic acid is preserved by at least 80%, as measured by transduction efficiency and/or transfection efficiency, after storage. In some aspects, the nucleic acid is preserved by at least 85%, as measured by transduction efficiency and/or transfection efficiency, after storage. In some aspects, the nucleic acid is preserved by at least 90%, as measured by transduction efficiency and/or transfection efficiency, after storage. In some aspects, the nucleic acid is preserved by at least 95%, as measured by transduction efficiency and/or transfection efficiency, after storage. In some aspects, the nucleic acid is preserved by at least 99%, as measured by transduction efficiency and/or transfection efficiency, after storage.

Also disclosed are methods for delivering an agent (e.g., therapeutic agent) to a subject comprising administering to the subject an effective amount of a composition of the present disclosure, in some cases where the composition has been stored (e.g., for at least 1, 7, 14, 30, 60, 90, 120, 150, 180, 210, 240, 270, 300, 330, 360, or more days) at a temperature above 0 °C (e.g., about 4 °C, about 25 °C, between 15-30 °C, or between 0-8 °C) prior to administration to the subject. In some aspects, the composition is administered intravenously, intradermally, intra-arterially, intra-graft, intraperitoneally, intralesionally, intracranially, intraspinally, intracisternally, intraarticularly, intraprostatically, intrapleurally, intratracheally, intranasally, intravitreally, intravaginally, intrarectally, topically, intratumorally, intramuscularly, intraperitoneally, subcutaneously, subconjunctivally, intravesicularly, mucosally, intrapericardially, intraumbilically, intraocularly, intranasally, orally, topically, locally, inhalation, injection, infusion, continuous infusion, localized perfusion bathing target cells directly, *via* a catheter, *via* lavage, in cremes, in lipid compositions (e.g., liposomes), or combinations thereof. In particular aspects, disclosed is a method of delivering an agent (e.g., therapeutic agent) to a subject comprising intravenously, intramuscularly, intranasally, sublingually, or buccally administering an effective amount of a composition described herein.

Disclosed, in some aspects, is a method for making a composition of the disclosure, the method comprising forming an aqueous solution comprising an agent (e.g., therapeutic agent), a sugar, a zwitterionic compound, and a base polymer by mixing the agent, the sugar, the zwitterionic compound, and the base polymer until homogenous. The agent (e.g., therapeutic agent), the sugar, the zwitterionic compound, and the base polymer may be combined in any order to obtain the aqueous solution. As one non-limiting example, the method may comprise mixing the sugar and the base polymer to form a first mixture; mixing the agent (e.g., therapeutic agent) and the zwitterionic compound to form a second mixture; and mixing the first and second mixtures to form the aqueous solution. In some aspects, the aqueous solution comprises about 2% PVA, about 1% arginine, about 1% trehalose, and about 0.5% sorbitol, wherein the aqueous solution has a pH between 8.0 and 9.0. In some aspects, the aqueous solution comprises about 2% PVA, about 1% arginine, about 1% sorbitol, and about 1.5% glycerol, wherein the aqueous solution has a pH between 8.0 and 9.0.

In some aspects, the aqueous solution further comprises a plasticizer, and the agent (e.g., therapeutic agent), the sugar, the zwitterionic compound, the base polymer, and the plasticizer may be combined in any order to obtain the aqueous solution. As one example, the method may comprise mixing the plasticizer with the sugar and the base polymer to form a first mixture; mixing the agent (e.g., therapeutic agent) and the zwitterionic compound to form a second mixture; and mixing the first and second mixtures to form the aqueous solution.

In some aspects, the aqueous solution further comprises a surfactant, and the agent (e.g., therapeutic agent), the sugar, the zwitterionic compound, the base polymer, and the surfactant may be combined in any order to obtain the aqueous solution. As one example, the method may comprise mixing the surfactant with the sugar and the base polymer to form a first mixture; mixing the agent (e.g., therapeutic agent) and the zwitterionic compound to form a second mixture; and mixing the first and second mixtures to form the aqueous solution.

In some aspects, the aqueous solution further comprises a surfactant and a plasticizer, and the agent (e.g., therapeutic agent), the sugar, the zwitterionic compound, the base polymer, the surfactant, and the plasticizer may be combined in any order to obtain the aqueous solution. As one example, the method may further comprise mixing the surfactant and the plasticizer with the sugar and the base polymer to form a first mixture; mixing the agent (e.g., therapeutic agent) and the zwitterionic compound to form a second mixture; and mixing the first and second mixtures to form the aqueous solution.

Disclosed, in some aspects, is a method for making the composition of the disclosure, the method comprising forming an aqueous solution comprising an agent (e.g., therapeutic agent), a plasticizer, a surfactant, and a base polymer by mixing the agent, the plasticizer, the surfactant, and the base polymer until homogenous. The agent (e.g., therapeutic agent), the plasticizer, the surfactant, and the base polymer may be combined in any order to obtain the aqueous solution. In one aspect, disclosed is a method for making the composition of the disclosure, the method comprising forming an aqueous solution comprising an agent (e.g., therapeutic agent), a plasticizer, a surfactant, and a base polymer by: mixing the agent with the surfactant to form a first mixture; mixing the plasticizer with the base polymer to form a second mixture; and mixing the first and second mixtures to form the aqueous solution. In some aspects, the method further comprises mixing the agent (e.g., therapeutic agent) with a pegylated lipid before mixing the agent with the surfactant to form the first mixture. In some aspects, the aqueous solution comprises about 1.5% HPMC, about 0.01% poloxamer, and about 3% glycerol. In some aspects, the aqueous solution comprises about 1% HPMC, about 0.012% poloxamer, about 3% glycerol, about 0.008% pegylated lipid. In some aspects, the aqueous solution has a pH between 7.5 and 8.5.

In some aspects, the components of the aqueous solution are mixed in the presence of a buffering agent sufficient to promote a pH of 6.0 to 9.0 of the composition. In some aspects, the agent (e.g., therapeutic agent) is added to the homogeneous mixture at ambient temperature for dispersion in the homogenous mixture to generate a composition in liquid form. In some aspects of the composition and methods disclosed herein, the liquid composition is stored at temperatures above 0 °C, above 4 °C, above 15 °C, above 25 °C, above 30 °C, or above 35 °C, for at least, at most, equal to, or between any two of 1 day, 7 days, 14 days, 30 days, 60 days, 90 days, 120 days, 150 days, 180 days, 210 days, 240 days, 270 days, 300 days, 330 days, or 360 days; at least, at most, equal to, or about 1 year; or at least, at most, equal to, or between any two of 1 month, 2 months, 3 months, 4 months, 5 months, 6 months, 7 months, 8 months, 9 months, 10 months, 11 months, 12 months, 18 months, 24 months, or 36 months. Additionally or alternatively, in some aspects of the composition and methods disclosed herein, the liquid composition is subject to freeze thawing.

In some aspects, the method further comprises drying the aqueous solution to form a substantially solid film. In some aspects, the composition is dried under ambient temperature and pressure, and optionally, under constant airflow. In some aspects, the method does not comprise drying the aqueous solution to form a substantially solid film. In some aspects, the dried substantially solid film is stored at temperatures above 0 °C, above 4 °C, above 15 °C, above 25 °C, above 30 °C, or above 35 °C, for at least, at most, equal to, or between any two of 1 day, 7 days, 14 days, 30 days, 60 days, 90 days, 120 days, 150 days, 180 days, 210 days, 240 days, 270 days, 300 days, 330 days, or 360 days; at least, at most, equal to, or about 1 year; or at least, at most, equal to, or between any two of 1 month, 2 months, 3 months, 4 months, 5 months, 6 months, 7 months, 8 months, 9 months, 10 months, 11 months, 12 months, 18 months, 24 months, or 36 months. Additionally or alternatively, in some aspects, of the composition and methods disclosed herein, the dried substantially solid film is subject to freeze thawing. In some aspects, the method does not comprise storing the dried substantially solid film.

In some aspects, the method further comprises dissolving the substantially solid film in an appropriately buffered aqueous solution to generate the composition in liquid reconstituted form. In some aspects, the appropriately buffered aqueous solution comprises buffered saline or a poloxamer. The buffered saline may be at a concentration of between 0.1% and 10%, e.g., at least, at most, equal to, or between any two of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, or 10%. In some aspects, the appropriately buffered aqueous rehydrating solution comprises buffered saline at a concentration of 0.9%. In a preferred embodiment, a given % value in respect of the buffered saline as described in this paragraph, and/or elsewhere in the present application, refers to a % weight to volume (w/v), that is the % weight of the non-carrier components of the buffered saline relative to the volume of the carrier, within the buffered saline composition. In a alternative embodiment, a given % value in respect of the buffered saline as described in this paragraph, and/or elsewhere in the present application, refers to a % weight to weight (w/w), that is the % weight of the non-carrier components of the buffered saline relative to the weight of whole buffered saline composition. For example, the value of 1% (w/w) of buffered saline may correspond to 1g of the non-carrier components of the buffered saline per 100g of the whole buffered saline composition. The poloxamer may be any poloxamer disclosed herein and may be at a concentration of between 0.00001% and 0.1%, e.g., at least, at most, equal to, or between any two of 0.00001, 0.00002, 0.00003, 0.00004, 0.00005, 0.00006, 0.00007, 0.00008, 0.00009, 0.0001, 0.0002, 0.0003, 0.0004, 0.0005, 0.0006, 0.0007, 0.0008, 0.0009, 0.001, .002, 0.003, 0.004, 0.005, 0.006, 0.007, 0.008, 0.009, 0.01, 0.02, 0.03, 0.04, 0.05, 0.06, 0.07, 0.08, 0.09, and 0.1%, in the solution. In some aspects, the poloxamer is poloxamer 188, and the appropriately buffered aqueous rehydrating solution comprises about 0.001% of the poloxamer. In some aspects, the poloxamer is poloxamer 407, and the rehydrating solution comprises about 0.01% of the poloxamer.

The term "about" or "approximately" are defined as being close to as understood by one of ordinary skill in the art. In one non-limiting aspect, the terms are defined to be within 10%, preferably within 5%, more preferably within 1%, and most preferably within 0.5%.

The use of the word "a" or "an" when used in conjunction with the term "comprising" may mean "one," but it is also consistent with the meaning of "one or more," "at least one," and "one or more than one."

The phrase "and/or" means "and" or "or". To illustrate, A, B, and/or C includes: A alone, B alone, C alone, a combination of A and B, a combination of A and C, a combination of B and C, or a combination of A, B, and C. In other words, "and/or" operates as an inclusive or.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of' any of the ingredients or steps disclosed throughout the specification. Compositions and methods "consisting essentially of' any of the ingredients or steps disclosed limits the scope of the claim to the specified materials or steps which do not materially affect the basic and novel characteristic of the claimed aspects.

"Individual," "subject," and "patient" are used interchangeably and can refer to a human or non-human.

"Ambient temperature" and "room temperature" can each include a temperature of 15 °C to 30 °C, or any range or value derivable therein. In some aspects, ambient or room temperature can include a temperature of 15 °C to 25 °C, 20 °C to 25 °C, 18 °C to 28 °C, or any range or value derivable therein. In certain aspects, ambient or room temperature includes 25 °C.

Any method in the context of a therapeutic, diagnostic, or physiologic purpose or effect may also be described in "use" claim language such as "Use of' any compound, composition, or agent discussed herein for achieving or implementing a described therapeutic, diagnostic, or physiologic purpose or effect.

It is specifically contemplated that any limitation discussed with respect to one aspect of the disclosure may apply to any other aspect of the disclosure. Furthermore, any composition of the disclosure may be used in any method of the disclosure, and any method of the disclosure may be used to produce or to utilize any composition of the disclosure. Any embodiment discussed with respect to one aspect of the disclosure applies to other aspects of the disclosure as well and vice versa. For example, any step in a method described herein can apply to any other method. Moreover, any method described herein may have an exclusion of any step or combination of steps. Aspects of an embodiment set forth in the Examples are also aspects that may be implemented in the context of embodiments discussed elsewhere in a different Example or elsewhere in the application, such as in the Summary, Detailed Description, Claims, and Brief Description of the Drawings.

Other objects, features and advantages of the present disclosure will become apparent from the following detailed description. It should be understood, however, that the detailed description and the specific examples, while indicating specific aspects of the disclosure, are given by way of illustration only, since various changes and modifications within the spirit and scope of the disclosure will become apparent to those skilled in the art from this detailed description.

## BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific aspects presented herein.

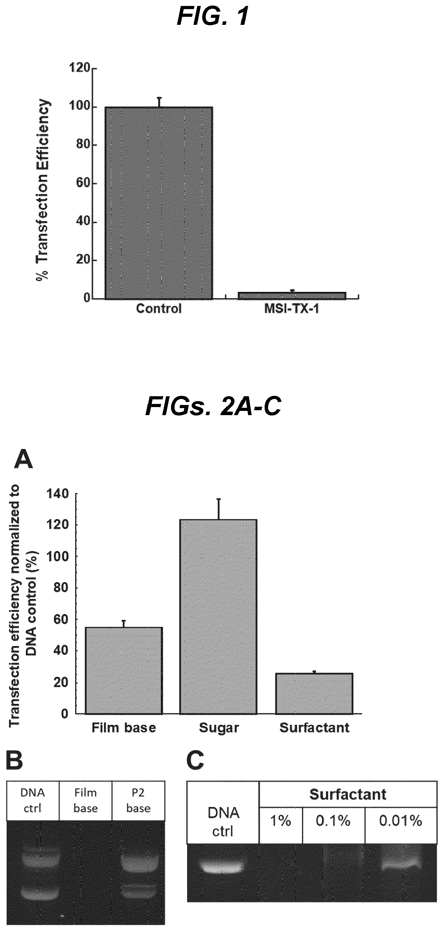

**FIG. 1** shows transduction efficiency of an adenovirus versus transfection efficiency of a plasmid after long term storage at 25 °C in films having the same MSI-TX-1 formulation.
**FIGs. 2A-2C** show the effects of film base and surfactant on plasmid transfection efficiency (**FIG. 2A**) and mobility of plasmid DNA on an agarose gel (**FIG. 2B** and **FIG. 2C**).

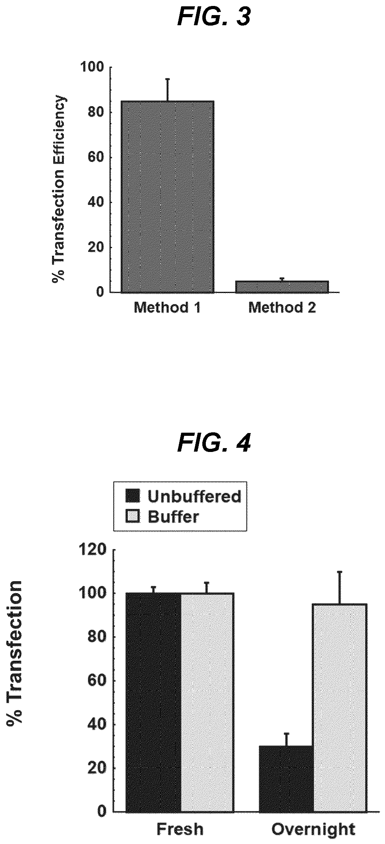

**FIG. 3** shows the compatibility of a hydroxypropyl methylcellulose (HPMC) film base with transfection reagents.

Method 1 corresponds to transfection with calcium phosphate. Method 2 corresponds to transfection with polyethylenimine (PEI).

FIG. 4 shows the effect of film base buffer on transfection efficiency after storage at room temperature.

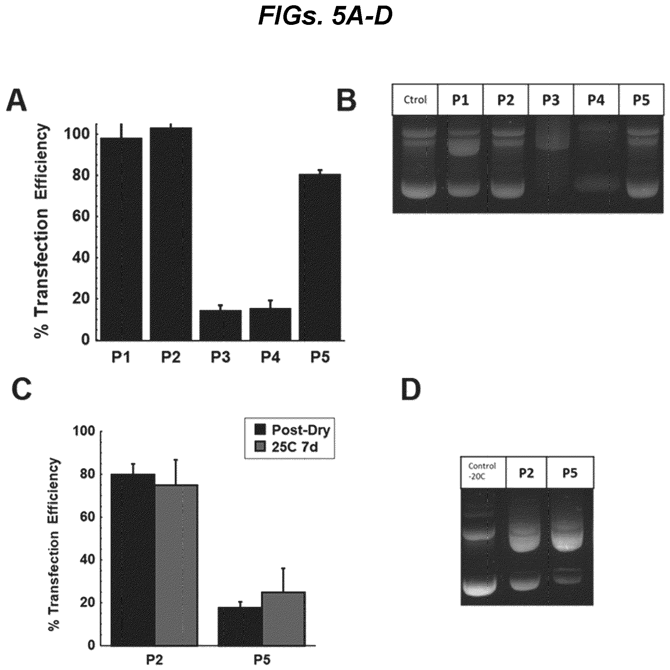

FIGs. 5A-5D show the impact of various film formulations P1-P5 on transfection efficiency (**FIG. 5A** and **FIG. 5C**) and plasmid conformation (**FIG. 5B** and **FIG. 5D**) after storage of plasmid DNA with the formulation in the liquid state for 48 hours (**FIG. 5A** and **FIG. 5B**) or post-rehydration of plasmid DNA films after storage for 7 days (**FIG. 5C** and **FIG. 5D**) at 25 °C.

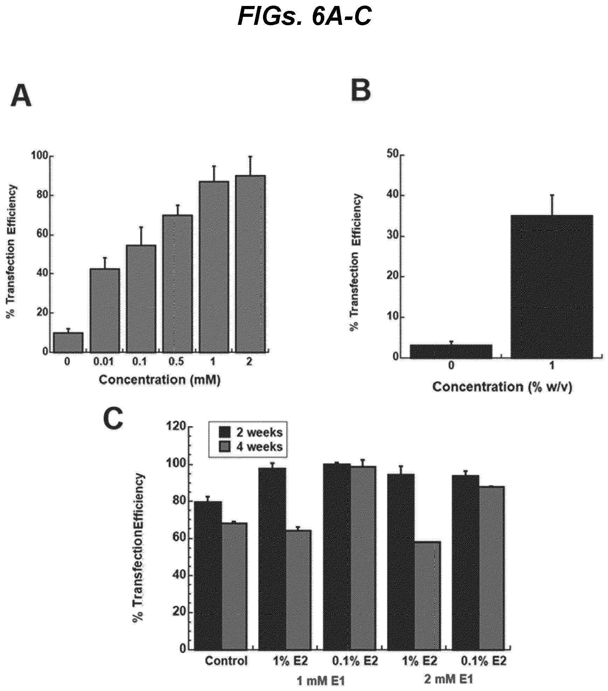

FIGs. 6A-6C show the impact of excipients EDTA (**FIG. 6A**; E1), Arginine (**FIG. 6B**; E2), and EDTA+Arginine (**FIG. 6C**) on transfection efficiency of plasmid DNA after storage for 4 weeks at 40 °C (**FIG. 6A** and **FIG. 6C**) or 7 days at 50 °C (**FIG. 6B**).

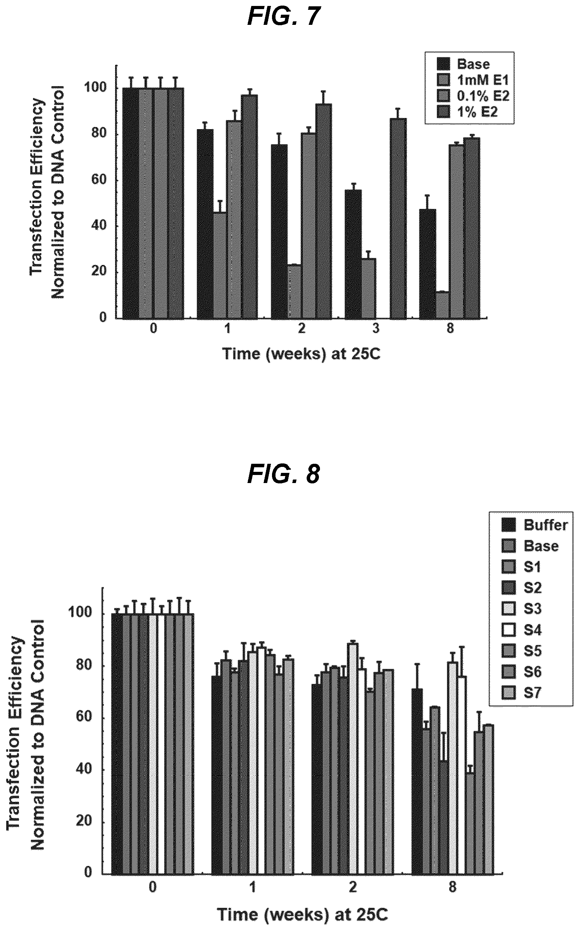

FIG. 7 shows the effect of film formulations comprising EDTA (E1) or arginine (E2) on plasmid DNA stability after storage for 8 weeks at 25 °C.

FIG. 8 shows the impact of film formulations comprising various sugars S1-S7 on the transfection efficiency of plasmid DNA prepared in the films and stored for 8 weeks at 25 °C.

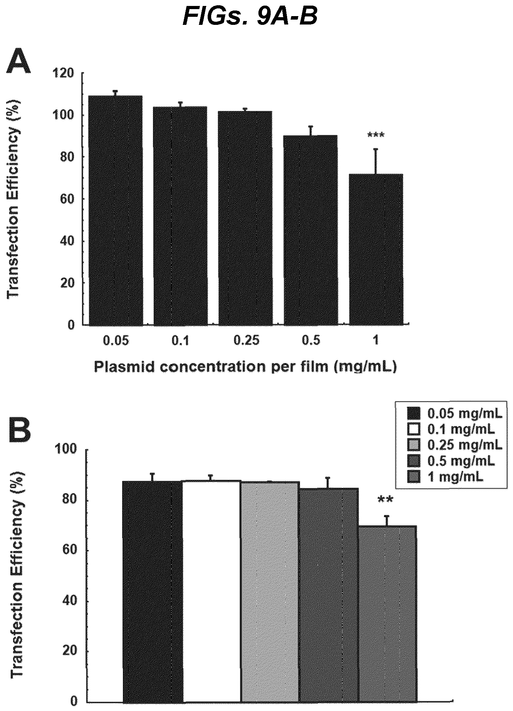

FIGs. 9A-9B show the effect of plasmid DNA concentration on transfection efficiency of plasmid DNA after drying of a film comprising the plasmid DNA (**FIG. 9A**) and after storage of the dried film for 30 days at 25 °C (**FIG. 9B**).

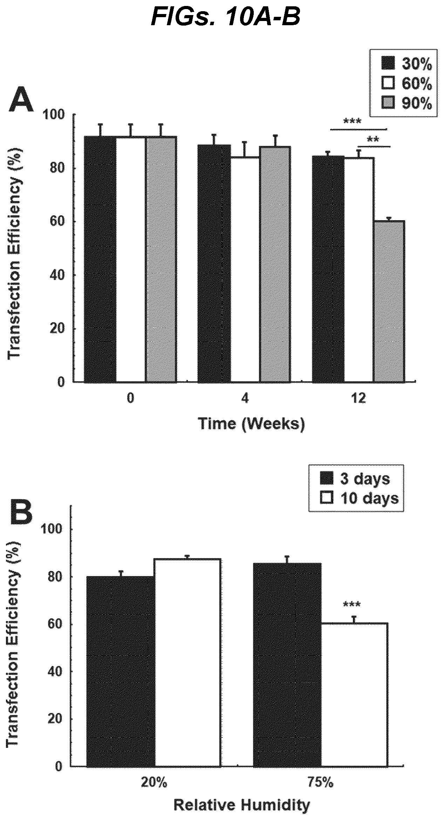

FIGs. 10A-10B show the effect of RH on transfection efficiency of plasmid DNA prepared in films after storage of the films for 12 weeks at 25 °C (**FIG. 10A**) or for 10 days at 40 °C (**FIG. 10B**) and various RHs.

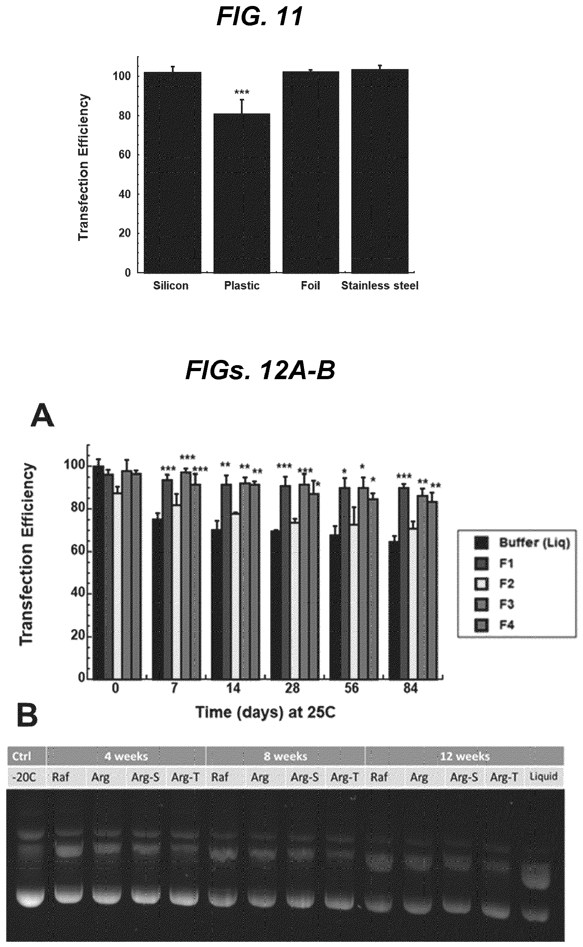

FIG. 11 shows the effect of film mold materials on transfection efficiency of plasmid DNA.

FIGs. 12A-12B show the impact of optimized film formulations F1-F4 on the transfection efficiency (**FIG. 12A**) and conformation (**FIG. 12B**) of plasmid DNA prepared in the films and stored in for 12 weeks at 25 °C and 60% RH. ***, $p < 0.001$, **, $p < 0.01$, *, $p < 0.05$ with respect to a buffer-only control (Buffer (Liq)).

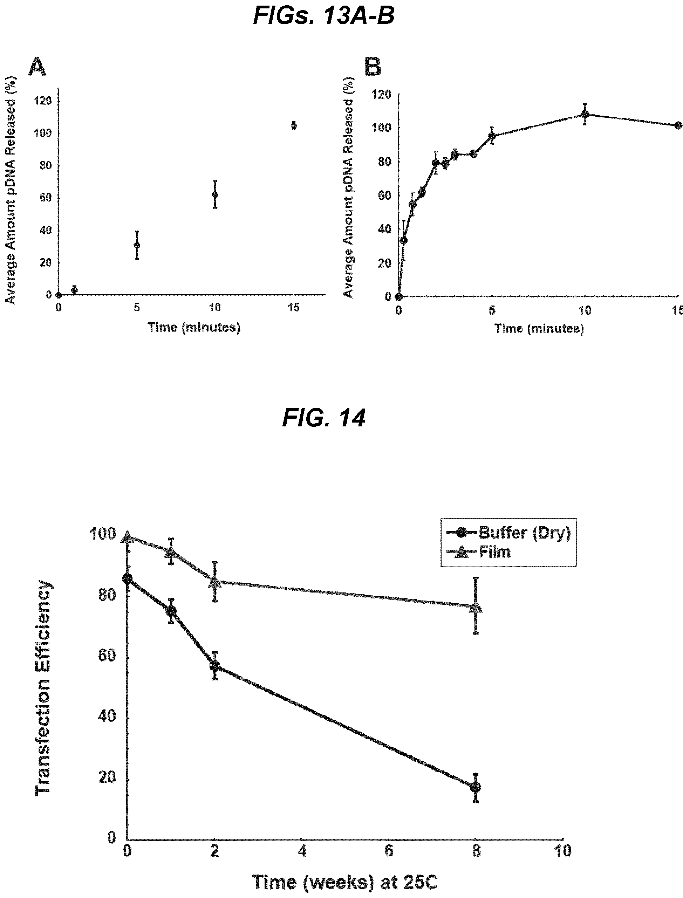

FIGs. 13A-13B show the effect of a film formulation consisting of HPMC (**FIG. 13A**) and a film formulation including polyvinyl alcohol (**FIG. 13B**) on release of plasmid DNA from the film.

FIG. 14 shows transfection efficiency of plasmid DNA after storage of the plasmid DNA in a solid film state for up to 8 weeks at 25 °C.

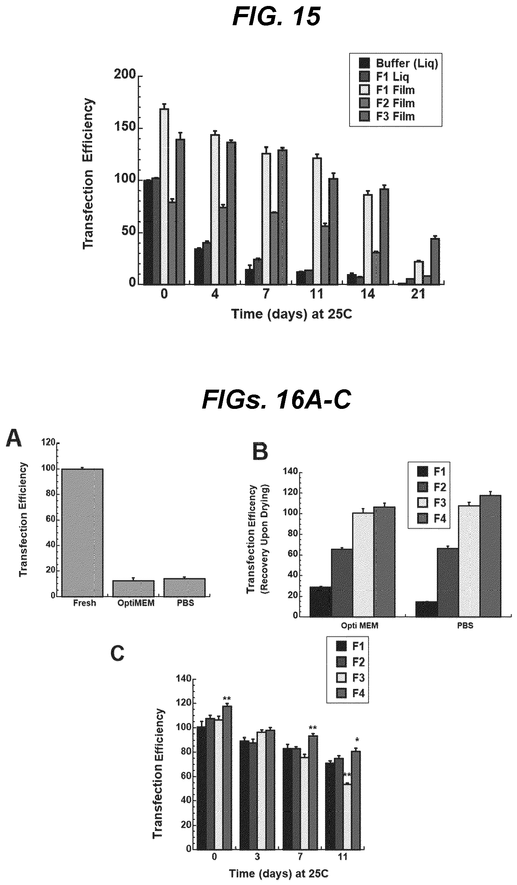

FIG. 15 shows the impact of optimized liquid (liq) or film formulations F1-F3 on the transfection efficiency of lipofectamine-complexed plasmid DNA prepared in the liquid or film formulations after storage for 2 weeks at 25 °C and 60% relative humidity (RH).

FIGs. 16A-16C show the impact of film formulations F1-F4 comprising gelatin on the transfection efficiency of plasmid DNA complexed with PEI in OPTI-MEM™ or PBS as a solvent after storage for 4 hours at room temperature (**FIG. 16A**); after drying of films comprising the plasmid DNA complexed with PEI in OPTI-MEM™ or PBS as a solvent (**FIG. 16B**), and after storage of films comprising the PEI-complexed plasmid DNA for up to 11 days at 25 °C and 60% RH (**FIG. 16C**). * $p < 0.05$, ** $p < 0.01$.

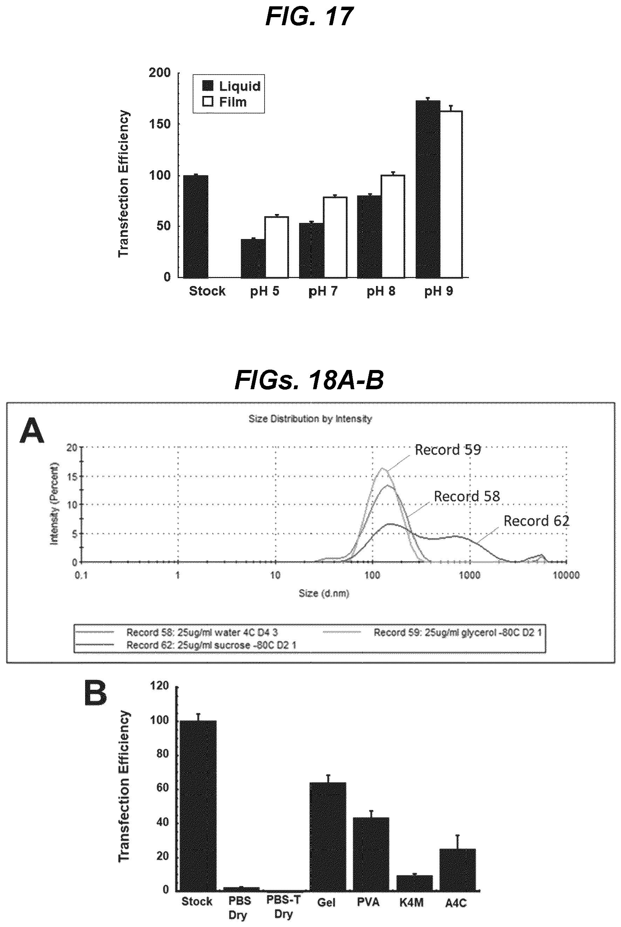

FIG. 17 shows the effect of film formulations (pH 5-9) comprising gelatin on the transfection efficiency of lipid nanoparticle (LNP)-encapsulated mRNA prior to drying (Liquid) and after films were formed (Film).

FIGs. 18A-18B show the impact of sugars on stability of LNP-encapsulated mRNA in frozen and dried states (**FIG. 18A**) and of polymers on transfection efficiency of LNP-encapsulated mRNA during the film forming process (**FIG. 18B**).

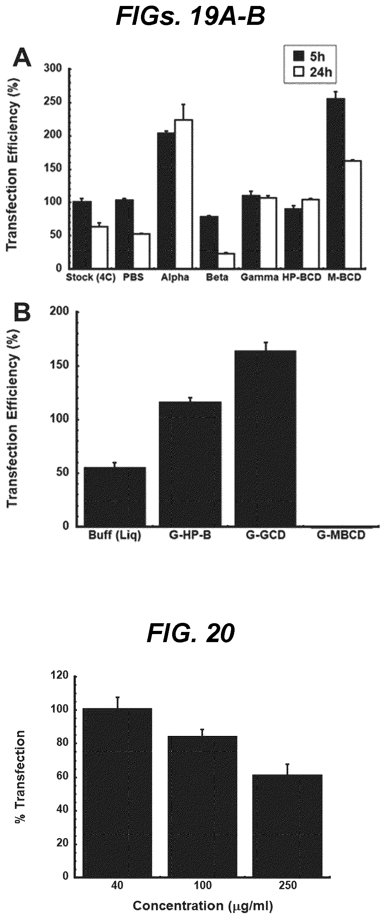

FIGs. 19A-19B show the impact of cyclodextrins on the transfection efficiency of LNP-encapsulated mRNA prepared in liquid film formulations stored for up to 24 hours at 20 °C (**FIG. 19A**) or in dried films stored for 4 days at 4 °C (**FIG. 19B**).

FIG. 20 shows the effect of LNP-encapsulated mRNA concentration on transfection efficiency of LNP-encapsulated mRNA after drying of a film comprising the LNP-encapsulated mRNA drying and rehydration.

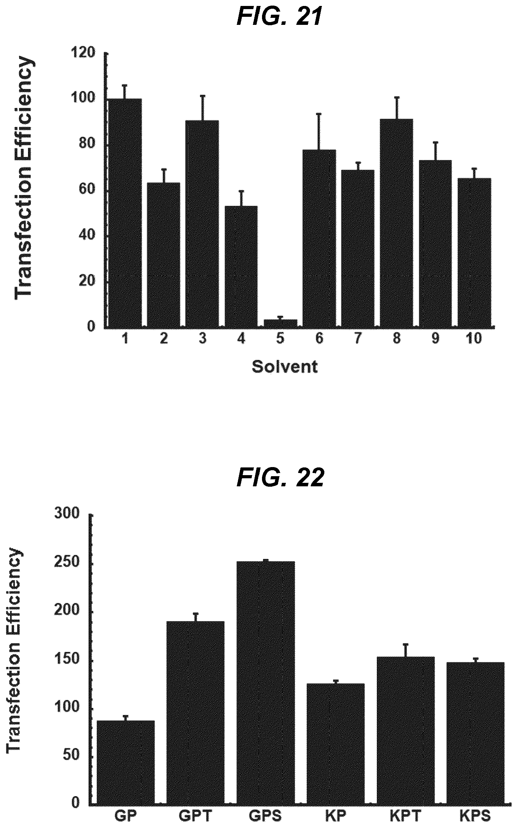

FIG. 21 shows the impact of various solvents 1-10 on transfection efficiency of LNP-encapsulated mRNA prepared in the films after rehydration of the films with the solvents 1-10.

FIG. 22 shows the impact of optimized film formulations GP, GPT, GPS, KP, KPT, and KPS on the transfection efficiency of LNP-encapsulated mRNA prepared in the film formulations.

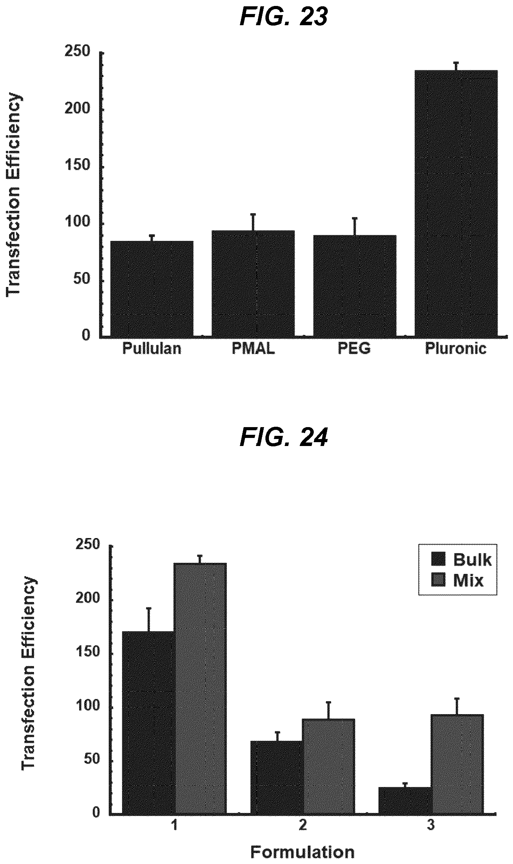

FIG. 23 shows the effect of Pluronic F127 on transfection efficiency of LNP-encapsulated mRNA compared to transfection efficiency of LNP-encapsulated mRNA prepared in films comprising other surfactants or plasticizers.

FIG. 24 shows the impact of excipient addition order during preparation of LNP-encapsulated mRNA films having formulations 1-3 on transfection efficiency of LNP-encapsulated mRNA.

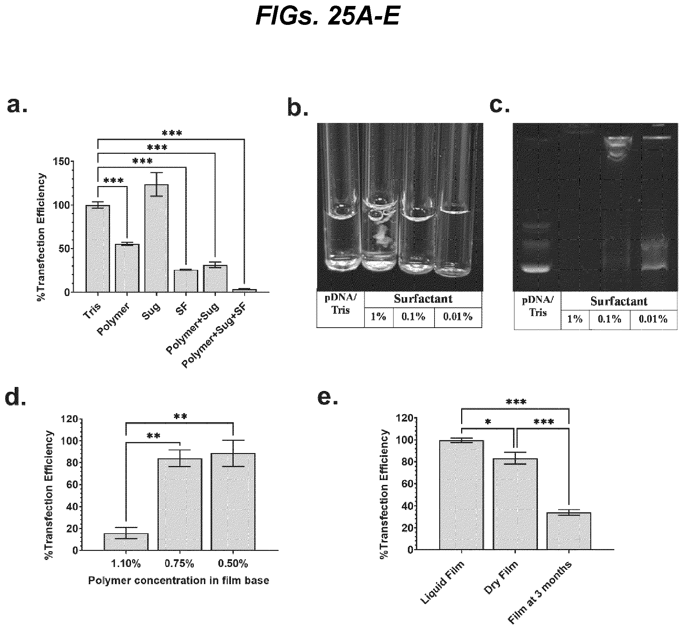

FIGs. 25A-25E. Impact of an Established Film Formulation and its Individual Components on Transfection Efficiency of Plasmid DNA. **FIG. 25A**. Surfactant (SF) and polymer base significantly compromised transfection efficiency. **FIG. 25B**. Precipitation of plasmid in the presence of surfactant. **FIG. 25C**. Mobility pattern of plasmid DNA in the presence of different concentrations of surfactant. **FIG. 25D**. Impact of polymer base concentration on transfection efficiency. **FIG. 25E** Impact of modified film formulation on preservation of transfection efficiency during the film forming process and during storage at room temperature for 3 months. Calculation of transfection efficiency is outlined in the Material and Methods section. In **FIGs. 25A**, **25D**, and **25E**, data represent the average $\pm$ the standard error of the mean for 4 replicates for each formulation. Statistical significance between formulations with respect to unformulated plasmid in 10 mM Tris buffer pH 8.0 was determined by one-way ANOVA was followed by Dunnet's multiple comparison tests (**FIGs. 25A & 25D**) Tukey's multiple comparison tests (**FIG. 25E**) *p < 0.05, **p < 0.01, ***p < 0.001.

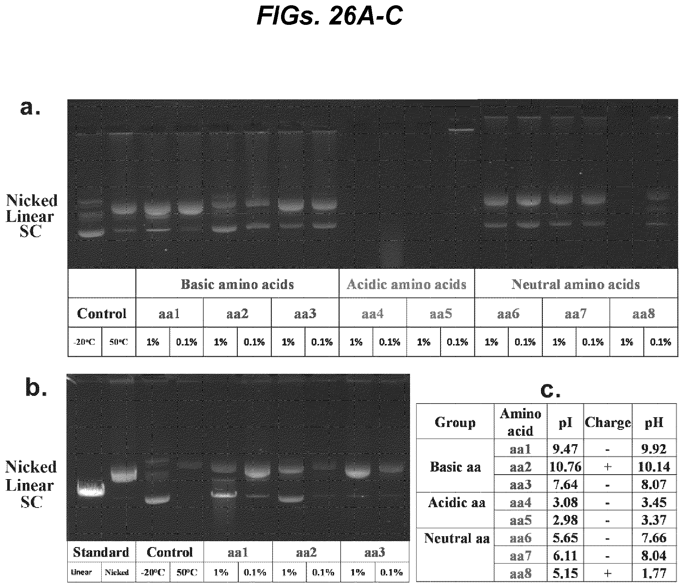

**FIGs. 26A-26C.** A Cationic, Basic Amino Acid Improves Thermostability of Plasmid DNA in Solution at 50oC. **FIG. 26A**. Resolution of the 3 different conformations of plasmid DNA stored in various amino acid-based formulations after 2 days at 50 °C by agarose gel electrophoresis. **FIG. 26B**. Resolution of the 3 different conformations of plasmid DNA stored in formulations containing basic amino acids after 4 days at 50 °C by agarose gel electrophoresis. **FIG. 26C**. Tabular summary of the isoelectric points (pI), measured pH and predominant charge (estimated using pH and pI values) of formulations containing 1% w/v of each amino acid. Specific details of each formulation denoted in this figure are summarized in **Table 1**.

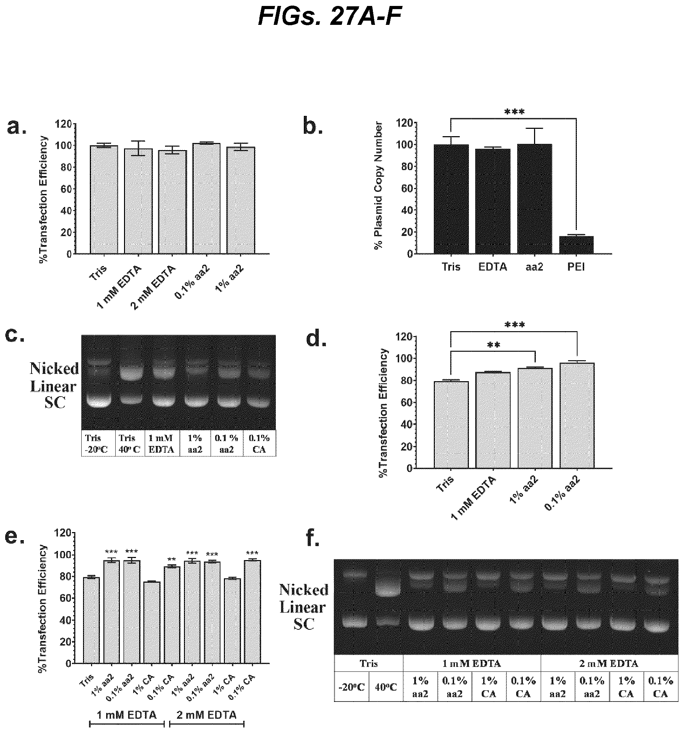

**FIGs. 27A-27F.** A Positively Charged Amino Acid Improves Thermostability of Plasmid DNA at Elevated Temperatures in Solution with Respect to EDTA. Each excipient was added to Tris buffer (pH 8) alone to determine if they interfered with transfection efficiency (**FIG. 27A**) or transgene-specific qPCR reactions (**FIG. 27B**). NOTE: Polyethyleneimine (PEI) at 4 ng/ml in combination with pDNA at 1 ng/ml was included as a positive control for qPCR. **FIG. 27C**. Resolution of the 3 different forms of plasmid DNA after storage in solution at 40 °C for 2 weeks by agarose gel electrophoresis. **FIG. 27D**. Transfection efficiency of plasmids stored in solution at 40 °C for 2 weeks. **FIG. 27E**. Impact of EDTA on transfection efficiency of plasmid in solutions of an amino acid (aa2) or choline (CA) at various concentrations. **FIG. 27F**. Agarose gel of plasmid DNA stored in various formulations for 2 weeks at 40 °C. Specific details of each formulation denoted in this figure are summarized in **Table 1**. Calculation of transfection efficiency is outlined in the Material and Methods section. In **FIGs. 27A**, **27B**, **27D**, and **27E**, data represent the average $\pm$ the standard error of the mean for 4 replicates for each respective formulation. Statistical significance between formulations with respect to unformulated plasmid in 10 mM Tris buffer was determined by one-way ANOVA followed by Dunnett's multiple comparison tests. *p < 0.05, **p < 0.01, ***p < 0.001.

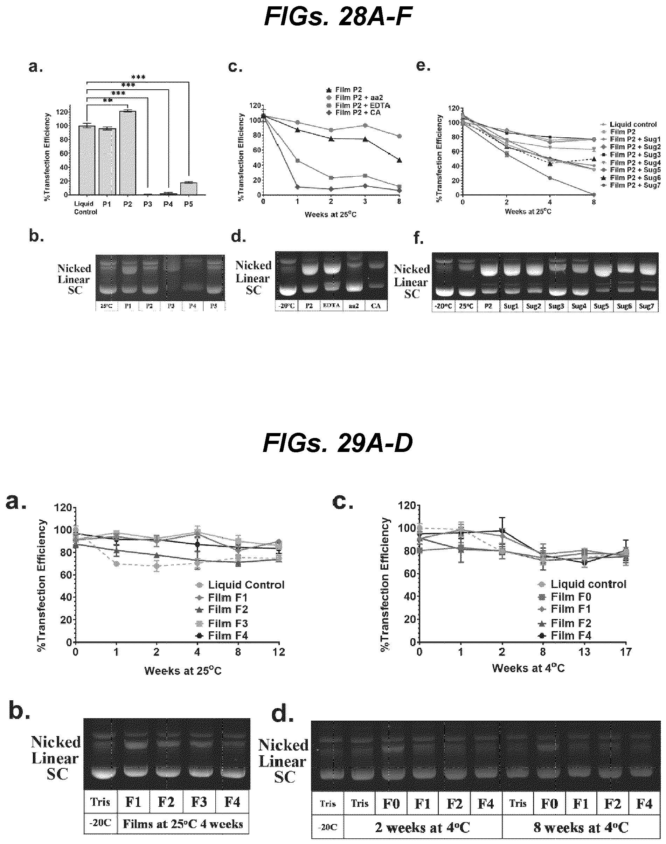

**FIGs. 28A-28F.** Evaluation of Film Forming Polymers, an Amino Acid and Sugars to Stabilize Plasmid DNA within a Film Matrix at 25 °C. **FIG. 28A.** Evaluation of 5 different polymers for their ability to maintain plasmid transfection efficiency during the film forming process. P2 was selected for use in additional studies. **FIG. 28B.** The absence of supercoiled DNA and the presence of notable amounts of nicked DNA were visualized in samples from films prepared with polymers that failed to maintain transfection efficiency after a week at 25 °C. **FIG. 28C.** Addition of an Amino Acid to the Polymer Base Significantly Improves Plasmid Stability at 25 °C. This preparation contained the most supercoiled DNA after 3 weeks at 25 °C than other formulations tested (**FIG. 28D**). **FIG. 28E.** Stability Profiles of Formulations Containing Polymer P2 and Seven Different Sugars. None of the formulations improved the transfection efficiency of plasmid stored in Tris buffer pH 8 (Liquid Control) over the 8 week study period. **FIG. 28F.** Visualization of the three different forms of plasmid DNA at the 8 week time point. Specific details of each formulation denoted in this figure are summarized in **Table 1** and calculation of transfection efficiency is outlined in Example 5. In **FIGs. 28A**, **28C**, and **28E**, data represent the average $\pm$ the standard error of the mean for 4 replicates for each formulation at each time point. Statistical significance between formulations with respect to a liquid control was evaluated by one-way ANOVA with Dunnett's post-hoc multiple comparison tests. **p < 0.01, ***p < 0.001.

**FIGs. 29A-29D.** Incorporation of an Amino Acid and a Sugar within a Film Matrix Consisting of a Plasticizer and a Polymer Significantly Improves Plasmid Stability at 25 °C. **FIG. 29A.** Twelve Week Stability Profile of Plasmid Stored at 25 °C in Different Film Matrices. **FIG. 29B.** Resolution of the 3 different conformations of DNA after 4 weeks at 25 °C. **FIG. 29C.** Seventeen Week Stability Profile of Plasmid Stored at 4 °C. **FIG. 29D.** Resolution of the 3 different conformations of DNA after storage at 4 °C for 2 and 8 weeks. In **FIGs. 29A** and **29C**, the Liquid Control consisted of plasmid in 10 mM Tris pH 8.0 stored at 4 or 25 °C. This corresponds to the "Tris" lane of gels shown in **FIG. 29D**. Specific details of each formulation denoted in this figure are summarized in **Table 1** and calculation of transfection efficiency is outlined in Example 5. Data reported in these panels represent the average $\pm$ the standard error of the mean for 4 replicates per formulation at each timepoint.

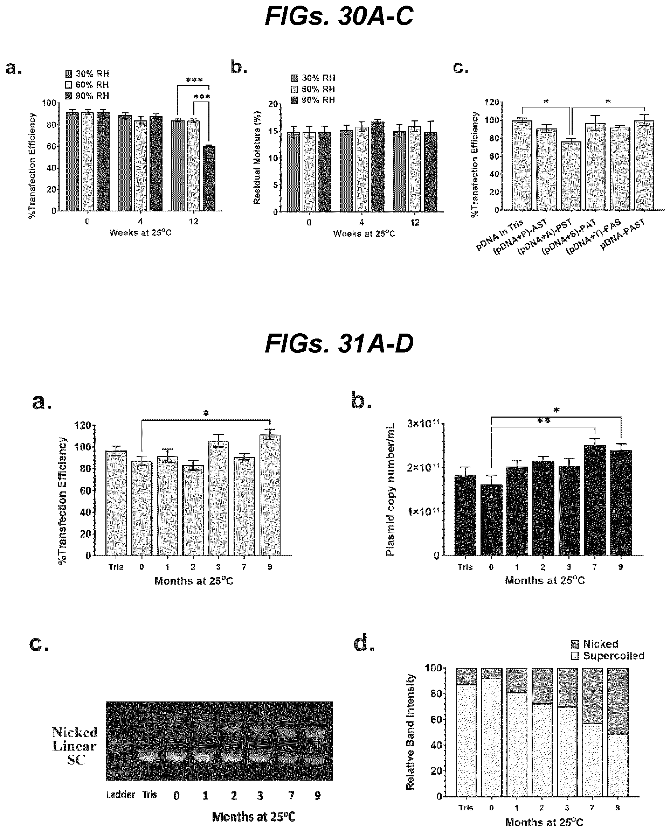

**FIGs. 30A-30C.** Environmental Storage Conditions and Manner in which Excipients are Mixed in the Bulk Formulation Impact Long Term Stability of Plasmids within a Film Matrix. Plasmid-containing films prepared with formulation F4 were packaged and stored at 25 °C in chambers with different relative humidities (RHs). This formulation contained a polymer (P), an amino acid (A), a plasticizer (S) and a sugar (T), collectively, PAST. **FIG. 30A.** Transfection efficiency was not impacted by RH until the 12 weeks when transfection efficiency was reduced by 20% in films stored in 90% RH. Despite this effect, the moisture content within the films did not significantly change over time (**FIG. 30B**). **FIG. 30C.** Addition of the amino acid with the plasmid prior to addition to the bulk formulation (pDNA + A)-PST significantly reduced transfection efficiency after drying. In each panel, data represents the average $\pm$ the standard error of the mean for 3 replicates for each condition and/or timepoint tested. Calculation of transfection efficiency is outlined in Example 5. Statistical significance between storage conditions was determined by two-way ANOVA and Tukey's post hoc multiple comparison tests. *p < 0.05, ***p < 0.001.

**FIGs. 31A-31D.** The PAST Formulation Maintains Transfection Efficiency of Plasmid within a Film Matrix for 9 Months at 25 °C. **FIG. 31A.** Nine Month Stability Profile of Plasmid Embedded within the PAST Matrix at 25 °C. Increased transfection efficiency toward the end of the study correlates with an increase in plasmid copy number (**FIG. 31B**) due to an increase in nicked DNA as visualized by agarose gel electrophoresis (**FIG. 31C**). Further analysis of band intensity revealed an increase in the ratio of nicked : supercoiled DNA in rehydrated samples over time (**FIG. 31D**). In **FIGs. 31A** and **31B**, data represents the average $\pm$ the standard error of the mean for 3 replicates for each timepoint tested. Calculation of transfection efficiency is outlined in Example 5. Statistical significance between data from each timepoint and fresh film (t=0) was determined by two-way ANOVA and Dunnett's post hoc tests. *p < 0.05, **p < 0.01.

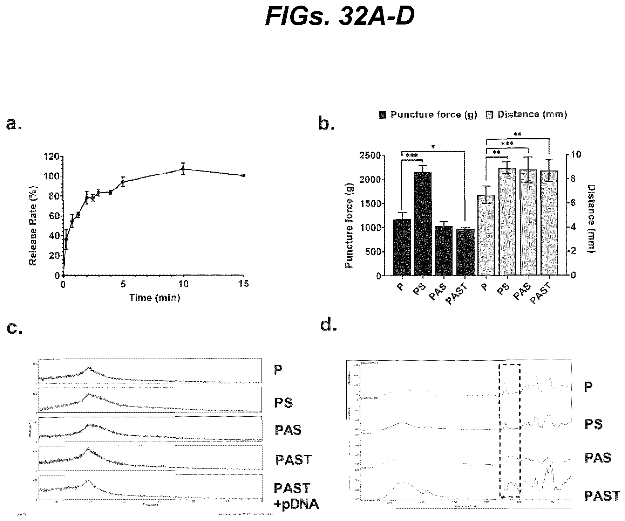

**FIGs. 32A-32D.** Films Prepared with the PAST Formulation are Amorphous Solids and Rapidly Release Plasmid. **FIG. 32A.** Release profile at 37°C in PBS. Data is reported as the fraction of plasmid in the film (100 $\mu$g) found in PBS cumulatively over time. **FIG. 32B.** Presence of amino acid and sugar within the film matrix creates a pliable film. Two components of tensile strength: force needed to puncture a film and distance to cross the film layer are shown here. **FIG. 32C.** X-Ray diffraction. Films remain amorphous solids with addition of each excipient and plasmid. **FIG. 32D.** FITR scans suggest that plasmids are not bound by excipients in the film matrix. In **FIGs. 32A** and **32B**, data represents the average $\pm$ the standard variation of the mean with 5 replicates per formulation and/or timepoint. Statistical significance was determined by one-way ANOVA with Tukey's post hoc test for multiple comparisons between every two formulations. *p < 0.05, **p < 0.01, ***p < 0.001.

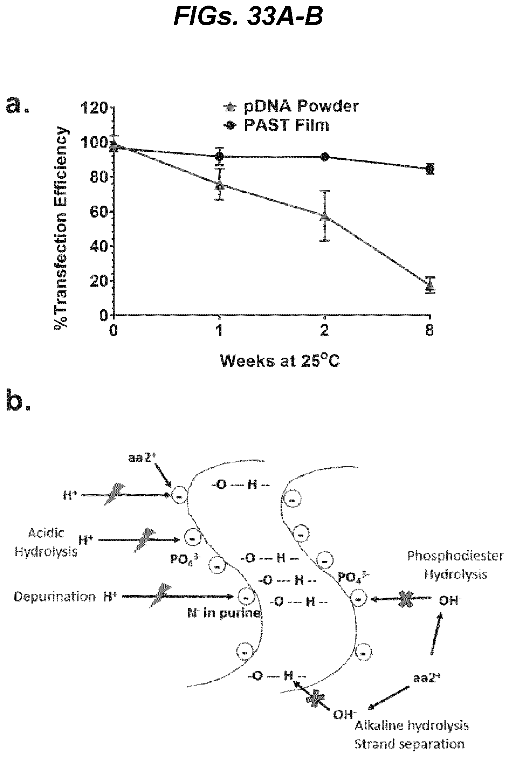

**FIGs. 33A-33B.** Proposed Mechanism for Stabilization of Plasmid DNA within a Film Matrix. **FIG. 33A.** The PAST formulation significantly improves stability of plasmid DNA in the solid state at 25 °C with respect to plasmid dried in Tris buffer only (Powder). **FIG. 33B.** The amino acid present in the film matrix plays a key role in mitigating plasmid degradation. The positively charged amino acid shields electron dense groups such as phosphates, amines or purines along the helical strands of the plasmid, limiting access of free H+ to these regions which in turn limits acidic hydrolysis or depurination (red lightning). The positively charged amino acid can also pull hydroxyl ions (OH-) away from electrophilic hydrogens between the helical strands of the plasmid (red X), further reducing disruption of hydrogen bonds between nucleotides or hydrolysis of phosphodiester bonds along the plasmid.

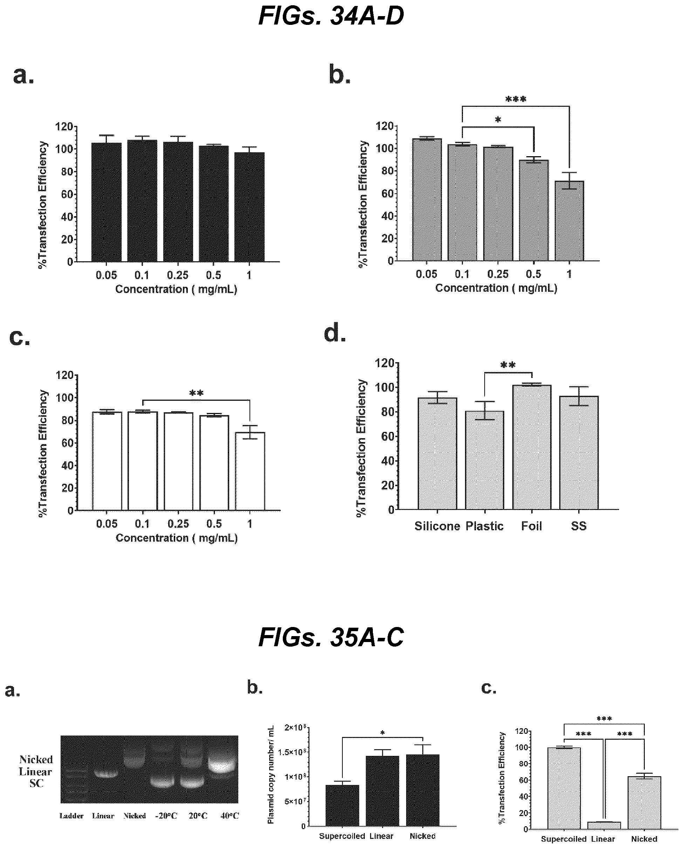

**FIGs. 34A-34D.** Impact of Plasmid Concentration and Mold Composition on Transfection Efficiency of Plasmids Stabilized within a Film Matrix. Transfection efficiency of a liquid film formulation slightly decreased with increasing amounts of plasmid (**FIG. 34A**). This effect was more pronounced when the formulation was dried and rehydrated (**FIG. 34B**) and demonstrated that concentrations of more than 0.5 mg/ml were detrimental to long-term stability within the film matrix (**FIG. 34C**). **FIG. 34D.** Transfection efficiency was significantly compromised when plasmid-containing films were dried in polystyrene (Plastic) molds. While transfection efficiency remained high when films were dried on stainless steel (SS) and foil laminate (Foil) molds, they were not used due to risk of fostering oxidative degradation during long term storage. Silicone molds were used to produce all films in this study. Calculation of transfection efficiency is outlined in the Material and Methods section. Data represent the average $\pm$ the standard error of the mean for 3 replicates for each formulation. In **FIGs. 34A-34C**, statistical significance with respect to the 0.1 mg/ml plasmid concentration was determined by two-way ANOVA followed by a Dunnett's post hoc test. In **FIG. 34D**, statistical significance between different mold surfaces was determined by one-way ANOVA followed by Tukey's post hoc multiple comparison tests. *p < 0.05, **p < 0.01, ***p < 0.001.

**FIGs. 35A-35C.** The Three Conformations of Plasmid DNA and Their Impact on Stability Indicating Assays. **FIG. 35A.** Visualization of linear, nicked and supercoiled plasmid DNA through agarose gel electrophoresis. Placing plasmid stored frozen in Tris buffer at room (20 °C) or elevated (40 °C) temperatures enhanced transition of supercoiled plasmid DNA to the linear and nicked format. **FIG. 35B.** The presence of linear and nicked DNA in a sample can inflate plasmid copy number as determined by quantitative PCR. **FIG. 35C.** Conversion of supercoiled plasmid DNA to the linear and nicked form significantly compromises transfection efficiency. Data represent the average $\pm$ the standard deviation of the mean for 3 replicates for each condition. Statistical significance between the different DNA conformations was determined using a one-way ANOVA followed by a Tukey's post hoc test. *p < 0.05 and ***p < 0.001.

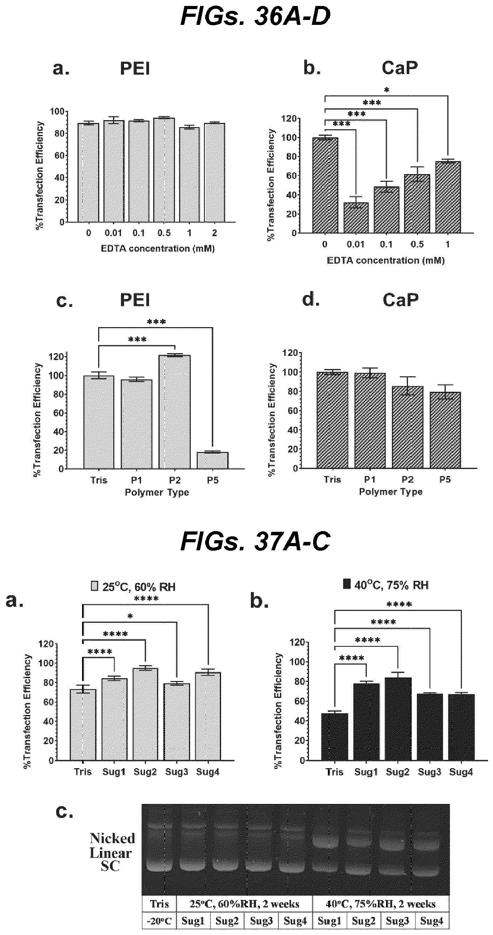

**FIGs. 36A-36D.** Compatibility of Transfection Reagents with Components of the Film Matrix. Polyethyenimine (PEI)-mediated transfection was not affected by the presence of EDTA (**FIG. 36A**) while calcium phosphate (CaP))-mediated transfection was significantly reduced in a concentration dependent manner (**FIG. 36B**). PEI-mediated transfection was impacted by several film forming polymers (**FIG. 36C**) while CaP was only mildly impacted by different polymers (**FIG. 36D**). These results and that PEI provides highly reproducible transfection efficiency supported use of PEI for the remainder of the experiments summarized in this manuscript. Specific details of each formulation denoted in this figure are summarized in **Table 1**. Data represent the average $\pm$ the standard error of the mean of 4 replicates for each formulation. Statistical significance between samples and plasmid in Tris buffer alone (0 mM EDTA in **FIGs. 36A** and **36B**, Tris in **FIGs. 36C** and **36D**) was determined by one-way ANOVA and Dunnett's multiple comparison tests. * p < 0.05, *** p < 0.001.

**FIGs. 37A-37C.** Sugars Impact Plasmid Stability within a Film Matrix Under Standard (25 °C, 60% RH, **FIG. 37A**) and Stressed (40 °C, 75% RH, **FIG. 37B**) Storage Conditions. The most optimal formulations contained more supercoiled DNA after 2 weeks (**FIG. 37C**). Specific details of each formulation denoted in this figure are summarized in **Table 1**. Data represent the average $\pm$ the standard error of the mean for 4 replicates per formulation. In **FIGs. 37A** and **37B**, statistical significance between plasmid stored in Tris buffer under the same condition (Tris) was determined by one-way ANOVA followed by Tukey's post hoc multiple comparison tests. *p < 0.05, ***p < 0.001.

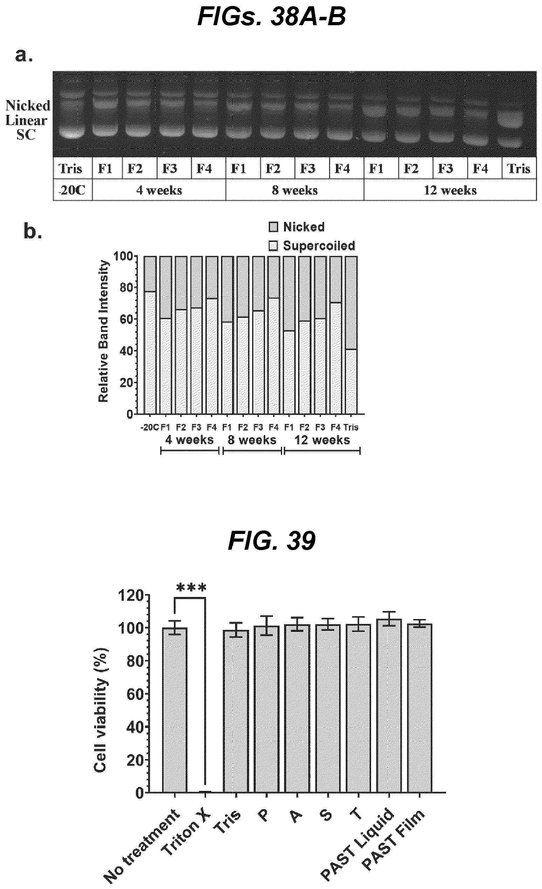

**FIGs. 38A-38B**. Transition of Plasmid DNA Stabilized within Different Film Formulations from the Supercoiled to the Nicked Form Over Twelve Weeks at 25 °C. **FIG. 38A.** Representative agarose gel showing different forms of plasmid DNA in samples of rehydrated film collected after 4, 8 and 12 weeks at 25 °C. **FIG. 38B.** Change in Supercoiled:Nicked Plasmid Ratio During 12 Weeks at 25 °C. The density of supercoiled and nicked bands was analyzed using ImageJ software. Data was calculated as the ratio of the fluorescence intensity of the supercoiled (yellow) or the nicked (grey) DNA band to the sum of the intensities of bands representing the three DNA conformations. Specific details of each formulation denoted in this figure are summarized in **Table 1**.

**FIG. 39.** Individual Excipients and Film Composite are not Cytotoxic After a 2 Hour Exposure on HEK 293 Cells. Specific details of each formulation component denoted in this figure are summarized in **Table 1**. A positive control solution of 0.1% Triton X-100 was used to qualify the assay. Data represent the average $\pm$ the standard error of the mean for 3 replicates for each formulation. Statistical significance between cells maintained in culture media (No Treatment) and each formulation component was determined with one-way ANOVA followed by Dunnett's post hoc multiple comparison tests. ***p < 0.001.

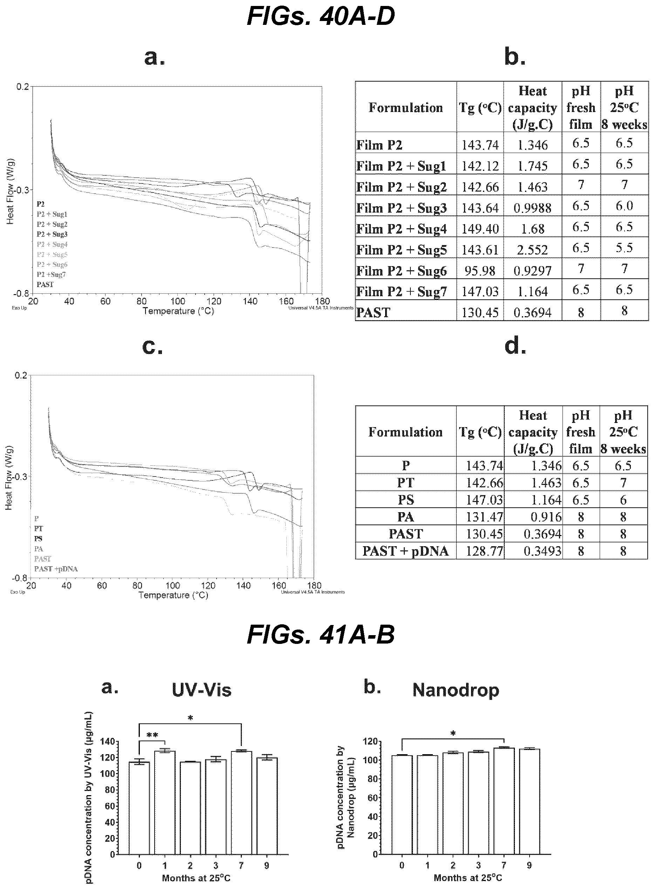

**FIGs. 40A-40D.** Impact of Sugars and an Amino Acid on the Glass Transition Temperature (Tg) of Thin Film Formulations. **FIG. 40A.** DSC Thermograms of Formulations Containing Film Base and Sugars. **FIG. 40B.** Tabular summary of Tg and pH values for film formulations containing sugars. **FIG. 40C.** DSC thermograms of films made from individual excipients of the PAST Formulation. FIG. 40AD. Tabular summary detailing how Tg and pH in the film matrix change in response to each component of the PAST formulation. Specific details of each formulation component denoted in this figure are summarized in **Table 1**. pH was measured by visual inspection of films prepared with Universal pH Indicator.

**FIGs. 41A-41B.** Assessment of DNA content in Plasmid Containing Films Stored for Nine Months at 25 °C, 60% RH. The amount of DNA in rehydrated film was quantitated by measuring absorbance at 260 nm using by a standard UV-Vis (**FIG. 41A**) or Nanodrop (**FIG. 41B**) spectrophotometer. Data was normalized to the concentration of plasmid in Tris buffer stored frozen at -20 °C. Data represents the average $\pm$ the standard error of the mean for 3 replicates for each time point. Statistical significance with respect to data collected at the start of the study (t=0) was determined by one-way ANOVA followed by Dunnett's post hoc tests. * p < 0.05, ** p < 0.01.

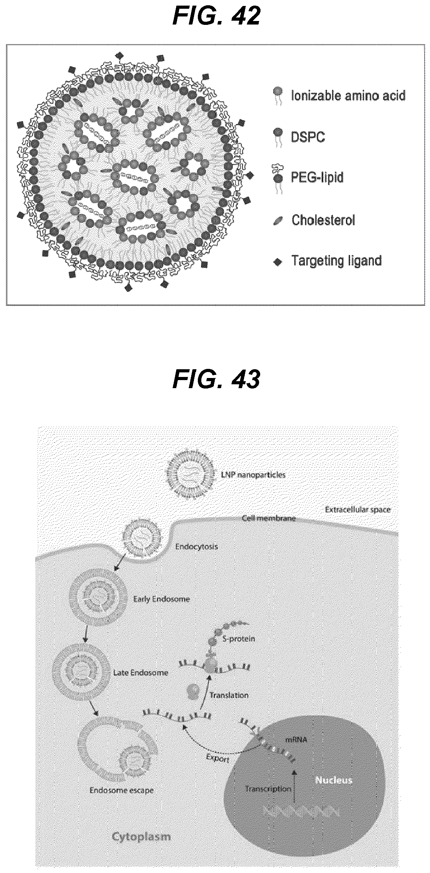

**FIG. 42.** General structure of a lipid nanoparticle.

**FIG. 43**. Intracellular fate of mRNA lipid nanoparticles.

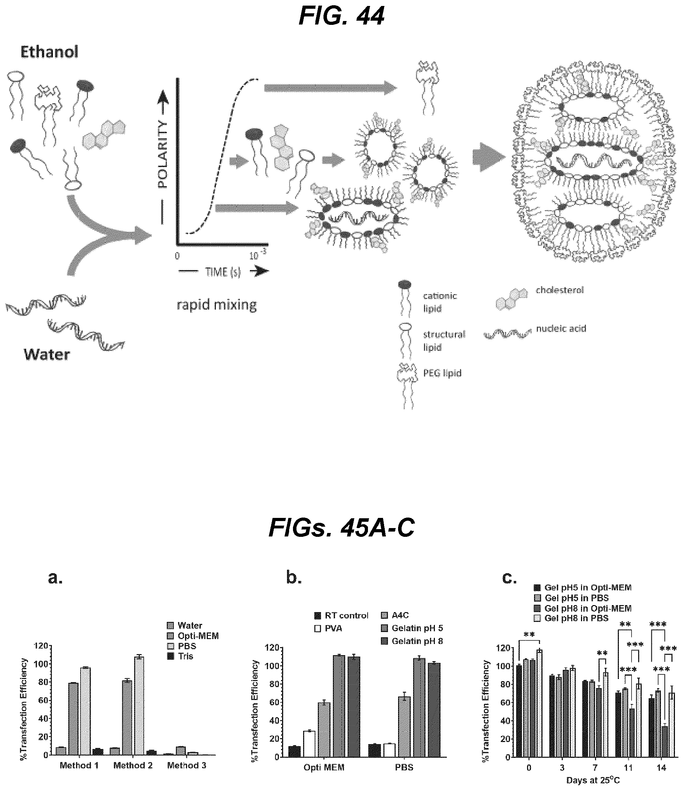

**FIG. 44.** Representative mRNA LNP manufacturing process.

**FIGs. 45A-45C**. Transfection Efficiency of PEI-DNA Complexes is Maintained within a Film Matrix. **FIG. 45A**. Analysis of Different Methods of Complex Formation Prior to Addition to Film Formulation on Transfection Efficiency. Method 1: Equal volume mixture of PEI and DNA. Method 2: Dropwise addition of PEI to plasmid. Method 3: Dropwise addition of plasmid to PEI. In each case, transfection efficiency of complexes containing 1 $\mu$g DNA in 32 $\mu$l media following dilution to 1 $\mu$g DNA in 100 $\mu$l media were normalized to a control prepared at 1 $\mu$g DNA in 100 $\mu$l media. **FIG. 45B**. Impact of Media Recovery of Transfection Efficiency During the Film Forming Process. **FIG. 45C**. Stability of PEI-DNA complexes in films stored at 25 °C, 60% RH for 2 weeks. In each panel, % Transfection Efficiency is expressed as the amount of beta-galactosidase present in a given cell population for each formulation with respect to cells transfected with fresh complexes made from plasmid stored at -20 °C in Tris buffer. Data represent the average $\pm$ the standard error of the mean for 4 replicates for each formulation. Statistical significance between formulations with respect to unformulated complexes was determined by one-way ANOVA followed by Tukey's multiple comparison tests **p < 0.01, ***p < 0.001.

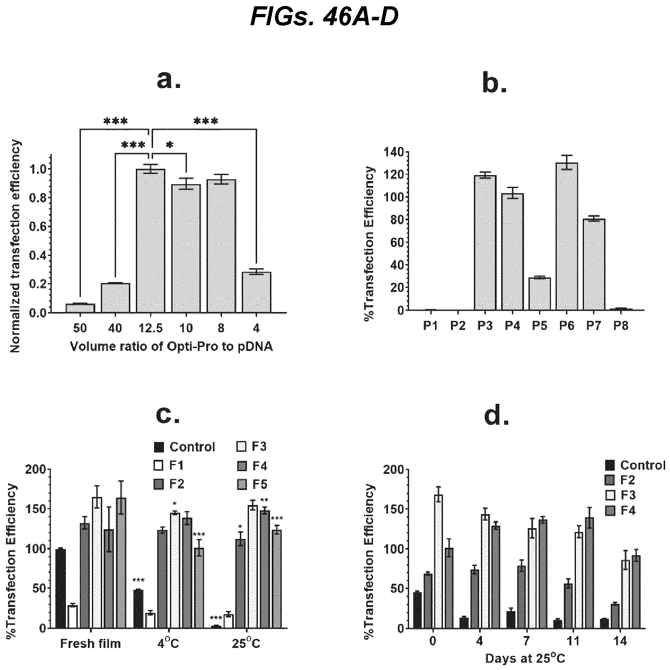

**FIGs. 46A-46D**. Transfection Efficiency of Lipofectamine (LPF)-DNA Complexes is Enhanced During Storage at 25 °C within a Film Matrix. **FIG. 46A**. Impact of Media Volume Ratio on Transfection Efficiency of LFP-DNA Complexes. **FIG. 46B**. Impact of Polymer on Transfection Efficiency During the Film Forming Process. **FIG. 46C**. Stability of LPF-DNA complexes within Films After 4 days at 4 °C and 25 °C. **FIG. 46D**. Stability of LPF-DNA complexes over 14 days at 25 °C. Specific details of each formulation denoted in this figure are summarized in **Table 4**. In each panel, %Transfection Efficiency is the amount of beta-galactosidase present in a given cell population for each formulation with respect to cells transfected with fresh complexes made from plasmid stored at -20 °C. Data represent the average $\pm$ the standard error of the mean of 4 replicates for each formulation. Statistical significance between formulations with respect to unformulated complexes placed in the same condition was determined by one-way ANOVA followed by Dunnett's multiple comparison tests. *p < 0.05, **p < 0.01, ***p < 0.001.

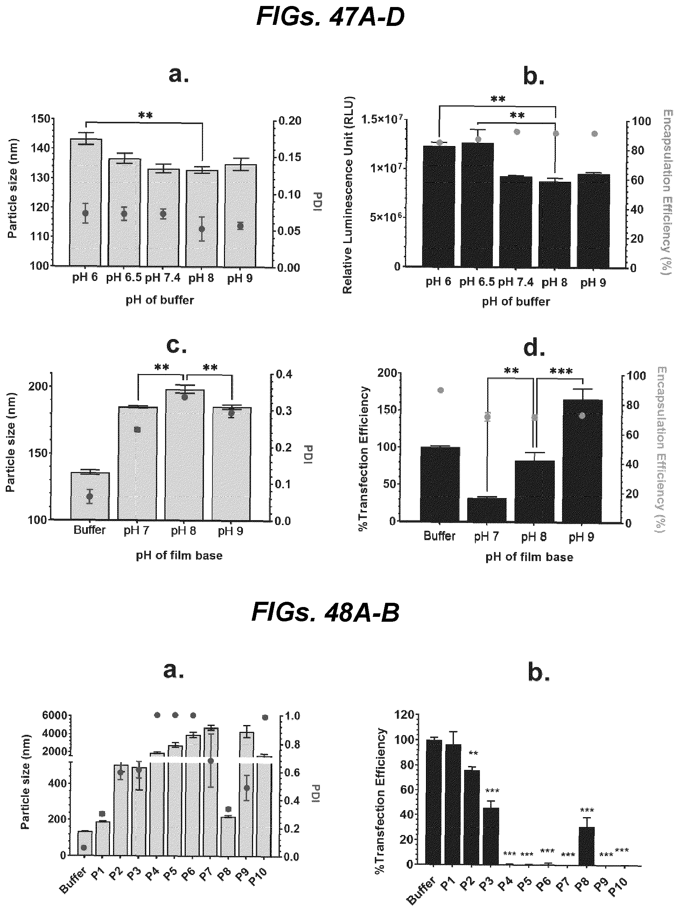

**FIGs. 47A-47D**. Impact of pH on LNP stability in Solution and within the Film Matrix. Impact of pH on Particle Size and PDI (blue dots, **FIG. 47A**) and Transfection and Encapsulation Efficiency (orange dots, **FIG. 47B**) of LNPs in Solution for 3 days at 4 °C. Impact of pH of K100LV HPMC on the particle size and PDI (**FIG. 47C**) and Transfection and Encapsulation Efficiency (**FIG. 47D**) of LNPs with respect to an unthawed stock solution (Buffer) once the film forming process was complete. Buffers used in this study were: 20 mM Histidine (pH 6 and 6.5), 150 mM PBS (pH 7), 10 mM Tris (pH 8 and 9). In each panel, data represent the average $\pm$ the standard error of the mean for 3 replicates for each formulation. Statistical analysis was conducted with two-way ANOVA and Dunnett's post hoc test in which properties of LNPs in a formulation were compared to that seen in Tris pH 8. \*\*p<0.01, \*\*\*p < 0.001.

**FIGs. 48A-48B**. Impact of Polymer on the Preservation of Particle Size (**FIG. 48A**) and Transfection Efficiency (**FIG. 48B**) of LNPs During the Film Forming Process. LNPs were placed in low viscosity polymer formulations and dried under ambient and aseptic conditions. Films were then reconstituted with 0.9% NaCl for analysis and comparison to an unthawed stock solution (Buffer). Specific details of formulations utilized in this figure are summarized in **Table 3**. Data represent the average $\pm$ the standard error of the mean for 3 replicates for each formulation. Statistical significance with respect to freshly thawed LNPs in Tris buffer was determined by two-way ANOVA and Dunnett's post hoc tests. \*\*p < 0.01, \*\*\*p < 0.001.

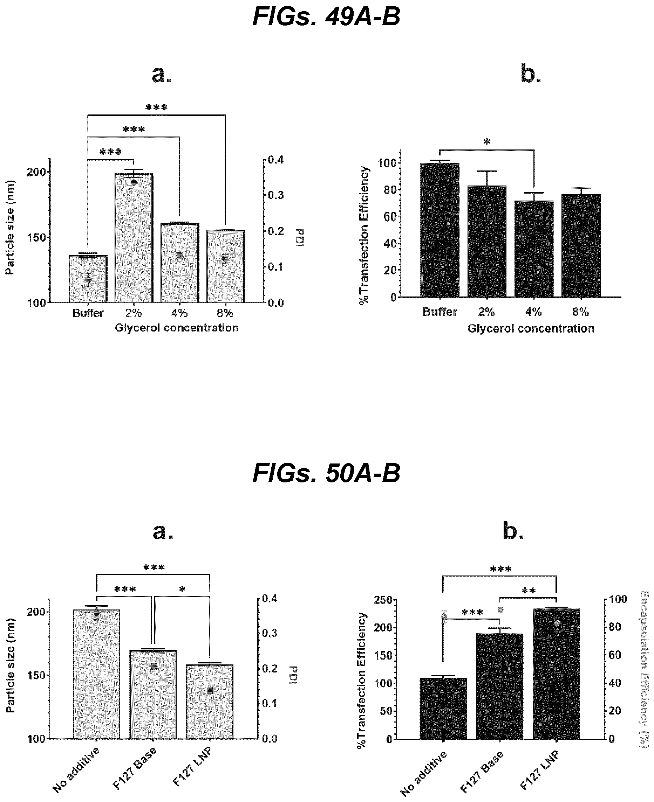

**FIGs. 49A-49B**. Glycerol Improves Stabilization of mRNA Containing LNPs within the Film Matrix. **FIG. 49A**. Glycerol at concentrations of greater than 2% v/v maintain particle size and PDI (blue dots) of LNPs with respect to an unthawed stock solution (Buffer). **FIG. 49B**. Impact of glycerol on transfection efficiency of LNPs during the film forming process. The formulation utilized in these studies was K100LV HPMC in 10 mM Tris buffer pH 8. In each panel, data represent the average $\pm$ the standard error of the mean for 3 replicates for each formulation. Statistical analysis was conducted with two-way ANOVA and Dunnett's post hoc test in which properties of mRNA LNPs in a film formulation were compared to that of freshly thawed mRNA LNPs in Tris buffer. \*p<0.05, \*\*\*p < 0.001.

**FIGs. 50A-50B**. Addition of Pluronic F127 to LNPs Before Incorporation into the Film Matrix Significantly Improves Physical and Biological Properties During the Drying Process. Pluronic F127 was added either directly to the film formulation (1.5% K100LV, 3% glycerol) before mixing with LNPs (F127 Base) or to the LNP stock prior to mixing with the bulk formulation (F127 LNP). **FIG. 50A**. Impact of mixing on particle size and PDI (blue dots). **FIG. 50B**. Impact of mixing on transfection and encapsulation (orange dots) efficiencies. In each panel, data represent the average $\pm$ the standard error of the mean for 3 replicates for each formulation. Statistical significance between each condition was determined by two-way ANOVA and Tukey's multiple comparison tests. \*p < 0.05, \*\*p < 0.01, \*\*\*p < 0.001.

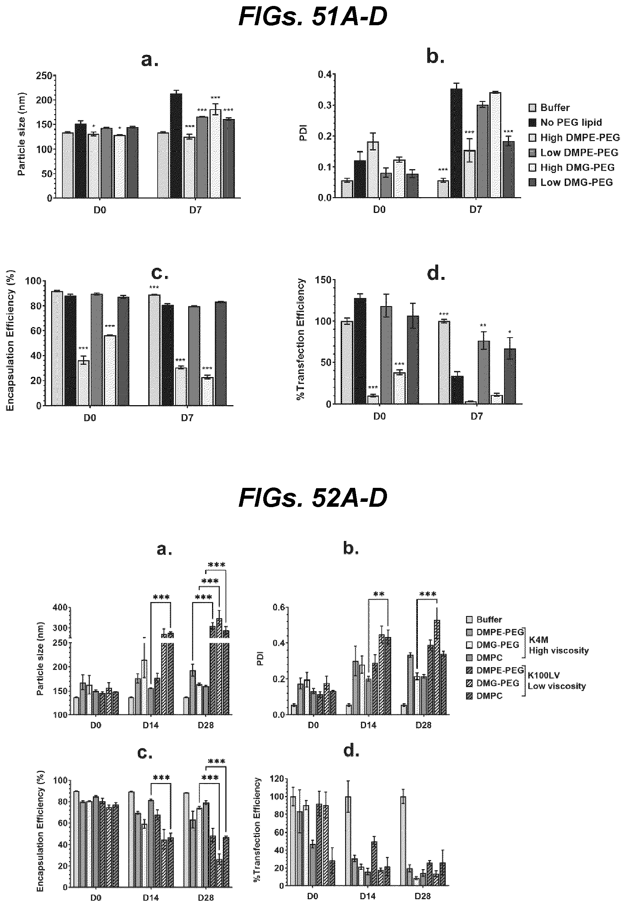

**FIGs. 51A-51D**. Use of PEG Lipids as Excipients Improves LNP stability within the Film Matrix at 4 °C. PEG conjugated lipids were included in bulk film formulation (1.5% K100LV + 3% Glycerol + 0.01% Pluronic F127) at high (0.04%) and low (0.008%) concentrations. Freshly prepared films (D0) and those stored at 4 °C for a week (D7) were rehydrated for measurement of particle size (**FIG. 51A**), PDI (**FIG. 51B**), encapsulation efficiency (**FIG. 51C**) and transfection efficiency (**FIG. 51D**). Specific details of formulations utilized in this figure are summarized in **Table 4**. Data generated from frozen stock LNPs stored at 400 $\mu$g/ml (Buffer) was also included for comparison. In each panel, data represent the average $\pm$ the standard error of the mean for 3 replicates for each formulation. Statistical between formulations with and without PEG-lipid was determined using two-way ANOVA and Dunnett's post hoc tests. \*p < 0.05, \*\*p < 0.01, \*\*\*p < 0.001.

**FIGs. 52A-52D**. Formulations Containing High Viscosity Polymer and DMPC Lipid Preserved Physical Properties of mRNA LNPs but Failed to Support Transfection Efficiency at 4 °C. Individual lipids (DMPE-PEG at 0.008%, DMG-PEG at 0.008% or DMPC at 0.01%) were added to bulk formulations containing either K4M (solid bars) or K100LV (hatched bars) HPMC. Films were rehydrated immediately after drying (D0) and storage at 4°C fir 14 and 28 days for measurement of particle size (**FIG. 52A**), PDI (**FIG. 52B**), encapsulation efficiency (**FIG. 52C**) and transfection efficiency (**FIG. 52D**). Specific details of formulations utilized in this figure are summarized in **Table 4**. In each panel, data represent the average $\pm$ the standard error of the mean for 3 replicates for each formulation. Statistical differences between K4M and K100LV formulations containing the same lipid component was determined using two-way ANOVA and Tukey's multiple comparison tests. \*\*p < 0.01, \*\*\*p < 0.001.

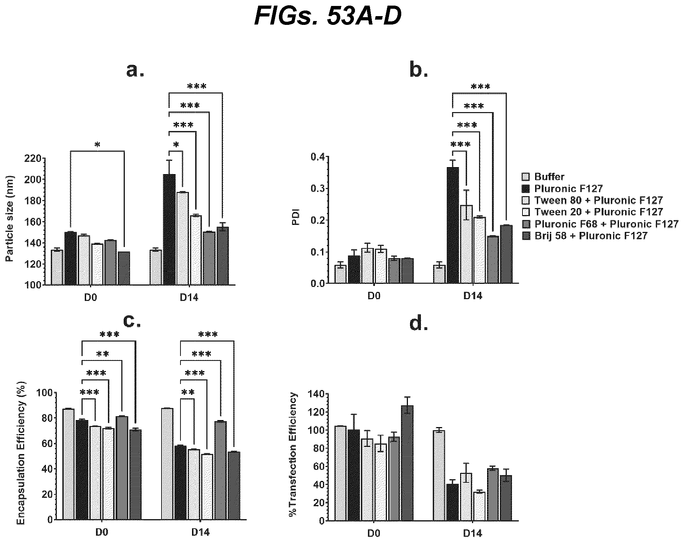

**FIGs. 53A-53D**. Combinations on Non-Ionic Surfactants within the Film Matrix Improves Physical Characteristics of LNPs but Fails to Improve Transfection Efficiency After 14 Days at 4 °C. Combinations of Pluronic F127 and a second non-ionic surfactant were added to LNPs prior to mixing with K4M HPMC. Films in full packaging were stored at 4 °C for 2 weeks and then rehydrated for measurement of particle size (**FIG. 53A**), PDI (**FIG. 53B**), encapsulation efficiency (**FIG. 53C**) and transfection efficiency (**FIG. 53D**). Specific details of formulations are listed in **Table 4**. In each panel, data represent the average $\pm$ the standard error of the mean for 3 replicates for each formulation. Statistical differences between formulations containing 2 surfactants and that containing Pluronic F127 was determined by two-way ANOVA and Dunnett's post hoc tests . \*p<0.05, \*\*p<0.01, \*\*\*p < 0.001.

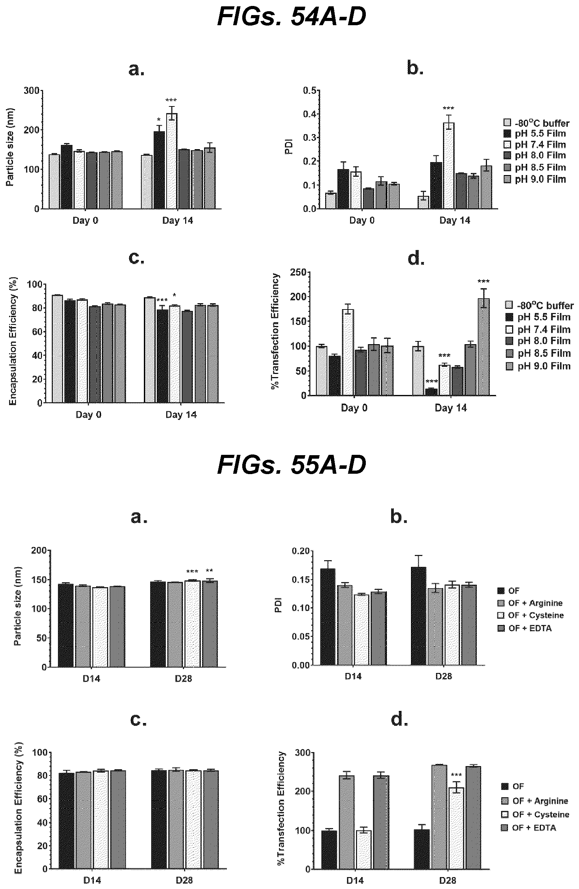

**FIGs. 54A-54D**. Buffers of pH 8.5 Preserve Physical and Biological Properties of LNPs within the Film Matrix During Storage at 4 °C. Film base was prepared with several different buffers ranging in pH from pH 5.5 - 9.0. The internal pH of films prepared with these buffers and their composition is summarized in **Table 6**. Films were stored at 4 °C for 14 days and rehydrated with 10mM Tris buffer pH 8 to determine particle size (**FIG. 54A**), PDI (**FIG. 54B**), encapsulation efficiency (**FIG. 54C**) and transfection efficiency (**FIG. 54D**). In each panel, data represent the average $\pm$ the standard error of the mean for 3 replicates for each formulation. Statistical significance between measurements taken on day 0 and day 14 within the same formulation was determined by two-way ANOVA and Sidak's multiple comparison tests. p<0.05, **p<0.01, ***p < 0.001.

**FIGs. 55A-55D**. Excipients that Prevent Degradation of Naked DNA within a Film Matrix Significantly Improved LNP stability at 4 °C for 28 days. Arginine (0.05%), cysteine (0.005%) and EDTA (1mM) were added individually to an optimized formulation (OF, **Table 4**). Films in full packaging were stored at 4 °C for up to 28 days and rehydrated with 10mM Tris buffer pH 8 for measurement of particle size (**FIG. 55A**), PDI (**FIG. 55B**), encapsulation efficiency (**FIG. 55C**) and transfection efficiency (**FIG. 55D**). In each panel, data represent the average $\pm$ the standard error of the mean for 3 replicates for each formulation. Statistical significance between LNP properties on day 14 and day 28 within the same film formulation was determined by two-way ANOVA and Sidak's multiple comparison tests. **p<0.01, ***p < 0.001

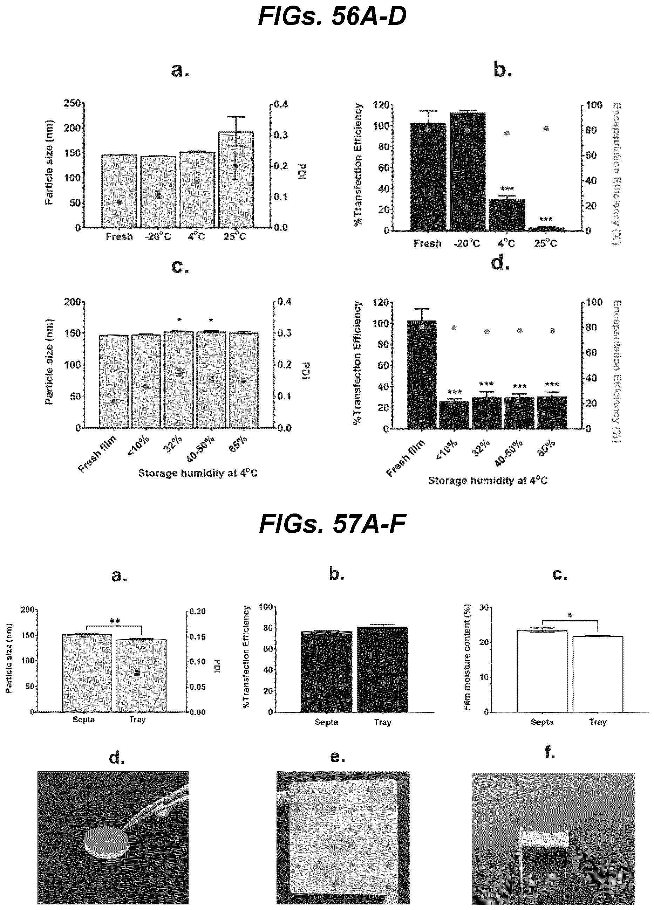

**FIGs. 56A-56D**. Storage Temperature Impacts Biological Properties of mRNA LNPs with in a Film Matrix More Than Changes in Relative Humidity at 4 °C. **FIG. 56A**. Impact of Storage Temperature on Particle Size and PDI (blue dots) of LNPs. **FIG. 56B**. Impact of Storage Temperature on Encapsulation (orange dots) and Transfection Efficiency of LNPs. **FIG. 56C**. Impact of Relative Humidity on Particle Size and PDI (blue dots) of LNPs. **FIG. 56D**. Impact of Relative Humidity on Encapsulation (orange dots) and Transfection Efficiency of LNPs. In each panel, films were prepared with optimized formulation as described in **Table 4** (OF). Different humidity conditions were established using desiccants or Boveda packs. In each panel, data represent the average $\pm$ the standard error of the mean for 3 replicates for each formulation. Statistical differences between fresh and 2 week old films for each formulation was determined using two-way ANOVA and Dunnett's multiple comparison tests. p<0.05, **p<0.01, ***p < 0.001.

**FIGs. 57A-57F**. Mold design significantly impacts physical properties. Films of the optimized formulation in 10 mM Tris buffer pH 8.0 (OF formulation, **Table 4**) were cast on singular silicone septa in the form of flat disk or in silicone tray with individual wells. Films were rehydrated for measurement of particle size and PDI (**FIG. 57A**), transfection efficiency (**FIG. 57B**) or used directly to moisture determination by Karl Fisher titration (**FIG. 57C**). A picture of silicon septa was captured in **FIG. 57D** while the silicon tray was shown in the form of multiple wells (**FIG. 57E**) and the cross section of a single well (**FIG. 57F**). Data represent the average $\pm$ the standard error of the mean for 3 replicates for each mold type. *p<0.05, **p<0.01, ***p < 0.001. Statistical analysis was performed by unpaired t-test.

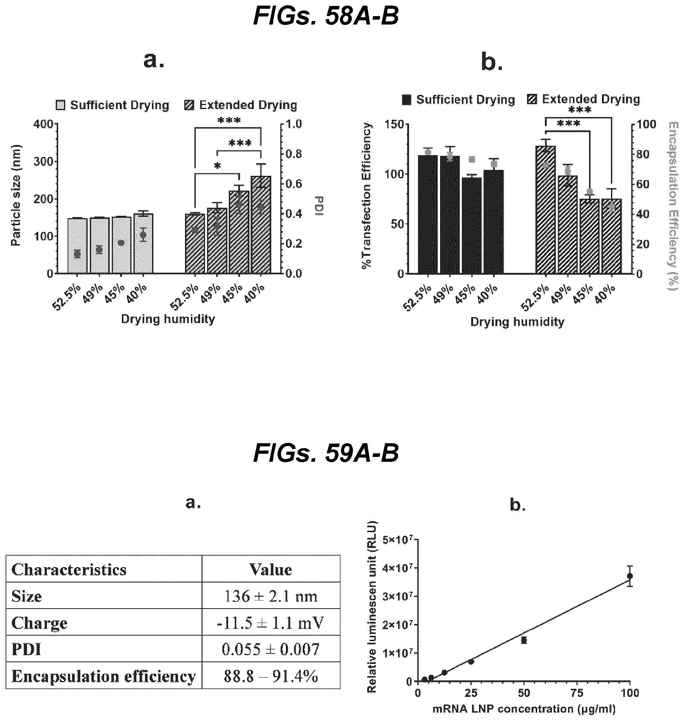

**FIGs. 58A-58B**. Manufacturing of LNP containing films within a Low Humidify Environment and Over Drying Significantly Impacts Physical and Biological Properties. Films containing mRNA LNPs were dried in a chamber under controlled airflow, temperature (20 °C) and different relative humidity conditions. For each condition, four films were collected at two time points: Sufficient Drying, when films looked visually dry and Extended Drying, one hour after films were determined dry by visual inspection. Films were immediately rehydrated and analyzed for particle size and particle distribution (blude dots, **FIG. 58A**), transfection and encapsulation efficiency (orange dots, **FIG. 58B**). Optimized formulation made in Tris buffer pH 8 was used in this study (**Table 4**, OF formulation). In each panel, data represent the average $\pm$ the standard error of the mean for 3 replicates for each formulation. Statistical differences between films prepared under each condition was determined by two-way ANOVA and Tukey's post hoc tests. *p<0.05, ***p < 0.001.

**FIGs. 59A-59B**. Physical and Biological Properties of LNP Stock. **FIG. 59A**. Physical properties of particles as determined by dynamic light scattering and a Ribogreen assay. **FIG. 59B**. Linear relationship between luciferase expression and amount of LNP utilized for assessment of transfection efficiency.

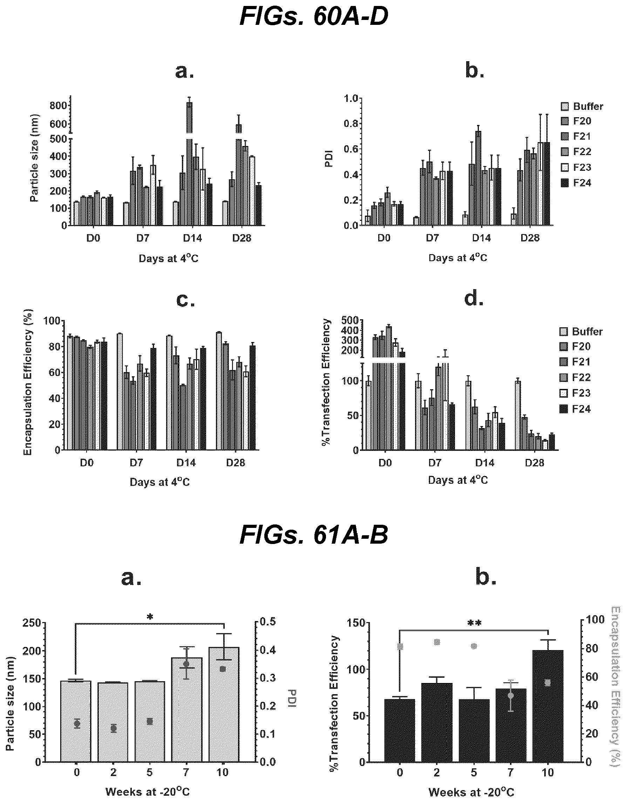

**FIGs. 60A-60D**. Impact of Polymer Viscosity on Physical and Biological Properties of LNPs after Storage within a Film Matrix at 4 °C for 28 Days. Formulations of varying viscosity (56-169 cp) were prepared by mixing K100LV (low viscosity HPMC) with K4M (high viscosity HPMC). Specific details of formulation F20-F24 are summarized in **Table 4**. Films in full packaging were stored at 4 °C and collected at 1, 2, 4 weeks. They were then rehydrated and particle size (**FIG. 60A**), PDI (**FIG. 60B**), encapsulation efficiency (**FIG. 60C**) and transfection efficiency (**FIG. 60D**) assessed. In each panel, data represents the average $\pm$ the standard error of the mean for 3 replicates for each formulation.

**FIGs. 61A-61B**. Stability Profile of LNPS within A Film Matrix at -20 °C for 10 Weeks. Films containing LNPS and an optimized formulation in 10 mM Tris pH 8 were dried and placed in particle free Ziploc-like bags and grouped in triplicate to be packaged in heat-sealed foil bags for storage at -20 °C. At each timepoint, films were warmed to room temperature and rehydrated for analysis of particle size and PDI (blue dots, **FIG. 61A**), as well as encapsulation (orange dots, **FIG. 61B**) and transfection efficiency. Optimized formulation (OF) in Tris buffer pH 8 was used in this study with formulation details in **Table 4**. In each panel, data represent the average $\pm$ the standard error of the mean for 3 replicates for each formulation. Statistical significance between properties of LNPs in aged and fresh films (t=0) was determined by two-way ANOVA and Dunnett's post hoc tests. *p<0.05, **p < 0.01.

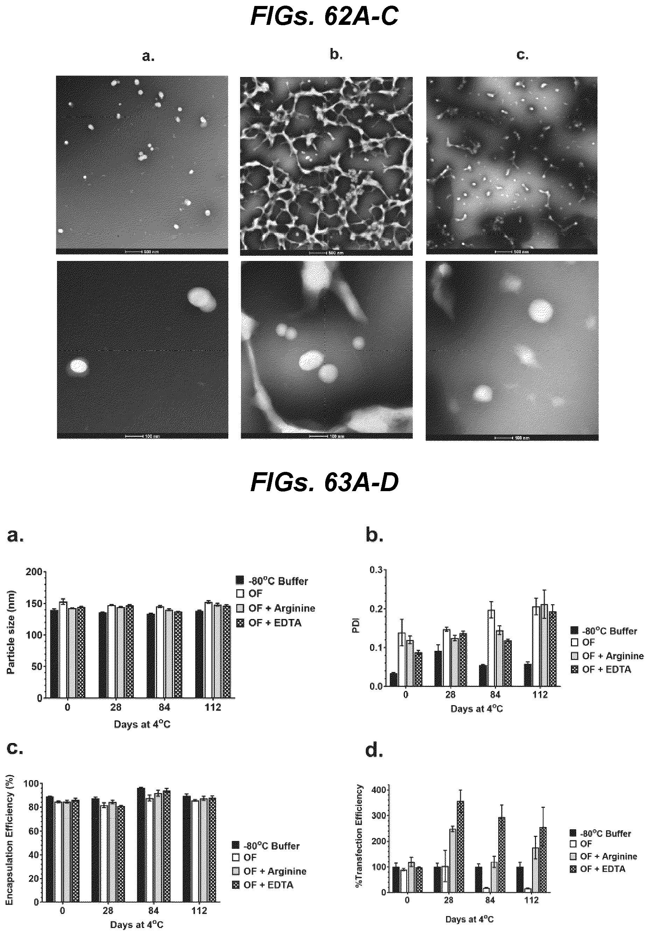

**FIGs. 62A-62C**. TEM images of mRNA LNPs in film. Each panel showed images of mRNA LNPs in specific formulation at two magnifications. The upper row presents low magnification captured with ruler at 500 nm and the bottom row presents high magnification captured with ruler at 100 nm. **FIG. 62A**. mRNA LNPs in Tris buffer pH 8.0. **FIG. 62B**. mRNA LNPs in film base of optimized formulation before drying. **FIG. 62C**. mRNA LNPs in optimized formulation film after drying and rehydration. Average size of particle diameter estimated by ImageJ in **FIGs. 62A**, **62B**, and **62C** are 116 $\pm$ 13 nm, 115 $\pm$ 13 nm and 125 $\pm$ 14 nm, respectively. The background captured in **FIG. 62B** might come from film-forming polymer as the major component of film formulation, which network was broken down into separate moieties scattering in **FIG. 62C**.

**FIGs. 63A-63D**. Stability Profile of mRNA LNPs at 4°C for 112 Days. Arginine (0.05%) and EDTA (1mM) were added individually to the optimized formulation as described in **Table 4** (OF). Films in full packaging were stored at 4 °C for up to 112 days and rehydrated with 10mM Tris buffer pH 8 for measurement of particle size (**FIG. 63A**), PDI (**FIG. 63B**), encapsulation efficiency (**FIG. 63C**), and transfection efficiency (**FIG. 63D**). In each panel, data represent the average $\pm$ the standard error of the mean for 3 replicates for each formulation.

## DETAILED DESCRIPTION

Aspects of the present disclosure relate generally to compositions comprising agents (e.g., therapeutic agents), including nucleic acids, formulated in stabilizing carriers, including liquid carriers and substantially solid carriers such as a thin film matrix. Also disclosed are methods for storage and delivery of such agents (e.g., therapeutic agents) (e.g., polypeptides, small molecules, nucleic acids). As described herein, the disclosed formulations provide compositions and methods for stable storage of agents (e.g., therapeutic agents) (e.g., polypeptides, small molecules, nucleic acids) at ambient temperatures. As disclosed herein, agents (e.g., therapeutic agents) (e.g., polypeptides, small molecules, nucleic acids) stabilized as disclosed herein retain significant biological activity even after extended storage conditions that would normally inactivate the compositions. Thus, the compositions of the disclosure offer significant advantages relative to previous formulations for agents (e.g., therapeutic agents) (e.g., polypeptides, small molecules, nucleic acids), which may require refrigeration or even freezing to maintain activity for any significant period of time. This allows previously highly unstable compositions to be stored and transported over large distances with-out the typically required cold chain. Another advantage of the provided compositions is that the composition may foster storage of agents (e.g., therapeutic agents) (e.g., nucleic acids) at concentrations that largely exceed solubility limits, but which may be desired for administration and/or use without compromising the physical stability and performance of the agent. This is a significant advantage over lyophilization and conventional formulations. Specific methods of generating the formulations described herein and also of using the formulations (e.g., formulations thereby generated), to deliver agents (e.g., therapeutic agents) (e.g., polypeptides, small molecules, nucleic acids) to a subject are also encompassed. Particular aspects are directed to dissolvable formulations and delivery of such formulations *via* intravenous, intramuscular, intranasal, sublingual, or buccal methods.

### I. Compositions

In some aspects, the present disclosure provides a composition including an agent (e.g., a therapeutic agent) (e.g., a polypeptide, small molecule, nucleic acid, and/or components thereof), such as for use in gene delivery/gene therapy, vaccine delivery, etc., dispersed within a liquid or amorphous solid carrier, such as an amorphous film/thin film matrix. The agent (e.g., a therapeutic agent) and/or components thereof, may include, e.g., a whole organism, killed, attenuated or live (including killed, attenuated viruses); a subunit or portion of an organism; a recombinant vector containing an insert; a piece or fragment of a nucleic acid (DNA, RNA, etc.) associated with a bacteria, fungus, parasite, or virus, recombinant bacteria, fungus, parasite, or virus, a bacterial, fungal, parasitic, or viral vector, and/or components thereof; a protein, a polypeptide, a peptide associated with a bacteria, fungus, parasite, or virus, recombinant bacteria, fungus, parasite, or virus, a bacterial, fungal, parasitic, or viral vector, and/or components thereof, such as, for example, capsid proteins and/or empty capsids, bacterial, fungal, parasitic, or viral particles and/or infectious particles; a small molecule targeting any of the foregoing; or any combination thereof.

In some aspects, the agent (e.g., a therapeutic agent) and/or components thereof may be derived from or target (e.g., inhibit), without departing from the scope of the disclosure, a piece or fragment of a nucleic acid (DNA, RNA, etc.) and/or a protein, a polypeptide, a peptide, or fragment thereof from viruses including, but not limited to, those from the family *Arenaviridae* (e.g., Lymphocytic choriomeningitis virus), *Arterivirus* (e.g., Equine arteritis virus), *Astroviridae* (Human astro virus 1), *Birnaviridae* (e.g., Infectious pancreatic necrosis virus, Infectious bursal disease virus), *Bunyaviridae* (e.g., California encephalitis virus Group), *Caliciviridae* (e.g., Caliciviruses), *Coronaviridae* (e.g., Human coronaviruses 299E and OC43, SARS-CoV-1, SARS-CoV-2), *Deltavirus* (e.g., Hepatitis delta virus), *Filoviridae* (e.g., Marburg virus, Ebola virus), *Flaviviridae* (e.g., Yellow fever virus group, Hepatitis C virus), *Hepadnaviridae* (e.g., Hepatitis B virus), *Herpesviridae* (e.g., Epstein-Bar virus, Simplexvirus, Varicellovirus, Cytomegalovirus, Roseolovirus, Lymphocryptovirus, Rhadino virus), *Orthomyxoviridae* (e.g., Influenzavirus A, B, and C), *Papovaviridae* (e.g., Papillomavirus), *Paramyxo-*

*vindae* (e.g., Paramyxovirus such as human parainfluenza virus 1 , Morbillivirus such as Measles virus, Rubulavirus such as Mumps virus, Pneumovirus such as Human respiratory syncytial virus), *Picornaviridae* (e.g., Rhinovirus such as Human rhinovirus 1A, Hepatovirus such Human hepatitis A virus, Human poliovirus, Cardiovirus such as Encephalomyocarditis virus, Aphthovirus such as Foot-and-mouth disease virus 0, Coxsackie virus), *Poxyiridae* (e.g., Orthopoxvirus such as Variola virus or monkey poxvirus), *Reoviridae* (e.g., Rotavirus such as Groups A-F rotaviruses), *Retroviridae* (Primate lentivirus group such as human immunodeficiency virus 1 and 2), *Rhabdoviridae* (e.g., rabies virus), *Togaviridae* (e.g., Rubivirus such as Rubella virus), Human T-cell leukemia virus, Murine leukemia virus, Vesicular stomatitis virus, Wart virus, Blue tongue virus, Sendai virus, Feline leukemia virus, Simian virus 40, Mouse mammary tumor virus, Dengue virus, HIV-1 and HIV-2, West Nile, H1N1, SARS, 1918 Influenza, Tick-borne encephalitis virus complex (Absettarov, Hanzalova, Hypr), Russian Spring-Summer encephalitis virus, Congo-Crimean Hemorrhagic Fever virus, Junin Virus, Kumlinge Virus, Marburg Virus, Machupo Virus, Kyasanur Forest Disease Virus, Lassa Virus, Omsk Hemorrhagic Fever Virus, FIV, SIV, Herpes simplex 1 and 2, Herpes Zoster, Human parvovirus (B19), Respiratory syncytial virus, Pox viruses (all types and serotypes), Coltivirus, Reo viruses - all types, and/or Rubivirus (rubella); bacteria and fungi including, but not limited to, *Streptococcus agalactiae, Legionella pneumophilia, Streptococcus pyogenes, Escherichia coli, Neisseria gonorrhosae, Neisseria meningitidis, Pneumococcus, Hemophilis influenzae B, Treponema pallidum,* Lyme disease spirochetes, *Pseudomonas aeruginosa, Mycobacterium leprae, Brucella abortus, Mycobacterium tuberculosis, Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Babesia bovis, Elmeria tenella, Onchocerca volvulus, Leishmania tropica, Trichinella spiralis, Theileria parva, Taenia hydatigena, Taenia ovis, Taenia saginata, Echinococcus granulosus, Mesocestoides corti, Mycoplasma arthritidis, M. hyorhinis, M. orale, M. arginini, Acholeplasma laidlawii, M. salivarium, M. pneumoniae, Candida spp., Candida albicans, Cryptococcus spp., Cryptococcus neoformans, Histoplasma spp., Histoplasma capsulatum, Coccidioides spp., Coccidioides immitis, Blastomyces spp., Blastomyces dermatitidis, Aspergillus spp., Aspergillus fumigatus, Pneumocystis spp., Penicillium marneffei, Bacillus anthracis, Bartonella, Bordetella pertussis, Brucella - all serotypes, Chlamydia trachomatis, Chlamydia pneumoniae, Clostridium botulinum, Clostridium - all serotypes, Haemophilus influenzae, Helicobacter pylori, Klebsiella - all serotypes, Legionella - all serotypes, Listeria, Mycobacterium - all serotypes, Mycoplasma - human and animal serotypes, Rickettsia - all serotypes, Shigella - all serotypes, Staphylococcus aureus, Streptococcus - S. pneumoniae, S. pyogenes, Vibrio cholera, Yersinia enterocolitica,* and/or *Yersinia pestis;* parasites including, but not limited to, *Ancylostoma* human hookworms, *Leishmania* - all strains, *Microsporidium, Necator* human hookworms, *Onchocerca filarial* worms, *Plasmodium* - all human strains and simian species, *Toxoplasma* - all strains, *Trypanosoma* - all serotypes, and/or *Wuchereria bancrofti* filarial worms; combination of any two or more of the foregoing, or any type of bacterial or viral vector by which one of skill in the art would contemplate for gene delivery.

In some aspects, the agent (e.g., a therapeutic agent) and/or components thereof may include a heterologous nucleic acid that encodes for a heterologous polypeptide that may have biological function and/or activity, and/or may include a heterologous nucleic acid that encodes for a heterologous nucleic acid that may have biological function and/or activity, for example, but not limited to, RNAi, crRNA, enhancer RNA, long non-coding RNA, microRNA, sRNA, and/or shRNA. For example, in some aspects, the heterologous nucleic acid may express a polypeptide having biological function and/or activity, and express and RNA having biological function and/or activity that directs/targets expression of a polypeptide to particular cells, tissues, and/or organs in a subject.

Heterologous nucleic acids can include DNA (e.g., genomic DNA or recombinant DNA or plasmid DNA) and RNA (e.g., mRNA, RNAi, crRNA, enhancer RNA, long non-coding RNA, microRNA, sRNA, and/or shRNA). In one aspect, the nucleic acid is plasmid DNA, and the composition can comprise between 0.05 mg/mL and 1 mg/mL, e.g., at least, at most, exactly, or between any two of 0.05, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, or 1.00 mg per mL, plasmid DNA. In one aspect, the nucleic acid is genomic DNA, and the composition can comprise between 0.05 mg/mL and 2 g/mL, e.g., at least, at most, exactly, or between any two of 0.05, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 1.55, 1.60, 1.65, 1.70, 1.75, 1.80, 1.85, 1.90, 1.95, or 2.00 mg or g per mL, genomic DNA. In one aspect, the nucleic acid is mRNA, and the composition can comprise between 0.001 mg/mL and 0.5 mg/mL, e.g., at least, at most, exactly, or between any two of 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, or 0.50 mg per mL, mRNA.

The nucleic acid can be naked, e.g., nucleic acid that is not associated with proteins, lipids, or any other protective molecule. Alternatively, the nucleic acid can be associated with one or more lipids, proteins, carbohydrates, and or other organic molecules. Additionally or alternatively, the nucleic acid can be associated with one or more transfection reagents (e.g., LIPOFECTAMINE® (a mixture of DOSPA (2,3-dioleoyloxy-N-[2(sperminecarboxamido)ethyl]-N,N-dimethyl-1-propaniminium trifluoroacetate) and DOPE (1,2-Dioleoyl-sn-glycerophosphoethanolamine)), calcium phosphate, polyethylenimine).

In specific aspects, compositions disclosed herein can include lipids and a heterologous nucleic acid (e.g., DNA, mRNA, RNAi, crRNA, enhancer RNA, long non-coding RNA, microRNA, sRNA, and/or shRNA), and the lipids and heterologous nucleic acid can together form nanoparticles, thereby producing heterologous nucleic acid-containing nanoparticles comprising lipids. The lipids can encapsulate or associate with the heterologous nucleic acid in the form of a lipid nanoparticle (LNP) to aid stability, cell entry, and intracellular release of the heterologous nucleic acid/lipid nanoparticles. A LNP can comprise, e.g., a micelle, a solid lipid nanoparticle, a nanoemulsion, a liposome, etc., or a combination thereof. The lipid component of a LNP may include, for example, a cationic lipid, a phospholipid (such as an unsaturated lipid, e.g., DOPE or DSPC), a polymer-lipid conjugate (e.g., a PEGylated lipid), a structural lipid (e.g., cholesterol), an ionizable lipid, a neutral lipid, or any combination thereof. The elements of the lipid component may be provided in specific fractions. Suitable cationic lipids, phospholipids, polymer-lipid conjugates, structural lipids, ionizable lipids, and neutral lipids and the specific fractions at which these lipids should be provided for the compositions and methods of the present disclosure are known in the art. In addition to these lipid components, lipid nanoparticles may include any substance useful in pharmaceutical compositions. For example, the lipid nanoparticle may include one or more pharmaceutically acceptable excipients or accessory ingredients such as, but not limited to, one or more solvents, dispersion media, diluents, dispersion aids, suspension aids, surface active agents, buffering agents, preservatives, and other species.

The chemical properties of the LNP, LNP suspension, lyophilized LNP composition, or LNP formulation of the present disclosure may be characterized by a variety of methods. For example, microscopy (e.g., transmission electron microscopy or scanning electron microscopy) may be used to examine the morphology and size distribution of an LNP. Dynamic light scattering or potentiometry (e.g., potentiometric titrations) may be used to measure zeta potentials. Dynamic light scattering may also be utilized to determine particle sizes. Instruments such as the Zetasizer Nano ZS (Malvern Instruments Ltd, Malvern, Worcestershire, UK) may also be used to measure multiple characteristics of a LNP, such as particle size, polydispersity index, and zeta potential.

In some aspects, between 0.001 and 10 ng, $\mu$g, or mg per mL of the agent (e.g., a therapeutic agent) (e.g., a polypeptide, small molecule, nucleic acid) is included in the formulation, e.g., at least, at most, equal to, or between any two of 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2.0, 3.0, 4.0, 5.0, 6.0, 7.0, 8.0, 9.0, and 10 ng, $\mu$g, or mg per mL of the agent (e.g., a therapeutic agent). In some aspects, a composition may comprise about $1 \times 10^6$ to about $1 \times 10^{13}$ virus particles per mL or about $1 \times 10^3$ to about $1 \times 10^{13}$ bacterial colony forming units per mL. When the formulation is in non-liquid form (e.g., substantially solid film) the measurement of agent (e.g., a therapeutic agent) may be referred to as the ng, $\mu$g, or mg per mL or virus particles per mL or colony forming units per mL of the liquid prior to conversion into the non-liquid form, for convenience. As discussed herein, when the amount of agent (e.g., a therapeutic agent) is referred to in a composition in non-liquid form, in units of ng, $\mu$g, or mg per mL or virus particles per mL or colony forming units per mL, this is what is intended. Without being bound by theory, it is believed that the disclosed formulations increase the ability to store higher concentrations of agent (e.g., a therapeutic agent) while avoiding aggregation, and therefore allows storage and use at higher concentrations.

A composition of the disclosure may comprise 1, 2, 3, 4, or more different components disclosed herein. For example, a composition of the disclosure may comprise an agent (e.g., a therapeutic agent) (e.g., a nucleic acid) and an additional component, such as one or more lipids, peptides, or small molecules. A composition of the disclosure may comprise two or more components (e.g., a nucleic acid and a lipid, a nucleic acid and a peptide, a nucleic acid and a small molecule, etc.) where all of the components in the composition retain biological activity (e.g., immunogenicity, biological activity, etc.) when stored at ambient temperature (e.g., between 15 °C and 30 °C) for at least 1, 2, 3, 4, 5, 6, 7, 8, 9, 10, 20, 30, 40, 50, 60, 70, 80, 90, or 100 days, or more, or any range or value derivable therein.

In some aspects, a composition of the disclosure is an amorphous solid, such as a substantially solid film. In some aspects, an amorphous solid suitable for use in the present disclosure is dissolvable upon contact with an aqueous liquid. In some aspects, amorphous solids suitable for use in the present disclosure may be formed from any sugar, sugar derivative, or combination of sugars/derivatives so long as the sugar and/or derivative is prepared as a liquid solution at a concentration that allows it to flow freely when poured but also forms an amorphous phase at ambient temperatures on a physical surface that facilitates this process, such as aluminum or polytetrafluoroethylene. Examples of suitable sugars include, but are not limited to glucose, dextrose, fructose, lactose, maltose, xylose, sucrose, corn sugar syrup, sorbitol, hexitol, maltitol, xylitol, mannitol, melezitose, raffinose, cyclodextrin (e.g., alpha-cyclodextrin, beta-cyclodextrin, gamma-cyclodextrin, hydroxypropyl-beta-cyclodextrin, or methoxy-beta-cyclodextrin), and a combination thereof. In some aspects, a composition of the disclosure comprises trehalose. In some aspects, a composition of the disclosure comprises sorbitol. In some aspects, a composition of the disclosure comprises trehalose and sorbitol. In some aspects, a composition of the disclosure comprises sucrose. In some aspects, a composition of the disclosure comprises cyclodextrin. In some aspects, a composition of the disclosure comprises a sugar that is not sorbitol, trehalose, and/or sucrose. Any one or more of the foregoing sugars may be explicitly excluded from the compositions of the disclosure, in some aspects. In certain aspects, it may be desirable for the properties of the sugar and/or derivative to allow preparation as a liquid solution at a concentration that allows it to flow freely when poured but also forms an amorphous phase at ambient temperatures on a physical surface that facilitates this process, such as aluminum or polytetrafluoroethylene. While not being bound to any particular theory, it is believed that sugars minimize interaction of the agent (e.g., a therapeutic agent) molecules (e.g., polypeptide, nucleic acid, antigen, antibody, small molecule) with water during storage and drying, in turn, preventing damage to the three-dimensional shape due to crystal formation during the drying process and subsequent loss of efficacy.

In some aspects, an amorphous solid suitable for use in the present disclosure may have a thickness of about 0.05 millimeters to about 5 millimeters, e.g., at least, at most, equal to, or between any two of 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, 1.95, 2.0, 2.05, 2.1, 2.15, 2.2, 2.25, 2.3, 2.35, 2.4, 2.45, 2.5, 2.55, 2.6, 2.65, 2.7, 2.75, 2.8, 2.85, 2.9, 2.95, 3.0, 3.05, 3.1, 3.15, 3.2, 3.25, 3.3, 3.35, 3.4, 3.45, 3.5, 3.55, 3.6, 3.65, 3.7, 3.75, 3.8, 3.85, 3.9, 3.95, 4.0, 4.05, 4.1, 4.15, 4.2, 4.25, 4.3, 4.35, 4.4, 4.45, 4.5, 4.55, 4.6, 4.65, 4.7, 4.75, 4.8, 4.85, 4.9, 4.95, or 5.0 mm.

In some aspects, the amorphous solid may include an amount of moisture following drying. For example, amorphous solids of the disclosure may have a moisture content of about 1-30%, e.g., at least, at most, equal to, or between any two of 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%, after drying. In some aspects, amorphous solids of the disclosure may comprise plasmid or genomic DNA and may have a moisture content of about 10-20%, e.g., at least, at most, equal to, or between any two of 10%, 11%, 12%, 13%, 14%, 15%, 16%, 17%, 18%, 19%, or 20%, after drying. In some aspects, amorphous solids of the disclosure may comprise plasmid or genomic DNA and may have a moisture content of about 14-17% after drying. In some aspects, amorphous solids of the disclosure may comprise mRNA LNPs and may have a moisture content of about 20-30%, e.g., at least, at most, equal to, or between any two of 20%, 21%, 22%, 23%, 24%, 25%, 26%, 27%, 28%, 29%, or 30%, after drying. In some aspects, amorphous solids of the disclosure may comprise mRNA LNPs and may have a moisture content of about 22-25% after drying. In a preferred embodiment, a given % value in respect of the moisture content of an amorphous solid as described in this paragraph, and/or elsewhere in the present application, refers to a % weight to weight (w/w), that is the % weight of the residual moisture relative to the weight of the amorphous solid. For example, the value of 10% (w/w) of residual moisture may correspond to 10g residual moisture per 100g of amorphous solid.

In addition, in some aspects, certain sugars may also function as a binder which may provide "substance" to pharmaceutical preparations that contain small quantities of very potent medications for ease of handling/administration. They may also hold components together or promote binding to surfaces (like film backing) to ease drug delivery and handling. Lastly, they may also contribute to the overall pharmaceutical elegance of a preparation by forming uniform glasses upon drying.

Compositions of the present disclosure also may include a water-soluble polymer including, but not limited to, carboxymethyl cellulose, carboxyvinyl polymers, high amylose starch, hydroxyethyl cellulose, hydroxypropyl cellulose, hydroxypropyl methylcellulose (HPMC), methylmethacrylate copolymers, polyacrylic acid, polyvinyl alcohol, polyvinyl pyrrolidone, gelatin, pullulan, sodium alginate, poly(lactic-co-glycolic acid), poly(ethylene) oxide, poly(hydroxyalkanoate), polyethylene glycol, and a combination thereof. Any one or more of the foregoing water-soluble polymers may be explicitly excluded from the compositions of the disclosure, in some aspects.

In some aspects, the water-soluble polymer is chosen to provide particular characteristics to the composition. In some aspects, the water-soluble polymer is chosen to provide particular characteristics to the composition, for example, following reconstitution in solution. In some aspects, the water-soluble polymer is HPMC. Grades of HPMC encompassed by the disclosure include, for example, more viscous grades, such as K4M, E10M, and/or J75MS HPMC, as well as less viscous grades, such as K100LV, A4M, A15LV, E4M, F4M, E6LV (also "E6 Premium LV"), and/or F50 HPMC, but are not limited thereto.

It is envisioned that varying molecular weight HPMC may be used in the formulations and methods described herein. Varying the molecular weight of the HPMC in the composition will result in different viscosities of the formulation. In some aspects, the HPMC has a molecular weight that produces a viscosity of from about 12 cp to about 4000 cp (including any range or value derivable therein) at a concentration of 2% in water. In some aspects, the HPMC has a MW that produces a viscosity of less than 4000 cp, less than 3000 cp, less than 2000 cp, less than 1800 cp, or less than 400 cp at a concentration of 2% in water. In some aspects, the HPMC has a MW that produces a viscosity of from about 12 cp to about 3000 cp or from about 12 cp to about 1800 cp at a concentration of 2% in water. In some aspects, the HPMC has a MW that produces a viscosity of from about 12 cp to about 400 cp, from about 12 cp to about 100 cp, or from about 100 cp to about 400 cp at a concentration of 2% in water. In some aspects, a formulation, solution, or composition of the disclosure comprises an HPMC having a molecular weight that produces a viscosity that is at least, at most, about, or exactly 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, 1510, 1520, 1530, 1540, 1550, 1560, 1570, 1580, 1590, 1600, 1610, 1620, 1630, 1640, 1650, 1660, 1670, 1680, 1690, 1700, 1710, 1720, 1730, 1740, 1750, 1760, 1770, 1780, 1790, 1800, 1810, 1820, 1830, 1840, 1850, 1860, 1870, 1880, 1890, 1900, 1910, 1920, 1930, 1940, 1950, 1960, 1970, 1980, 1990, 2000, 2010, 2020, 2030, 2040, 2050, 2060, 2070, 2080, 2090, 2100, 2110, 2120, 2130, 2140, 2150, 2160, 2170, 2180, 2190, 2200, 2210, 2220, 2230, 2240, 2250, 2260, 2270, 2280, 2290, 2300, 2310, 2320, 2330, 2340, 2350, 2360, 2370, 2380, 2390, 2400, 2410, 2420, 2430, 2440, 2450, 2460, 2470, 2480, 2490, 2500, 2510, 2520, 2530, 2540, 2550, 2560, 2570, 2580, 2590, 2600, 2610, 2620, 2630, 2640, 2650, 2660, 2670, 2680, 2690, 2700, 2710, 2720, 2730, 2740, 2750, 2760, 2770, 2780, 2790, 2800, 2810, 2820, 2830, 2840, 2850, 2860, 2870, 2880, 2890, 2900, 2910, 2920, 2930, 2940, 2950, 2960, 2970, 2980, 2990, 3000, 3010, 3020, 3030, 3040, 3050, 3060, 3070, 3080, 3090, 3100, 3110, 3120, 3130, 3140, 3150, 3160, 3170, 3180, 3190, 3200, 3210, 3220, 3230, 3240, 3250, 3260, 3270, 3280, 3290, 3300, 3310, 3320, 3330, 3340, 3350, 3360, 3370, 3380, 3390, 3400, 3410, 3420, 3430, 3440, 3450, 3460, 3470, 3480, 3490, 3500, 3510, 3520, 3530, 3540, 3550, 3560, 3570, 3580, 3590, 3600, 3610, 3620, 3630, 3640, 3650, 3660, 3670, 3680, 3690, 3700, 3710, 3720, 3730, 3740, 3750, 3760, 3770, 3780, 3790, 3800, 3810, 3820, 3830, 3840, 3850, 3860, 3870, 3880, 3890, 3900, 3910, 3920, 3930, 3940, 3950, 3960, 3970, 3980, 3990, 4000, 4010, 4020, 4030, 4040, 4050, 4060, 4070, 4080, 4090, 4100, 4110, 4120, 4130, 4140, 4150, 4160, 4170, 4180, 4190, 4200, 4210, 4220, 4230, 4240, 4250, 4260, 4270, 4280, 4290, 4300, 4310, 4320, 4330, 4340, 4350, 4360, 4370, 4380, 4390, 4400, 4410, 4420, 4430, 4440, 4450, 4460, 4470, 4480, 4490, 4500, 4510, 4520, 4530, 4540, 4550, 4560, 4570, 4580, 4590, 4600, 4610, 4620, 4630, 4640, 4650, 4660, 4670, 4680, 4690, 4700, 4710, 4720, 4730, 4740, 4750, 4760, 4770, 4780, 4790, 4800, 4810, 4820, 4830, 4840, 4850, 4860, 4870, 4880, 4890, 4900, 4910, 4920, 4930, 4940, 4950, 4960, 4970, 4980, 4990, or 5000 cp at a concentration of 2% in water, including any range or value derivable therein.

Examples of HPMC of varying molecular weights are known and available in the art, and include, without limitation A4C, A4M, A15C, A15LV, E4M, E6LV, F4M, K4M, or K100LV. HPMC of a single molecular weight may be used, or the use of a combination of various molecular weight HPMC, e.g., as disclosed herein, is also envisioned. A composition of the disclosure may comprise at least, at most, about, or exactly 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5% HPMC, including any range or value derivable therein. In some aspects, a composition of the disclosure comprises 1.0%, 1.5%, 2.0% HPMC, or any range or value derivable therein. In some aspects, a composition of the disclosure comprises about or exactly 1.5% HPMC. In some aspects, a composition of the disclosure comprises about or exactly 1% HPMC.

Other water soluble polymers may be substituted in part or in whole for HPMC. Examples of suitable water-soluble polymers include, without limitation, carboxymethyl cellulose, carboxyvinyl polymers, high amylase starch, hydroxyethyl cellulose, hydroxypropyl cellulose, hydroxypropyl methylcellulose, methylmethacrylate copolymers, polyacrylic acid, polyvinyl alcohol, polyvinyl pyrrolidone, gelatin, pullulan, sodium alginate, poly(lactic-co-glycolic acid), poly(ethylene) oxide, poly(hydroxyalkanoate), polyethylene glycol, and combinations thereof. In some aspects, the polymer is gelatin. In some aspects, the polymer is polyvinyl alcohol. In some aspects, the polymer is polyvinyl pyrrolidone. In some aspects, the polymer is pullulan. In some aspects, the polymer is PEG. Any one or more of the foregoing water-soluble polymers may be explicitly excluded from the compositions of the disclosure, in some aspects.

Furthermore, in some aspects, the compositions of the present disclosure may further include one or more oils, polyalcohols, plasticizers, surfactants, permeability enhancers, and/or edible organic acids. Any one or more of the foregoing components may be explicitly excluded from the compositions of the disclosure, in some aspects.

Examples of suitable oils may include, but are not limited to, eucalyptol, menthol, vacrol, thymol, methyl salicylate, verbenone, eugenol, gerianol and a combination thereof. Any one or more of the foregoing oils may be explicitly excluded from the compositions of the disclosure, in some aspects. Examples of suitable polyalcohols may include, but are not limited to, glycerol, polyethylene glycol, propylene glycol, and a combination thereof. In some aspects, a composition of the disclosure comprises glycerol. In some aspects, a composition of the disclosure comprises a polyalcohol that is not glycerol. Any one or more of the foregoing polyalcohols may be explicitly excluded from the compositions of the disclosure, in some aspects. Examples of suitable edible organic acids may include, but are not limited to, citric acid, malic acid, tartaric acid, fumaric acid, phosphoric acid, oxalic acid, ascorbic acid and a combination thereof. Any one or more of the foregoing edible organic acids may be explicitly excluded from the compositions of the disclosure, in some aspects. Examples of suitable surfactants may include, but are not limited to, difunctional block copolymer surfactants terminating in primary hydroxyl groups, such as poloxamer 188 (i.e., PLURONIC® F-68) or poloxamer 407 (i.e., PLURONIC® F-127) commercially available from BASF, poly(ethylene) glycol 3000, dodecyl-β-D-maltopyranoside, disodium PEG-4 cocamido MIPA-sulfosuccinate (DMPS), etc. Any one or more of the foregoing surfactants may be explicitly excluded from the compositions of the disclosure, in some aspects.

In some aspects, a composition of the disclosure may include a zwitterionic molecule or compound, such as a zwitterionic surfactant, or an amino acid or an amino acid derivative, or ethylenediaminetetraacetic acid (EDTA). In some aspects, the zwitterionic molecule is a molecule which contains a group which is a positively charged and a group which is negatively charged. In some aspects, both the positively charged and negatively charged groups exist at physiological pH such that the molecule has a net neutral charge. In some aspects, the positively charged group includes a protonated or quaternary ammonium. In some aspects, the negatively charged group includes a sulfate, a phosphate, or a carboxylate. The zwitterionic molecule can further include one or more lipid groups consisting essentially of an alkyl, cycloalkyl, or alkenyl groups. Preferably, the zwitterionic molecule includes one or more lipid groups consisting essentially of an alkyl, cycloalkyl, or alkenyl groups with a carbon chain of more than 12 carbon atoms. In some aspects, the lipid group has a carbon chain of 12-30 carbon atoms. In some aspects, the lipid group has a carbon chain of 12-24 carbon atoms. In some aspects, the lipid group has from 12, 13, 14, 15, 16, 17, 18, 19, 20, 21, 22, 23, to 24 carbons, or any range derivable thereof. In some aspects, the zwitterionic molecule is a polymeric structure which contains multiple zwitterionic groups and multiple lipid groups on a central backbone. In some aspects, the zwitterionic molecule is a polymer which has from about 50 to about 200 repeating units wherein each repeating unit includes one positively charged group, one negatively charged group, and one lipid group. In some aspects, the zwitterionic molecule is a polymer which has a 75 to 150 repeating units. In some aspects, the central backbone is an alkyl, polyethylene glycol, or polypropylene chain. In some aspects, the central chain is an alkyl group.

Non-limiting examples of zwitterionic surfactants include 3-(N,N-Dimethyltetradecylammonio)propanesulfonate (SB3-14), 3-(4-Heptyl)phenyl-3-hydroxypropyl)dimethylammoniopropanesulfonate (C7BzO), 3-(decyldimethylammonio) propanesulfonate inner salt (SB3-10), 3-(dodecyldimethylammonio) propanesulfonate inner salt (SB3-12), 3-(N,N-dimethyloctadecylammonio) propanesulfonate (SB3-18), 3-(N,N-dimethyl-octylammonio) propanesulfonate inner salt (SB3-8), 3-(N,N-dimethylpalmitylammonio) propanesulfonate (SB3-16), 3-[N,N-dimethyl(3-myristoylaminopropyl)ammonio]propane-sulfonate (ASB-14), CHAPS, CHAPSO, acetylated lecithin, alkyl(C12-30) dialkylamine-N-oxide apricotamidopropyl betaine, babassuamidopropyl betaine, behenyl betaine, bis 2-hydroxyethyl tallow glycinate, C12-14 alkyl dimethyl betaine, canolamidopropyl betaine, capric/caprylic amidopropyl betaine, capryloamidopropyl betaine, cetyl betaine, 3-[(Cocamidoethyl)dimethylammonio]-2-hydroxypropanesulfonate, 3-[(Cocamidoethyl)dimethyl-ammonio]propanesulfonate, cocamidopropyl betaine, cocamidopropyl dimethylamino-hydroxypropyl hydrolyzed collagen, N-[3-cocamido)-propyl]-N,N-dimethyl betaine, potassium salt, cocamidopropyl hydroxysultaine, cocamidopropyl sulfobetaine, cocaminobutyric acid, cocaminopropionic acid, cocoamphodipropionic acid, coco-betaine, cocodimethylammonium-3-sulfopropylbetaine, cocoiminodiglycinate, cocoiminodipropionate, coco/oleamidopropyl betaine, cocoyl sarcosinamide DEA, DEA-cocoamphodipropionate, dihydroxyethyl tallow glycinate, dimethicone propyl PG-betaine, N,N-dimethyl-N-lauric acid-amidopropyl-N-(3-sulfopropyl)-ammonium betaine, N,N-dimethyl-N-myristyl-N-(3-sulfopropyl)-ammonium betaine, N,N-dimethyl-N-palmityl-N-(3-sulfopropyl)-ammonium betaine, N,N-dimethyl-N-stearamidopropyl-N-(3-sulfopropyl)-ammonium betaine, N,N-dimethyl-N-stearyl-N-(3-sulfopropyl)-ammonium betaine, N,N-dimethyl-N-tallow-N-(3-sulfopropyl)-ammonium betaine, disodium caproamphodiacetate, disodium caproamphodipropionate, disodium capryloamphodiacetate, disodium capryloamphodipropionate, disodium cocoamphodiacetate, disodium cocoamphodipropionate, disodium isostearoamphodipropionate, disodium laureth-5 carboxyamphodiacetate, disodium lauriminodipropionate, disodium lauroamphodiacetate, disodium lauroamphodipropionate, disodium octyl b-iminodipropionate, disodium oleoamphodiacetate, disodium oleoamphodipropionate, disodium PPG-2-isodeceth-7 carboxyamphodiacetate, disodium soyamphodiacetate, disodium stearoamphodiacetate, disodium tallamphodipropionate, disodium tallowamphodiacetate, disodium tallowiminodipropionate, disodium wheatgermamphodiacetate, N,N-distearyl-N-methyl-N-(3-sulfopropyl)-ammonium betaine, erucamidopropyl hydroxysultaine, ethylhexyl dipropionate, ethyl hydroxymethyl oleyl oxazoline, ethyl PEG-15 cocamine sulfate, hydrogenated lecithin, hydrolyzed protein, isostearamidopropyl betaine, 3-[(Lauramidoethyl)dimethylammonio]-2-hydroxypropane-sulfonate, 3-[(Lauramidoethyl)dimethylammonio]propanesulfonate, lauramido-propyl betaine, lauramidopropyl dimethyl betaine, lauraminopropionic acid, lauroamphodipropionic acid, lauroyl lysine, lauryl betaine, lauryl hydroxysultaine, lauryl sultaine, linoleamidopropyl betaine, lysolecithin, milk lipid amidopropyl betaine, myristamidopropyl betaine, octyl dipropionate, octyliminodipropionate, n-octyl-N,N-dimethyl-3-ammonio-1-propanesulfonate, n-decyl-N,N-dimethyl-3-ammonio-1-propanesulfonate, n-dodecyl-N,N-dimethyl-3-ammonio-1-propanesulfonate, n-tetradecyl-N,N-dimethyl-3-ammonio-1-propanesulfonate, n-hexadecyl-N,N-dimethyl-3-ammonio-1-propanesulfonate, n-octadecyl-N,N-dimethyl-3-ammonio-1-propane-sulfonate, oleamidopropyl betaine, oleyl betaine, 4,4(5H)-oxazoledimethanol, 2-(heptadecenyl) betaine, palmitamidopropyl betaine, palmitamine oxide, PMAL-C6 substituted with 3-(dimethylamino) propylamine, PMAL-C12 substituted with 3-(dimethylamino) propylamine, PMAL-C16 substituted with 3-(dimethylamino) propylamine, ricinoleamidopropyl betaine, ricinoleamidopropyl betaine/IPDI copolymer, sesamidopropyl betaine, sodium C12-15 alkoxypropyl iminodipropionate, sodium caproamphoacetate, sodium capryloamphoacetate, sodium capryloamphohydroxypropyl sulfonate, sodium capryloamphopropionate, sodium carboxymethyl tallow polypropylamine, sodium cocaminopropionate, sodium cocoamphoacetate, sodium cocoamphohydroxypropyl sulfonate, sodium cocoamphopropionate, sodium dicarboxyethyl cocophosphoethyl imidazoline, sodium hydrogenated tallow dimethyl glycinate, sodium isostearoamphopropionate, sodium lauriminodipropionate, sodium lauroamphoacetate, sodium oleoamphohydroxypropylsulfonate, sodium oleoamphopropionate, sodium stearoamphoacetate, sodium tallamphopropionate, soyamidopropyl betaine, stearyl betaine, 3-[(Stearamidoethyl)dimethylammonio]-2-hydroxypropanesulfonate, 3-[(Stearamidoethyl)-dimethylammoniolpropanesulfonate, tallowamidopropyl hydroxysultaine, tallowamphopoly-carboxypropionic acid, trisodium lauroampho PG-acetate phosphate chloride, undecylenamidopropyl betaine, and wheat germamidopropyl betaine. In some aspects, the zwitterionic surfactant is PMAL-C16 substituted with 3-(dimethylamino) propylamine. The terms "PMAL," "PMAL C-16," and "PMAL-C16 substituted with 3-(dimethylamino) propylamine" are used interchangeably herein. Any one or more of the foregoing zwitterionic surfactants may be explicitly excluded from the compositions of the disclosure, in some aspects.

In some aspects, the zwitterionic molecule or compound may be an amino acid and/or an amino acid derivative, such as any natural or artificial/synthetic amino acid and/or amino acid derivative, including but not limited to Alanine, Arginine, Asparagine, Aspartic Acid, Glutamic Acid, Cysteine, Glutamine, Glycine, Histidine, Isoleucine, Leucine, Lysine, Methionine, Phenylalanine, Proline, Selenocysteine, Serine, Threonine, Tryptophan, Tyrosine, Valine, Citrulline, Ornithine, Theanine, Betaine, Carnitine, Taurine, Tyramine, and/or Gamma Aminobutyric Acid, and/or any derivative thereof without limitation. In specific aspects, the amino acid is arginine. In some aspects, the zwitterionic molecule or compound may be ethylenediaminetetraacetic acid (EDTA). Any one or more of the foregoing zwitterionic molecules or compounds may be explicitly excluded from the compositions of the disclosure, in some aspects.

In some aspects, a composition of the disclosure may include a plasticizer, such as any natural or artificial/synthetic plasticizer and/or plasticizer derivative, including but not limited to monosaccharides (glucose), disaccharides (sucrose), oligosaccharides (e.g., pullulan), polyols (e.g., sorbitol, glycerol, mannitol, glycerol derivatives, polyethylene glycols), lipids, or combinations thereof. In specific aspects, the plasticizer is glycerol. Any one or more of the foregoing plasticizers may be explicitly excluded from the compositions of the disclosure, in some aspects.

In some aspects, a composition of the disclosure may include a pegylated lipid, including but not limited to PEG-modified phosphatidylethanolamine, PEG-modified phosphatidic acid, PEG-modified ceramides (e.g., PEG-CerC14 or PEG-CerC20), PEG-modified dialkylamines, PEG-modified diacylglycerols, PEG-modified dialkylglycerols, 2-[(polyethylene glycol)-2000]-N,N-ditetradecylacetamide, glycol-lipids including PEG-c-DOMG, PEG-c-DMA, PEG-s-DMG, N-[(methoxy polyethylene glycol)2000)carbamoyl]-1,2-dimyristyloxlpropyl-3-amine (PEG-c-DMA), and PEG-2000-DMG, PEGylated diacylglycerol (PEG-DAG) such as 1-(monomethoxy-polyethyleneglycol)-2,3-dimyristoylglycerol (PEG-DMG), a PEGylated phosphatidylethanolamine (PEG-PE), a PEG succinate diacylglycerol (PEG-S-DAG) such as 4-O-(2',3'- di(tetradecanoyloxy)propyl-1-O-((o-methoxy(polyethoxy)ethyl)butanedioate (PEG-S-DMG), a PEGylated ceramide (PEG-cer), or a PEG dialkoxypropylcarbamate such as co-methoxy(polyethoxy)ethyl-N-(2,3di(tetradecanoxy)propyl)carbamate or 2,3-di(tetradecanoxy)propyl-N-(u>-methoxy(polyethoxy)ethyl)carbamate. Any one or more of the foregoing pegylated lipids may be explicitly excluded from the compositions of the disclosure, in some aspects.

In some aspects, a composition of the disclosure may include a neutral lipid. In some aspects, the neutral lipid can be pegylated. Neutral lipids include, but are not limited to, 1,2-distearoyl-sn-glycero-3-phosphocholine (DSPC), distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyl-oleoyl-phosphatidylethanolamine (POPE), dioleoyl-phosphatidylethanolamine 4-(N-maleimidomethyl)-cyclohexane-1-carboxylate (DOPE-mal), dipalmitoylphosphatidylethanolamine (DPPE), dimyristoylphosphoethanolamine (DMPE), distearoylphosphatidylethanolamine (DSPE), 16-O-monomethyl PE, 16-O-dimethyl PE, 18-1-trans PE, 1-stearoyl-2-oleoylphosphatidyethanolamine (SOPE), and/or 1,2-dielaidoyl-sn-glycero-3-phosphoethanolamine (transDOPE). In certain aspects, the pegylated lipid of a composition of the disclosure is DMPE-PEG. Any one or more of the foregoing neutral lipids may be explicitly excluded from the compositions of the disclosure, in some aspects.

Furthermore, in some aspects, the compositions of the present disclosure may further include one or more adjuvants, or substances formulated as part of a composition to boost immune response and enhance efficacy of the composition. Exemplary adjuvants that may be included in compositions of the disclosure include but are not limited to amorphous aluminum hydroxyphosphate sulfate (AAHS), aluminum hydroxide, aluminum phosphate, potassium aluminum sulfate (Alum), Monophosphoryl lipid A (MPL) and *Quillaja saponaria* Molina: fraction 21 (QS-21) (AS01, AS02), Monophosphoryl lipid A (MPL) and aluminum salt (AS04), squalene/dl-α-tocopherol/polysorbate 80 (AS03), MPL/CpG/QS-21 (AS15), ADDAS03™, ADDAVAX™, Cytosine phosphoguanine (CpG), ISCOM, ISCOMATRIX, MATRIX-M™, MF59, bacterial enterotoxins (e.g., dmLT, LTB, CT, mmCT), bacterial flagellins (e.g., FliC, FljB, FliCd), bacterial-derived enterocyte cell-targeting proteins (e.g., mInlA, FnBPA), bacterial derived proteins (e.g., MT, PorA, c-di-AMP, RCK), protozoan-derived proteins (VSPs), intestinal immune cell-targeting peptides (Co1, CKS9, Gb-1), small molecular immunomodulatory proteins (e.g., cytokines [e.g., RANKL, IL-1β, IL-2, IL-12, cGM-CSF, thymosin α-1 [Tα1]), Fc region of immunoglobulin (Ig) G, biodegradable polymers (e.g., poly(d,l-lactide-co-glycolide) [PLG], poly( d,l-lactic-co-glycolic acid) [PLGA], chitosan and its derivatives, alpha-galactosylceramide [α-GalCer], ulex europaeus agglutinin-1 [UEA-1]), some synthetic toll-like receptor agonists and their derivatives (e.g., GS-986), and the like, or any combination thereof. Any one or more of the foregoing adjuvants may be explicitly excluded from the compositions of the disclosure, in some aspects.

Components of the compositions other than the agent (e.g., a therapeutic agent) and/or components thereof as described herein (e.g., polypeptides, small molecules, nucleic acids) are exemplified in, for example, PCT International Publication No. WO 2012/018628 and U.S. Patent Application Publication No. 2019/0298836, incorporated by reference herein.

In some aspects of the disclosure, the pH of the composition is adjusted so that the final pH of the composition is from about pH 6.0 to 9 (including any range or value derivable therein).

Adjustment to a desired pH may be accomplished with a suitable buffer such as phosphate buffered saline (PBS) and tris (Tris(hydroxymethyl)aminomethane) buffer. In some aspects, the buffer or combination thereof, is added so that the final pH is about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0 (including any range or value derivable therein. In some aspects, a composition of the present disclosure has a pH of at least, at most, or about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, or 8.5. In some aspects, the pH of a composition of the disclosure is at least 7.0. In some aspects, the pH is between 7.5 and 8.5. In certain aspects, the pH is about or exactly 8.0. In some aspects, the pH is between 8.0 and 9.0. In certain aspects, the pH is about or exactly 8.5.

As described herein, the disclosed formulations, in both liquid and solid form, promote significant preservation of the agent (e.g., a therapeutic agent) contained therein to thereby stabilize the therapeutic agent for extended periods of time. This results in the agent (e.g., a therapeutic agent) composition retaining significantly more activity than with other forms of storage. For example, stabilization for lengths of time of at least 3 years when stored at room temperature, are expected. These formulations, or forms of the formulation (e.g., liquid or solid form), promote significant preservation of the agent (e.g., a therapeutic agent) when stored for at least, at most, equal to, or between any two of 1 day, 7 days, 14 days, 30 days, 60 days, 90 days, 120 days, 150 days, 180 days, 210 days, 240 days, 270 days, 300 days, 330 days, or 360 days; at least, at most, equal to, or about 1 year; or at least, at most, equal to, or between any two of 1 month, 2 months, 3 months, 4 months, 5 months, 6 months, 7 months, 8 months, 9 months, 10 months, 11 months, 12 months, 18 months, 24 months, or 36 months. Storage at temperatures at or above 0 °C is envisioned (e.g., at least, about, or exactly 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 °C, including any range or value derivable therein).

A significant amount of preservation is considered to be that which results in a usable amount of agent (e.g., a therapeutic agent) for the intended purpose (e.g., gene therapy to deliver nucleic acid, or diagnostic purposes). It is believed that considerable value is obtained even with a composition described herein that results in 50% or less (e.g., about 45%, about 40%, about 35%, about 30%, about 25%, etc.) preservation of agent activity, in light of the ease of storage and shipment.

The amount of preserved agent (e.g., a therapeutic agent) can be determined by comparison of active agent in the stored form versus the original amount of active agent. Activity can be measured by various methods known in the art such as transduction efficiency, transfection efficiency, infectivity, vector genome copy number, and/or percent recovery of agent (e.g., a therapeutic agent) following storage. In some aspects, agent (e.g., a therapeutic agent) activity is preserved by at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, or 99%. In some aspects, transduction efficiency, transfection efficiency, infectivity, vector genome copy number, and/or percent recovery of agent (e.g., a therapeutic agent) following storage is preserved by at least 50%, at least 60%, at least 70%, at least 80%, at least 90% or, more, after exposure to conditions up to 95% relative humidity and up to 40 °C temperature. In some aspects, agent (e.g., atherapeutic agent) is preserved by at least 50%, at least 60%, at least 70%, at least 80%, at least 90% or, more, for 150 days or, more.

One method of determining the preservation is by comparing the activity (e.g., as measured by transduction efficiency, transfection efficiency, infectivity, vector genome copy number, percent recovery of agent (e.g., a therapeutic agent) following storage, etc.) of the agent prior to storage (e.g., prior to or just after adding to the rest of the composition components, or just after film formation) to the activity after storage.

Various conditions may be encountered during the storage, such as storage at about 4 °C, storage at about 0 °C, storage at about 25 °C, combinations of different temperatures, exposure to extreme temperatures, freeze thawing, or combinations thereof. In some aspects, storage is at ambient temperature. In some aspects, varying temperatures are encountered during storage, ranging from 4 °C to 40 °C or higher. Such variations may be encountered during shipping, and/or storage in areas that lack refrigeration. In some aspects, the stored formulations are subjected to ambient temperature (e.g., 20 °C +/- 10 °, +/- 5 °, +/- 4 °, +/- 3 °, +/- 2 °, +/- 1 ° C) and ambient pressure (e.g., approximately 1 atm, +/- 0.5 atm). The formulations and methods described herein are expected to provide significant preservation of agent (e.g., a therapeutic agent) under varying conditions, including different relative humidity (e.g., 5% RH to 99% RH, such as about 20%, about 30%, about 40%, about 50%, about 60%, about 70%, about 80%, about 90% RH, about 95% RH), and different pressures.

**II. Methods of Preparation and Use**

In some aspects, a composition including an amorphous solid may be made by preparing a solution including a sugar, sugar derivative or combination of sugars/derivatives in a buffer and optionally other additives previously mentioned. In some aspects, a sugar, sugar derivative or combination of sugars/derivatives may be present in the solution in an amount up to about 50%, about 60%, about 70%, or up to about 80% by weight of the solution. In some aspects, an additive may be present in an amount of about 5% or less, about 4% or less, about 3% or less, about 2% or less, or 1% or less by weight of the solution. In general, the solution including the sugar, sugar derivative or combination of sugars/derivatives is made at a concentration higher than the desired final concentration to compensate for any dilution that may occur when the agent (e.g., a therapeutic agent) (e.g., nucleic acid, polypeptide, small molecule) or other molecules is added. The desired agent (e.g., a therapeutic agent) (e.g., nucleic acid, polypeptide, small molecule) or other molecule may be added to the solution at a concentration known to induce the desired immune response.

In some aspects, a composition including an amorphous solid may be made by preparing a solution including a plasticizer and/or a surfactant and a polymer in a buffer and optionally other additives previously mentioned. In some aspects, a plasticizer and/or a surfactant and a polymer may be present in the solution in an amount of about 5% or less, about 4% or less, about 3% or less, about 2% or less, or 1% or less by weight of the solution. In some aspects, an additive may be present in an amount of about 5% or less, about 4% or less, about 3% or less, about 2% or less, or 1% or less by weight of the solution. In general, the solution including the plasticizer and/or a surfactant and a polymer is made at a concentration higher than the desired final concentration to compensate for any dilution that may occur when the agent (e.g., a therapeutic agent) (e.g., nucleic acid, polypeptide, small molecule) or other molecules is added. The desired agent (e.g., a therapeutic agent) (e.g., nucleic acid, polypeptide, small molecule) or other molecule may be added to the solution at a concentration known to induce the desired immune response.

In some aspects, the solution has a pH of at least, at most, or about 5, 6, 7, 8, 9, or 10, or any range or value derivable therein. In some aspects, the solution has a pH between 7 and 9, including any range or value derivable therein. In some aspects, the solution has a pH of about or exactly 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, or 9, or any value derivable therein. In some aspects, the solution has a pH of about 8. In some aspects, the solution has a pH of 8. In some aspects, the solution has a pH of about 8.5. In some aspects, the solution has a pH of 8.5.

The mixture may then be stirred at ambient temperature until a substantially homogeneous mixture is obtained. In some aspects, the mixture may then be briefly sonicated under cooled conditions, e.g., about 4 °C, to remove any air bubbles that may have developed. In other aspects, the mixture may be slightly heated, e.g., heated to about 40 °C or below, about 45 °C or below, or about 50 °C or below, slightly cooled, and in some instances may be frozen. In some aspects, a composition of the present disclosure (e.g., nucleic acid-containing composition) may be made without freeze drying or spray draying. The final formulation may then be cast onto a flat backing surface under controlled air flow, such as a controlled, laminar flow of air and allowed to form an amorphous solid at ambient temperatures (e.g., about 15-25 °C). Examples of suitable backing surfaces may include, but are not limited to, aluminum, polytetrafluoroethylene, silicate, polyether etherketone, polyethylene, polypropylene, polyvinylchloride, polyamide, polyacrylate, polyester, ethyl cellulose, and silicone, including any combination thereof. In some aspects, a glass plate can be used for casting of the composition, which can be dried under a controlled, laminar flow of air at room temperature, or under refrigerated conditions. In some aspects, a silicone backing surface can be used for casting of the composition, which can be dried under a controlled, laminar flow of air at room temperature, or under refrigerated conditions. Once the process is complete, the composition can be peeled from the backing and administered immediately (e.g., by placement in the mouth or by injection) and/or stored at ambient temperature for up to about 1 day, up to about 7 days, up to about 14 days, up to about one month (about 30 days), up to about two months (about 60 days), up to about three months (about 90 days), up to about four months (about 120 days), up to about five months (about 150 days), up to about six months (about 180 days), up to about one year (about 50 weeks), up to about two years (about 100 weeks), or even up to about three years (about 150 weeks) from manufacture.

In some aspects, the composition of the present disclosure may be made by contacting an amorphous solid with an agent (e.g., a therapeutic agent) (e.g., a nucleic acid, polypeptide, or small molecule), or optionally, mixing an agent with one or more excipients (surfactants, plasticizers, sugars, starches, etc.) and contacting the amorphous solid with the mixture so as to disperse the agent within the amorphous solid. In some aspects, the mixture is then allowed to dry and is then ready for administration. A composition of the disclosure can be used in a variety of ways (e.g., as a therapeutic delivery vehicle such as for gene therapy, as a vaccine capable of eliciting an immune response from the immune system of a subject receiving the composition, for storage of one or more biological components such as nucleic acids or peptides, etc.).

Compositions suitable for use according to the present disclosure can be prepared in a single-layer or multi-layers. In some aspects, compositions of the present disclosure may further include a protective layer disposed on a surface of an amorphous solid including an agent (e.g., a therapeutic agent) (e.g., a nucleic acid, polypeptide, or small molecule). Exemplary protective layers may include, but are not limited to, an additional layer(s) of film, such as polyethylene, polyurethane, polyether etherketone, etc., and/or an additional layer(s) of an amorphous solid that does not contain any agent (e.g., a therapeutic agent). In some aspects, the use of a protective layer of film may minimize the absorption of moisture from the atmosphere and prevent adherence to other objects during storage and/or transport. Prior to administration, this layer may be removed from the amorphous solid including an agent (e.g., a therapeutic agent) (e.g., by peeling the layer off) and may be discarded.

The amount of agent (e.g., a therapeutic agent) (e.g., a nucleic acid, polypeptide, or small molecule) that may be used in a composition of the present disclosure may vary greatly depending upon the type of agent (e.g., a nucleic acid, polypeptide, or small molecule) being used, the formulation being used to prepare the composition, the size of the amorphous solid, solubility, etc. One of ordinary skill in the art with the benefit of this disclosure will be able to determine a suitable amount of agent (e.g., a therapeutic agent) (e.g., a nucleic acid, polypeptide, or small molecule) to include in a composition of the present disclosure. In aspects of the disclosure, compositions may include, for example, about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2.0, 3.0, 4.0, 5.0, 6.0, 7.0, 8.0, 9.0, and 10 mg/mL of the agent (e.g., a therapeutic agent). In some aspects, the agent (e.g., a therapeutic agent) is a nucleic acid, and the composition comprises between 0.001 mg/mL and 1 mg/mL of the nucleic acid, for example.

It is also important to note that when formulating a composition according to the present disclosure, one must also consider any toxicity and/or adverse effects. Furthermore, in an effort to create a stable composition, it may also be important to identify a ratio of ingredients that interacts with water and the molecule (e.g., nucleic acid, polypeptide, or small molecule) in a manner that prevents crystallization during drying.

In general, the compositions of the present disclosure may be formulated so as to dissolve from about 5 to 60 seconds up to a period of 2 hours. When administered, a composition of the present disclosure may be handled by a portion of the composition that does not contain the agent (e.g., a therapeutic agent) (e.g., a nucleic acid, polypeptide, or small molecule), and may be placed in the upper pouch of the cheek for buccal delivery, or far under the tongue for sublingual delivery, or reconstituted and utilized, for example as a solution for inhalation or as a nasal spray.

Reconstitution of the compositions of the disclosure, such as a thin film matrix, may be accomplished, for example, by solubilizing in, for example, buffered saline; PBS; salt solutions; formulations that require specific sugar or lipid based excipients; traditional solutions used in IV reconstitution of medicinal agents, body fluids; or any other fluid matrix that allows the compositions of the disclosure to reconstitute the agent (e.g., a therapeutic agent) (e.g., a nucleic acid, polypeptide, or small molecule) preserved in such a matrix. In some aspects, the composition is dissolved in an appropriately buffered aqueous solution to generate the composition in liquid reconstituted form. The appropriately buffered aqueous solution can comprise for example, buffered saline; PBS; salt solutions; formulations that require specific sugar or lipid based excipients; traditional solutions used in IV reconstitution of medicinal agents; body fluids; polyethylene glycol (PEG) (e.g., PEG 1500); a poloxamer (e.g., PLURONIC® F68 or F-127); or any other fluid matrix that allows the compositions of the disclosure to reconstitute the agent (e.g., a therapeutic agent) (e.g., a nucleic acid, polypeptide, or small molecule) preserved in such a matrix. In specific aspects, reconstitution of the compositions of the disclosure is accomplished by solubilizing in a solution comprising buffered saline. The buffered saline may be at a concentration of between 0.1% and 10%, e.g., at least, at most, equal to, or between any two of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, or 10%, in the solution. In specific aspects, reconstitution of the compositions of the disclosure is accomplished by solubilizing in a solution comprising a poloxamer, such as a poloxamer disclosed herein. The poloxamer may be at a concentration of between 0.00001% and 0.1%, e.g., at least, at most, equal to, or between any two of 0.00001, 0.00002, 0.00003, 0.00004, 0.00005, 0.00006, 0.00007, 0.00008, 0.00009, 0.0001, 0.0002, 0.0003, 0.0004, 0.0005, 0.0006, 0.0007, 0.0008, 0.0009, 0.001, .002, 0.003, 0.004, 0.005, 0.006, 0.007, 0.008, 0.009, 0.01, 0.02, 0.03, 0.04, 0.05, 0.06, 0.07, 0.08, 0.09, and 0.1%, in the solution. In specific aspects, reconstitution of the compositions of the disclosure is accomplished by solubilizing in a solution comprising PEG, such as a PEG disclosed herein. The PEG may be at a concentration of between 0.001 and 1.0%, e.g., at least, at most, equal to, or between any two of 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%, in the solution.

In some aspects, methods are provided for producing compositions in substantially solid carriers. Such a method may include obtaining or formulating a solution including sufficient stabilizers (e.g., sugars and sugar derivatives, polymers) and permeability enhancers (e.g., surfactants, such as a zwitterionic surfactant of the disclosure, and/or plasticizers) in a solvent system (e.g., distilled deionized water, ethanol, methanol). In some cases, formulation is such that the total amount of solid components added to the solvent are within the concentration of 10%-90% w/w. This suspension can be prepared by stirring, homogenization, mixing, and/or blending these compounds with the solvent. In some cases, small portions of each component (e.g., about 1/10 the total amount) are added to the solvent and the solution mixed before adding additional portions of the same agent or a new agent.

In some aspects, once each stabilizer and permeability enhancer is added, the bulk solution is placed at 4 °C. for a period of time between 2-24 hours. In some aspects, the bulk solution is subjected additional homogenization, such as sonication (e.g., for a period of about 5-60 minutes) to remove trapped air bubbles in the preparation. After sonication is complete, the agent (e.g., a therapeutic agent) (e.g., a nucleic acid, polypeptide, or small molecule) is added to the preparation. In some cases, the amount of the agent (e.g., a therapeutic agent) (e.g., a nucleic acid, polypeptide, or small molecule) will range from of about 0.001%-30% of the total solid concentration.

In some aspects, the preparation is then slowly piped into molds of a shape suitable for the application. The molds can be constructed of a variety of materials including, but not limited to, stainless steel, glass, silicone, polystyrene, polypropylene and other pharmaceutical grade plastics. In specific aspects, the mold is constructed of silicone. In some aspects, the preparation can be placed in the molds by slowly pouring by hand or by pushing the preparation through a narrow opening on a collective container at a slow controlled rate (e.g., about 0.25 mL/min) to prevent early hardening and/or bubble formation in the final film product. In certain preferred aspects, films can be poured to a thickness of about 12.5 μm-5000 μm. In some aspects, molds for casting of films can be sterilized by autoclaving and placed in laminar air flow hoods prior to casting.

In further aspects, molds may also be lined with a peelable backing material suitable for protection of the film product. Suitable backings include, without limitation, aluminum, gelatin, polyesters, polyethylene, polyvinyl and poly lactic co-glycolide polymers, wax paper and/or any other pharmaceutically acceptable plastic polymer.

In some cases, cast films will remain at ambient temperature (e.g., about 15-25 °C), such as in a laminar flow hood for 2-24 hours, after which time a thin, peelable film will be formed. In some cases, this film may be opaque or translucent. In some cases, individual films are peeled from the casting/drying surface, wrapped in wax paper and stored at room temperature (e.g., between 15 °C and 30 °C, between 18 °C and 28 °C, between 24 °C and 26 °C, or about 25 °C) in sealable plastic bags under controlled humidity conditions. However, in certain aspects, films can be stored at lower temperatures, such as at 4 °C, under controlled humidity as well.

In some aspects, multilayer films can also be created at this time by applying a second coating of as solution containing the same agent (e.g., therapeutic agent) (e.g., a nucleic acid, polypeptide, or small molecule), as the first layer or another different agent (e.g., therapeutic agent) (e.g., a nucleic acid, polypeptide, or small molecule) to the thin film. Again, in some aspects, this will remain at ambient temperature (e.g., about 15-30 °C, about 20-25 °C, about 18-28 °C, or about 25 °C), such as in a laminar flow hood, for an additional 2-24 hours after which time a thin, peelable film will be formed. Again, the film may be opaque or translucent.

In some aspects, films will be dissolved in a solution prior to use. For example, water or warmed buffered saline (e.g., about 37°C, body temperature) may be used. In specific aspects, films are dissolved in a solution comprising buffered saline. In specific aspects, films are dissolved in a solution comprising a poloxamer, such as a poloxamer disclosed herein. In specific aspects, films are dissolved in a solution comprising PEG, such as a PEG disclosed herein. In some aspects, the resulting solution can be screened for activity/particle count to determine the effectiveness of the formulation to retain the potency of the preparation over time. Such a dissolved film may be administered, for example, *via* intravenous, intramuscular, oral (e.g., buccal or sublingual), or intranasal administration to a subject.

An agent (e.g., therapeutic agent) contained within the disclosed formulations can be further diluted as needed prior to administration (e.g., in a pharmaceutically acceptable carrier). The appropriate route of administration can be determined by the skilled practitioner.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, and like materials and combinations thereof, as would be known to one of ordinary skill in the art (*see,* e.g., Remington's Pharmaceutical Sciences, 18th Ed., Mack Printing Company, 1990, pp. 1289-1329, incorporated herein by reference). Except insofar as any conventional carrier is incompatible with the active ingredient, its use in the therapeutic or pharmaceutical compositions is contemplated. Any one or more of the foregoing carrier ingredients may be explicitly excluded from the compositions of the disclosure, in some aspects.

In some aspects, the herein described formulations are used to deliver an effective amount of an agent (e.g., therapeutic agent) (e.g., a nucleic acid, polypeptide, or small molecule) to a subject. As such, another aspect of the disclosure relates to a method of delivering an agent (e.g., therapeutic agent) (e.g., a nucleic acid, polypeptide, or small molecule) to a subject comprising administering to the subject an effective amount of the composition described herein. In some aspects, the composition is administered directly. In some aspects, the composition is manipulated minimally (e.g., films are rehydrated then administered). In some aspects, the composition is even further diluted (e.g., with a pharmaceutically acceptable carrier).

In some aspects, such an effective amount is a therapeutically effective amount. A "therapeutically effective" amount as used herein is an amount that is sufficient to provide some improvement or benefit to the subject. Alternatively stated, a "therapeutically effective" amount is an amount that will provide some alleviation, mitigation, or decrease in at least one clinical symptom in the subject. Those skilled in the art will appreciate that the therapeutic effects need not be complete or curative, as long as some benefit is provided to the subject. In certain aspects, the therapeutically effective amount is not curative.

The pharmaceutical composition of the disclosure may exploit different types of carriers depending on whether it is to be administered in solid, liquid or aerosol form, and whether it need be sterile for such routes of administration as injection. The pharmaceutical compositions can be administered intravenously, intradermally, intra-arterially, intra-graft, intraperitoneally, intralesionally, intracranially, intraspinally, intracistemally, intraarticularly, intraprostatically, intrapleurally, intratracheally, intranasally, intravitreally, intravaginally, intrarectally, topically, intratumorally, intramuscularly, intraperitoneally, subcutaneously, subconjunctivally, intravesicularly, mucosally, intrapericardially, intraumbilically, intraocularly, intranasally, orally, buccally, sublingually, topically, locally, inhalation (e.g., aerosol inhalation), injection, infusion, continuous infusion, localized perfusion bathing target cells directly (e.g., in an autogenous tissue graft), *via* a catheter, *via* lavage, in cremes, in lipid compositions (e.g., liposomes), or by any other method or any combination of the foregoing as would be known to one of ordinary skill in the art (*see,* e.g., Remington's Pharmaceutical Sciences, 18th Ed., Mack Printing Company, 1990). Administration into istema magna, ventricles in brain, intraparenchymal in brain, lumbar puncture, intrathecal, into ureter, into renal artery, subretinal, subpial, intracoronary (into the heart), direct injections into salivary gland, into sensory organ of the ears (e.g., to treat deafness), into the ganglia, intra-articular, into placenta/vein, and limb perfusions is also envisioned. Any one or more of the foregoing routes of administration may be explicitly excluded for the compositions of the disclosure, in some aspects.

### III. General Pharmaceutical Compositions

The phrases "pharmaceutically acceptable" or "pharmacologically acceptable" refer to molecular entities and compositions that do not produce an adverse, allergic, or other untoward reaction when administered to an animal or human. As used herein, "pharmaceutically acceptable carrier" includes any and all solvents, dispersion media, coatings, anti-bacterial and anti-fungal agents, isotonic and absorption delaying agents, and the like. The use of such media and agents for pharmaceutical active substances is well known in the art. Except insofar as any conventional media or agent is incompatible with the active ingredients, its use in immunogenic and therapeutic compositions is contemplated. Supplementary active ingredients, such as other anti-infective agents and vaccines, can also be incorporated into the compositions. Any one or more of the foregoing ingredients may be explicitly excluded from the compositions of the disclosure, in some aspects.

The active compounds can be formulated for oral administration, e.g., formulated for administration *via* buccal or sublingual routes, or for intranasal administration. The active compounds can be formulated for parenteral administration, e.g., formulated for injection *via* intravenous, intramuscular, subcutaneous, or intraperitoneal routes. Typically, such compositions can be prepared as either liquid solutions or suspensions; solid forms suitable for use to prepare solutions or suspensions upon the addition of a liquid prior to injection can also be prepared; and, the preparations can also be emulsified. Also contemplated are substantially solid film formulations, as disclosed herein.

The pharmaceutical forms suitable for injectable use include sterile aqueous solutions or dispersions; formulations including, for example, aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

A pharmaceutical composition can include a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various anti-bacterial and anti-fungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various other ingredients enumerated above, as required, followed by filtered sterilization or an equivalent procedure. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain methods of preparation are vacuum-drying and freeze-drying techniques, which yield a powder of the active ingredient, plus any additional desired ingredient from a previously sterile-filtered solution thereof.

Administration of the disclosed compositions will typically be *via* any common route. This includes, but is not limited to oral or intravenous administration. In some aspects, the disclosed compositions are particularly useful for intravenous administration, e.g., due to low viscosity (e.g., less than 4000, 3000, 2000, 1000, 500, or 250 cp, or less) Alternatively, administration may be by orthotopic, intradermal, subcutaneous, intramuscular, intraperitoneal, or intranasal administration. Such compositions would normally be administered as pharmaceutically acceptable compositions that include physiologically acceptable carriers, buffers or other excipients.

Upon formulation, solutions will be administered in a manner compatible with the dosage formulation and in such amount as is therapeutically or prophylactically effective. The formulations are easily administered in a variety of dosage forms, such as the type of injectable solutions described above.

IV. Examples

The following examples are included to demonstrate certain aspects of the disclosure. It should be appreciated by those of skill in the art that the techniques disclosed in the examples which follow represent techniques discovered by the inventor to function well in the practice of the disclosure, and thus can be considered to constitute certain modes for its practice. However, those of skill in the art should, in light of the present disclosure, appreciate that many changes can be made in the specific aspects which are disclosed and still obtain a like or similar result without departing from the spirit and scope of the disclosure.

**Example 1 - Assessment of Long-Term Plasmid DNA Stability in Previously Optimized Formulation**

Plasmid DNA was prepared in a film formulation previously optimized for use with a recombinant adenovirus to evaluate changes in plasmid DNA (pDNA) transfection efficiency upon film drying. As shown in **FIG. 1,** the same film matrix (MSI-TX-1) that was previously shown to increase recombinant adenovirus potency and support long term storage of recombinant adenovirus at room temperature failed to preserve transfection efficiency of pDNA. The MSI-TX-1 formulation includes 1.5% HPMC, 0.2% tragacanth gum, 2% sorbitol, 1% PMAL C16, 10 mM Tris, pH 8.1.

To assess the effects of the various MSI-TX-1 film formulation components on transfection efficiency of pDNA after storage in a film, a model plasmid (AAVlacZ) was mixed with the individual film components in the liquid state, and transfection efficiency was measured. Both the HPMC film base and PMAL-C16 surfactant were shown to significantly impede transfection efficiency **(FIG. 2A)**. Additionally, gel electrophoresis was utilized to separate different pDNA conformations on agarose gel with linear and nicked pDNA standard produced by digestion with Bsa I and Nb.BsrD I, and both HPMC film base and PMAL-C16 surfactant were shown to impede the mobility of the pDNA on agarose gels **(FIG. 2B** and **FIG. 2C)**.

pAAVlacZ was mixed with the liquid formulation in a 1:3 ratio. The resulting solution transfected only 3.7 $\pm$ 0.8% of the cell population, suggesting that this formulation significantly compromised transfection efficiency by more than 90% with respect to plasmid placed in Tris buffer (100%, p <0.001, **FIG. 25A)**. Further evaluation of individual formulation components revealed that the surfactant (SF) individually compromised transfection to the greatest extent (25.7 $\pm$ 0.6%, p <0.001). The strong interaction between the surfactant and the recombinant DNA at the concentration utilized in the previously published formulation initiated precipitation of the plasmid **(FIG. 25B)** and hindered its mobility when analyzed by gel electrophoresis **(FIG. 25C)**. The polymer base alone reduced transfection efficiency to 55.2 $\pm$ 1.7% **(FIG. 25A)**. Mixing it with the sugar significantly compromised transfection from 123.6 $\pm$ 13.6% (Sug) to 31.6 $\pm$ 3.2% (Polymer + Sug). Reducing the polymer concentration to 0.5% and removing the surfactant significantly improved plasmid performance to 88.6 $\pm$ 12.0% **(FIG. 25D)**. Transfection efficiency of this formulation dropped from 99.5 $\pm$ 2.1% to 83.1 $\pm$ 5.3% during the film forming process and fell further after storage at room temperature for 3 months (34.0 $\pm$ 4.6%) **(FIG. 25E)**. Without wishing to be bound by theory, it is believed that HPMC and PMAL-C16 interfere with pDNA stability because they bind the pDNA, thereby compromising its biological function.

In addition to reducing transfection efficiency, components of the HPMC film base was also shown to be incompatible with transfection reagents including calcium phosphate and polyethylenimine (PEI), resulting in reduced transfection efficiency in transfection methods using either reagent **(FIG. 3;** Method 1: calcium phosphate; Method 2: PEI).

**Example 2 - Optimization of Plasmid DNA Film Formulation**

In view of the negative effects of the MSI-TX-1 formulation on pDNA stability, studies were initiated to optimize film formulations for pDNA stability and corresponding transfection efficiency.

**A. Summary of Formulations**

Specific details of Example 2 formulations are summarized in **Table 1.**

**Table 1**

| | Label | Formulation Components |
|---|---|---|
| **FIG. 25** | Polymer | 1.5% K4M HPMC |
| | Sug | 2% Sorbitol |
| | SF | 1% PMAL |
| **FIG. 26** | aa1 | Lysine |
| | aa2 | Arginine |
| | aa3 | Histidine |
| | aa4 | Glutamic Acid |
| | aa5 | Aspartic Acid |
| | aa6 | Glutamine |
| | aa7 | Alanine |
| | aa8 | Cysteine |
| **FIG. 27** | CA | Choline |
| **FIG. 28A** | P1 | 2% Polyvinylpyrrolidone (PVP) in 10 mM Tris buffer pH 8 |
| | P2 | 2% Polyvinylalcohol (PVA) in 10 mM Tris buffer pH 8 |
| | P3 | 1% Pectin in 10 mM Tris buffer pH 8 |
| | P4 | 1% Sodium Alginate in 10 mM Tris buffer pH 8 |
| | P5 | 1% A15C HPMC in 10 mM Tris buffer pH 8 |
| **FIG. 28B** | Film P2 | 2% PVA |
| | Film P2 + aa2 | 2% PVA + 1% Arginine |
| | Film P2 + EDTA | 2% PVA + 1 mM EDTA |
| | Film P2 + CA | 2% PVA + 1% Choline |
| **FIG. 28C** | Film P2 + Sug1 | 2% PVA + 1% Melezitose |
| | Film P2 + Sug2 | 2% PVA + 1% Trehalose |
| | Film P2 + Sug3 | 2% PVA + 1% Raffinose |
| | Film P2 + Sug4 | 2% PVA + 1% Sucrose |
| | Film P2 + Sug5 | 2% PVA + 1% Glucose |
| | Film P2 + Sug6 | 2% PVA + 1% Mannitol |
| | Film P2 + Sug7 | 2% PVA + 1% Sorbitol |
| **FIG. 29** | Film F0 | 2% PVA + 0.1% Arginine |
| | Film F1 | 2% PVA + 1% Arginine |
| | Film F2 | 2% PVA + 1% Raffinose |
| | Film F3 | 2% PVA + 1% Arginine + 0.5% Sorbitol |
| | Film F4 | 2% PVA + 1% Arginine + 0.5% Sorbitol + 1% Trehalose |
| **FIG. 30** | P | 2% PVA |
| | PS | 2% PVA + 0.5% Sorbitol |
| | PAS | 2% PVA + 1% Arginine + 0.5% Sorbitol |
| | PAST | 2% PVA + 1% Arginine + 0.5% Sorbitol + 1%Trehalose |

**B. Film Base Selection**

As shown in **FIG. 4,** film base can be buffered (10 mM Tris, pH 8.5) to maintain pDNA stability at room temperature. The film base to be included in an optimized film formulation for pDNA was determined by assessing the impact of film bases including Polyvinyl Pyrrolidone (P1; PVP, 2% w/v), Polyvinyl Alcohol (P2; PVA, 2% w/v), Pectin (P3; 2% w/v), Sodium Alginate (P4; 2% w/v), and Low Viscosity HPMC (P5; A15C, 1% HPMC) on transfection efficiency and plasmid conformation as measured by agarose gel electrophoresis. pDNA transfection efficiency **(FIG. 5A)** and pDNA conformation **(FIG. 5B)** were maintained when the pDNA was mixed with PVP, PVA, or low viscosity HPMC in the liquid state and stored for 48 hours at room temperature. pDNA transfection efficiency **(FIG. 5C)** and pDNA conformation **(FIG. 5D)** were also shown to be maintained for pDNA in films stored for 7 days 25 °C and then rehydrated. PVA was selected for further study based upon maintenance of transfection efficiency after drying and peelability of films.

In a second experiment, the same five polymers, some at different concentrations (*see* **Table 1)** were selected to determine their impact on transfection efficiency. P1 did not significantly interfere with transfection efficiency with respect to unformulated plasmid (96.1 + 2.3% vs 100 +3.6%, **FIG. 28A).** P2 improved transfection to 121.6 + 1.6% (p = 0.006). P3 blocked transfection efficiency with no transgene expression detected while P4 and P5 reduced transfection to 2.2% and 18.1% respectively. Additional investigation revealed that this reduction in transfection efficiency may be in part due to the ability of different polymers to interact with agents used in transfection assays **(FIG. 36)** and their inability to protect plasmid DNA from degradation after storage in solution at 25 °C **(FIG. 28B).**

**C. Excipient Selection**

Surfactants are often included in film-based formulations to improve dispersion of a medicinal agent throughout the matrix and release of agent during dissolution. While it is known that molecules with charged groups can potentially shield exposed hydrogen and hydroxyl groups on DNA strands, use of a novel zwitterionic surfactant interfered with transfection efficiency and did not support DNA stability within a film matrix. Because the surfactant in the MSI-TX-1 film formulation was shown to negatively impact pDNA stability, however, alternatives to such a surfactant were investigated.

As shown in **FIG. 6** and **FIG. 26,** charged components, EDTA and/or amino acids (e.g., arginine), may replace surfactant in some film formulations. In **FIG. 26,** 8 amino acids, divided into 3 groups (basic, acidic, neutral) based on isoelectric point (pI), were evaluated for their thermostabilizing properties in a 10 mM Tris buffer (pH 8) at 50°C. The fastest degradation rate was observed in formulations containing amino acids with a pI < 5.5 which included all considered "acidic" and one "neutral" amino acid **(FIG. 26C)** with no detectable DNA found after 2 days at 50 °C **(FIG. 26A).** In contrast, basic and neutral amino acids preserved DNA in the supercoiled conformation in solution at 50 °C. Namely, 83.8% of the original supercoiled conformation was found in a preparation of 1% arginine (aa2) while less than 57% was found in other formulations **(FIG. 26A).** After 4 days, 69.7% of original supercoiled DNA remained in the 1% aa2 formulations while that in the lysine (aa1) and histidine (aa3) formulations were fully converted to the nicked and linear conformation **(FIG. 26B).**

EDTA improves the thermostability of recombinant DNA through its ability to bind metal ions in solution and halt degradation processes that require these ions as cofactors. Thus, the next series of studies were designed to compare the stability profile of the aa2 formulation to those containing EDTA at elevated temperature **(FIG. 27).** Concentrations for each excipient were selected for their inability to interfere with transfection **(FIG. 27A)** and qPCR **(FIG. 27B)** based assays. After 2 weeks at 40 °C, solutions containing the amino acid, aa2, retained more of the supercoiled conformation (68.1% at 1% and 55.8% at 0.1% aa2) than plasmid in Tris buffer alone (26.6%, lane 2, **FIG. 27C).** This translated into an increase in transfection efficiency from 79.5% (Tris buffer) to 96.0% (p = 0.003) and 91.7% (p = 0.0077) for the 0.1% and 1% aa2 formulations respectively **(FIG. 27D).** There was no significant difference in transfection efficiency between the arginine (aa2) and EDTA containing formulations.

pDNA was formulated with the EDTA and Tris buffer (pH 8.1) and stored in the liquid state for 4 weeks at 40 °C. Both 1 mM and 2 mM concentrations of EDTA (E1) maintained >80% pDNA transfection efficiency **(FIG. 6A).** pDNA was formulated with the arginine and Tris buffer (pH 10) and stored in the liquid state for 7 days at 50 °C. Arginine (E2) at a concentration of 1% w/v in the formulation maintained >30% pDNA transfection efficiency **(FIG. 6B).** Combination of 0.1% w/v arginine with 1 mM or 2 mM EDTA preserved >80% pDNA transfection efficiency after storage for 4 weeks at 40 °C **(FIG. 6C).** These results demonstrate that, in some aspects, EDTA and arginine can work in tandem to maintain transfection efficiency of pDNA after long-term storage at higher temperatures.

Studies were also conducted to assess the effects of EDTA and arginine on pDNA stability at storage for 8 weeks at 25 °C. As shown in **FIG. 7,** transfection efficiency of pDNA in 2% w/v PVA film base alone dropped to 47.2%, but incorporation of arginine at 1% w/v increased transfection efficiency to 79% at the same timepoint. EDTA reduced pDNA stability in the 2% w/v PVA film matrix, as transfection efficiency after 8 weeks at 25 °C fell to 11%.

Due to the success of the amino acid, choline (CA) was also added to formulations to determine if the stabilizing effect observed for recombinant DNA is common to all compounds with a positively charged amine group or unique to arginine (aa2). More supercoiled DNA was found in a formulation containing 0.1% choline (59.0%) than in 1mM EDTA (53.6%) and Tris buffer alone (26.6%) after 2 weeks at 40 °C **(FIG. 27C).** CA and aa2 were also combined with EDTA to evaluate possible synergistic effects between the compounds. After 2 weeks at 40 °C, each formulation studied significantly improved transfection efficiency ($\geq 89.2 \pm 1.2\%$, $p < 0.001$) with respect to that observed in buffer alone ($79.4 \pm 1.3\%$) except for those containing 1% choline regardless of the amount of EDTA present (1 mM EDTA + 1% CA, $75.3 \pm 0.5\%$, 2 mM EDTA + 1% CA, $78.3 \pm 1.0\%$, **FIG. 27E).** While the supercoiled (SC) conformation of plasmid DNA rapidly degraded in unformulated, buffered samples at 40 °C (27.2% total DNA), solutions containing 1% choline contained 82.3% (1 mM EDTA + 1% CA) and 87.4% (2 mM EDTA + 1% CA) of DNA in the SC conformation. This pattern was similar to that seen for the 1% aa2 preparations respectively (83.1% and 84% SC DNA, **FIG. 27F)** despite the fact that these formulations could maintain transfection efficiency significantly better at 40 °C than those prepared with 1% choline (p <0.001), suggesting that the 1% choline/EDTA combination may have interfered with the transfection assay **(FIG. 27E).** While the presence of EDTA offered a slight improvement in the presence of SC DNA during the course of the study (EDTA + aa2, 69.6% SC DNA, **FIG. 27F** vs aa2 alone, 68.1% SC DNA, **FIG. 27C),** there was no significant change in transfection efficiency between these two formulations (EDTA + aa2, 94.9%, **FIG. 27E** vs 96.0% aa2 alone, **FIG. 27D,** p > 0.05).

### D. Film Base & Excipient Combination

Films were then prepared with P2 and each of the agents found to support stability of plasmids in solution (EDTA at 1 mM, arginine (aa2) and choline (CA) at 1%), packaged and stored at 25 °C for 8 weeks **(FIG. 28C).** Transfection efficiency of plasmid stabilized in films containing CA fell to 10.8 +3.0% within 7 days and remained at this level for the remainder of the study. Agarose gel electrophoresis revealed that 38.8% of the DNA was in the supercoiled form after 3 weeks **(FIG. 28D).** Combining EDTA with the polymer improved transfection to $46.2 \pm 2.2\%$ at the 7 day timepoint. This declined further to 23.2% by 2 weeks and was not statistically significant from that seen in the choline formulation by the end of the study. This preparation contained 35.3% of the DNA in the supercoiled form. Of all the formulations tested, films prepared from the polymer alone and in combination with aa2 were the most stable at 25 °C. After 3 weeks at 25 °C, transfection efficiency was $74.8 \pm 2.4\%$ and $93.2 \pm 2.8\%$ for the P2 and aa2 formulations respectively. The most significant difference between these formulations was seen at the 8 week timepoint where transfection efficiency fell to $47.2 \pm 0.2\%$ (P2) and $78.5 \pm 0.9\%$ (aa2). At this timepoint, 70.6% of the plasmid in the aa2 matrix remained in the supercoiled form while 37.4% was supercoiled in the P2 matrix **(FIG. 28D).**

### E. Sugar Selection

Sugars are often included in solid formulations of biologic drugs for their ability to form an amorphous glass that protects biologic drugs from degradation. Though not shown to negatively impact pDNA stability, the impact of various sugars on the transfection efficiency of pDNA prepared in films was assessed **(FIG. 8, FIGs. 28E-28F).** Each film contained 2% w/v PVA film base and 1% w/v sugar. Sugars tested included melezitose (S 1), trehalose (S2), raffinose (S3), sucrose (S4), dextrose (S5), mannitol (S6), and sorbitol (S7) **(Table 1).**

In a first experiment **(FIG. 8),** prepared films were stored for 8 weeks at 25 °C. Raffinose was determined to be most effective, with 81.4% transfection efficiency seen at 8 weeks, while dextrose was least effective, with 39% transfection efficiency seen at the same timepoint.

In a second experiment, of all the formulations tested, those containing glucose (Sug 5) performed the worst with transfection efficiency falling to $56.3 \pm 3.2\%$ after 2 weeks and even further to $23.6 \pm 1.1\%$ at 4 weeks at 25 °C. Transgene expression could not be detected in cells treated with solutions created from rehydrated films containing glucose. Agarose gel electrophoresis revealed that 14.6% of the DNA present at 8 weeks was in the supercoiled form **(FIG. 28F).** Plasmid stabilized in films prepared with melezitose (Sug1) had a similar profile to plasmid in films containing P2 alone after 4 weeks ($48.4 \pm 1.4\%$ P2 vs $48.8 \pm 0.7\%$ Sug1) and 8 weeks ($35.2 \pm 0.9\%$ P2 vs $40.7 \pm 1.4\%$ Sug1) at 25 °C. Each formulation contained approximately 25% of the supercoiled form of the plasmid DNA. While the remaining formulations demonstrated that all of the other sugars improved plasmid stability with respect to films made with polymer alone, transfection efficiency of each was significantly lower (p<0.001) that that of plasmid stored in Tris buffer alone ($77.3 \pm 1.0\%$) throughout the course of the study. Exceptions to this were preparations made with trehalose (Sug2) and raffinose (Sug3) **(FIG. 28E)** with transfection efficiency of $76.3 \pm 2.6\%$ and $79.5 \pm 3.6\%$ respectively after 8 weeks at 25 °C. Approximately 25.2% (Sug2) and 63.1% (Sug3) of the plasmid DNA remained in the supercoiled form in these preparations compared to that of plasmid stored at 25 °C in Tris buffer (69.1%, **FIG. 28F).** Further refinement of the film matrix included combining several sugars with the amino acid arginine (aa2) which supported plasmid stability in previous studies **(FIGs. 26** and **27).** Of these combinations, preparations made with aa2 and Sug2 supported the highest transfection efficiency and demonstrated reduced amounts of nicked and linear DNA after two weeks at 25 °C and 40 °C **(FIG. 37).** Given the superior ability of Sug3 to stabilize plasmid in the absence of aa2, it and the Sug2 + aa2 formulation were selected for additional studies.

**F. Film Base, Excipient, & Sugar Combination**

Transfection efficiency of plasmid preserved in films containing aa2 remained at 89.8% when stored at 25 °C for 12 weeks (Formulation F1, **FIG. 29A).** Addition of a plasticizer (S) to the aa2 formulation allowed the films to be more easily removed from molds without compromising transfection efficiency (F3, 86.2 $\pm$ 1.7 %). Adding Sug2 with aa2 to the film matrix (F4) maintained a transfection efficiency of 83.4 $\pm$ 2.2% after 12 weeks at 25 °C which was superior to that of plasmid in liquid buffer (74.8 $\pm$ 1.3%, Liquid Control, **FIG. 29A).** Visualization of DNA from the F4 preparation revealed that it contained more supercoiled DNA (73.6%) than all formulations after 4 weeks at 25 °C (60.9-67.5%, **FIG. 29B).** This trend continued through 12 weeks **(FIG. 38A).** Transfection efficiency of plasmid in films prepared with Sug3 alone (Formulation F2) declined at a rate of 3.55% per week over the first 4 weeks with transfection efficiency at the 12 week timepoint being not statistically different than the liquid control preparation (p =0.99) even though it contained more supercoiled DNA (59.1% vs 41.3%, **FIG. 38).**

When stored at 4 °C, plasmids embedded in films containing 0.1% aa2 alone (Formulation F0) maintained 77.1 $\pm$ 1.1% transfection efficiency throughout the 17 week period **(FIG. 29C).** This preparation contained the lowest amount of supercoiled DNA after 2 weeks (67.2%) and 8 weeks (68.0%) respectively **(FIG. 29D).** Transfection efficiency of plasmid in formulation F2 dropped from 91.3 $\pm$ 2.7% to 81.3 $\pm$ 5.7% after 1 week and remained at this level throughout the remainder of the study. Transfection efficiency of plasmids prepared in the F1 and F4 formulations was initially as high as the those in the liquid control formulation (above 90%) but fell sharply to 77.2 $\pm$ 4.5% and 76.2 $\pm$ 3.2% after 8 weeks at 4 °C. Transfection efficiency of plasmid in the liquid control formulation experienced a similar drop from 98.8 $\pm$ 3.2% to 79.6 $\pm$ 0.3% between the 1- and 2-week timepoints. In each case, this corresponded to a slight increase in the amount of nicked DNA in the sample **(FIG. 29D).**

**G. pDNA Concentration**

**FIGs. 9A-9B** show that the concentration of pDNA in films can impact the transfection efficiency after drying of the film and during storage of the film at 25 °C over the course of 30 days. Films were prepared in a base formulation of 2% (w/v) PVA, 1% (w/v) Arginine, 1% (w/v) Trehalose and 0.5% (w/v) sorbitol in 10mm Tris pH 8.1. pDNA was included in the films at a concentration of 0.05 mg/mL, 0.1 mg/mL, 0.25 mg/mL, 0.5 mg/mL, or 1 mg/mL. **FIG. 9A** shows the transfection efficiency after drying. **FIG. 9B** shows the transfection efficiency 30 days after storage of the film at 25 °C. As shown, a concentration of 1 mg/mL pDNA significantly reduces transfection efficiency after drying of the film and during storage of the film at 25 °C over the course of 30 days.

**H. Relative Humidity**

Environmental humidity can impact long-term stability of recombinant viruses within the film matrix. As shown in **FIGs. 10A-10B,** pDNA is most stable in films at low relative humidity (RH) regardless of temperature. pDNA (100 $\mu$g/ml) was prepared in a base formulation of 2% (w/v) PVA, 1% (w/v) Arginine, 1% (w/v) Trehalose, and 0.5% (w/v) sorbitol in 10mM Tris pH 8.1. Films were stored for 12 weeks at 25 °C and 30%, 60%, or 90% RH **(FIG. 10A)** or for 10 days at 40 °C and 20% or 75% RH **(FIG. 10B).** As shown, higher RH during storage of the pDNA-containing films can significantly decrease pDNA stability as measured by reduced transfection efficiency.

In a second experiment, the F4 formulation was stored at 25 °C under different humidity conditions. After 4 weeks at 25 °C, there was no significant difference between each storage condition (p = 0.43-0.99, **FIG. 30A).** However, transfection efficiency of plasmids stored at 90% RH was significantly reduced to 60.4 $\pm$ 0.7% while that of plasmids stored at 30 and 60% RH were 84.3 $\pm$ 1.0 % and 83.9 $\pm$ 1.6 % respectively (p<0.001, **FIG. 30A).** Residual moisture within films remained constant at 14.8-16.8% for 12 weeks regardless of the environment in which they were stored **(FIG. 30B).**

**I. Film Mold Selection**

As shown in **FIG. 11,** the composition of mold material on which the pDNA-containing films are casted can significantly impact transfection efficiency of pDNA during the film forming process. Plastic molds were made of polystyrene. Foil molds were AMCOR foil OPA25/AL45/PE60 cold pressed wells.

**J. Optimized pDNA Film Formulations**

Based on the foregoing studies, four optimized film formulations F1-F4 were generated and their impact on transfection efficiency assessed. In each formulation, film base was PVA, 2% w/v. Different excipients were added to the 2% w/v PVA base to obtain the following formulations F1-F4:

F1 (Arg): 2% w/v PVA, 1% w/v arginine, 10 mM Tris (pH 8.5)
F2 (Raf): 2% w/v PVA, 1% w/v raffinose, 10 mM Tris (pH 8.5)
F3 (Arg-S): 2% w/v PVA, 1% w/v arginine, 1% w/v sorbitol, 10 mM Tris (pH 8.5)
F4 (Arg-T): 2% w/v PVA, 1% w/v arginine, 1% w/v trehalose, 0.5% (w/v) sorbitol, 10 mM Tris (pH 8.5)

As shown in **FIG. 12A,** optimized formulations F1, F3, and F4 maintained significantly higher pDNA transfection efficiency after storage for up to 12 weeks at 25 °C and 60% relative humidity (RH) compared to pDNA prepared in a buffer-only control (Buffer (Liq)). Surprisingly, >80%, and in many cases, >90% transfection efficiency was observed for pDNA prepared in these formulations after storage for 12 weeks. All optimized formulations maintained pDNA conformation to a similar degree over 12 weeks **(FIG. 12B).**

Film matrices may be further optimized to control the rate of release of pDNA from the film matrix. **FIGs. 13A-13B** show the release profile of plasmid DNA (100 $\mu$g/ml) from film comprising 0.75% (w/v) HPMC K4M **(FIG. 13A)** versus the release profile of plasmid DNA (100 $\mu$g/ml) from film comprising 2% (w/v) PVA, 1% (w/v) Arginine, 1% (w/v) Trehalose in Tris buffer pH 8.1 **(FIG. 13A).**

Storage of pDNA in a film matrix, such as the optimized matrices described above, can better maintain pDNA stability over time compared to storage of pDNA that is buffered and dried. pDNA transfection efficiency was compared after storage for up to 8 days at room temperature for pDNA embedded in an optimized film matrix comprising 2% (w/v) PVA, 1% (w/v) arginine, 1% (w/v) trehalose in Tris buffer (pH 8.1) versus buffered and dried pDNA **(FIG. 14).** Plasmid in 10 mM Tris buffer pH 8.1 was dried under ambient conditions (Buffer (Dry)). Transfection efficiency was compared to plasmid (100 $\mu$g/ml) embedded in the optimized film matrix Replicate samples (n=3) were collected over time and transfection efficiency compared to that of samples at the start of the study. Transfection efficiency of dried plasmid experienced a 15% reduction in transfection efficiency due to the drying process itself (t=0). As shown in **FIG. 14,** however, the optimized film matrices provided superior stability of pDNA in the solid state for up to 8 weeks at room temperature compared to buffered pDNA alone.

### K. Long Term Stability of Plasmid DNA in Optimized Formulation

Prior to evaluating the long-term stability of recombinant DNA under optimized environmental conditions, one final optimization study was performed. This study revealed that the manner in which different components of the film were mixed prior to drying could significantly impact the performance of the plasmid in the rehydrated solution **(FIG. 30C).** Mixing plasmid with sugar or plasticizer alone prior to adding it to the bulk formulation did not significantly impact transfection efficiency once drying was complete (p = 0.75-0.99), however, mixing plasmid with the amino acid alone prior to bulk addition did reduce transduction efficiency by 23.3 % after drying was complete. This practice was avoided when preparing films for an extended stability study. Throughout the 9 month period in which films were stored at 25 °C and 60% RH, transfection efficiency remained high in the range of 83.0 - 111.5% with respect to plasmid stock stored frozen **(FIG. 31A).** Plasmid copy number of plasmid in the film matrix was stable for the first 3 months of the study with a significant increase found at 7 (117.6 $\pm$ 8.5%, p = 0.006) and 9 months (131.4 $\pm$ 7.3%, p=0.01) with respect to the copy number in freshly dried films (t=0, **FIG. 31B).** This change was also observed visually as 35% and 44% of the original supercoiled DNA conformation was converted to the nicked form at these timepoints **(FIG. 31C)** and the overall supercoiled:nicked ratio decreased over time **(FIG. 31D).**

### L. Physical and Biological Properties of Optimized Films

Films prepared with the optimized formulation (PAST; 2% PVA + 1% Arginine + 0.5% Sorbitol + 1% Trehalose) released 77.7 $\pm$ 3.3% of the plasmid payload within 2.5 minutes **(FIG. 32A).** Linear regression of data collected in the first 2 min of the dissolution curve revealed that plasmid was released at a rate of 21.3 $\pm$ 4.4% of the original concentration or 2.7 $\pm$ 0.5 $\times$ $10^{10}$ copies/ml per minute. Individual components of the PAST formulation, the complete liquid formulation (PAST Liquid) or a solution prepared from rehydrated film (PAST Film) were not cytotoxic **(FIG. 39).** Stepwise addition of excipients revealed that films prepared with plasticizer (S) alone were significantly stronger, thicker, heavier and more elastic than films containing polymer (P) alone **(FIG. 32B, Table 2).** Addition of the amino acid (A) with the plasticizer (S) to the formulation created a significantly more pliable film. Addition of the amino acid and sugar to the formulation containing plasticizer increased thickness from 53.8 $\pm$ 1.9 $\mu$m to 59.4 $\pm$ 5.1 $\mu$m **(Table 2).**

**Table 2**

| Formulation | Film weight (mg) | Film thickness (μm) |
|---|---|---|
| P | 27.8 ± 0.1 | 55.7 ± 8.0 |
| PS | 30.8 ± 0.1 | 53.8 ± 1.9 |
| PAS | 43.1 ± 0.3 | 57.3 ± 4.6 |
| PAST | 53.5 ± 0.1 | 59.4 ± 5.1 |

X-ray diffraction revealed that films prepared with individual components were amorphous solids **(FIG. 32C)**. Single glass transition events in the DSC analysis of the PAST formulation confirm the amorphous state of film matrix **(FIG. 40).** FTIR analysis showed that formulations containing the amino acid (PAS, PAST) contained peaks at 1664 and 1560 cm$^{-1}$ which correspond to C=O or C=N residues found on the amino acid (boxed region, **FIG. 32D).** Addition of plasmid to the PAST formulation did not create any additional peaks.

**M. Summary**

Plasmid stabilized within the optimized film matrix (PAST formulation; % PVA + 1% Arginine + 0.5% Sorbitol + 1% Trehalose) was significantly more stable than that formulated as a dry powder in the absence of any excipients **(FIG. 33A).** It is generally thought that the stability profile of plasmid DNA in the solid state is due to the restricted mobility of nucleotides in the absence of water. However, degradation of recombinant DNA can occur by enzymatic processes which can be mediated by residual materials from the manufacturing process and by non-enzymatic processes which are influenced by the macro- and microenvironments in which it is stabilized and stored. Non-enzymatic degradation of purified plasmids for clinical use occurs through free radical oxidation, hydroxylation, depurination and beta-elimination which are heavily influenced by temperature, pH and ionic strength **(FIG. 33B).** In the solid state, oxidation and phosphodiester hydrolysis are the most prominent mechanisms for DNA degradation. Phosphodiester bonds along sugar-phosphate backbone are susceptible to disruption by hydroxyl radicals/ions that convert deoxyribose molecules to carbon-based sugar radicals, breaking the nucleic acid strand. Hydrolysis and depurination mediated degradation can occur under acidic conditions and in the presence of water resulting in protonation of purine residues, cleavage of N-glycosidic bonds and release of nucleotides from DNA stands. Free protons generated from these processes also disrupt bonds along the phosphate backbone and between nucleotides of the DNA molecule. Under alkaline conditions, excess hydroxyl ions disrupt hydrogen bonds between two DNA strands. While this generally occurs at a much slower rate than degradation in acidic and elevated temperatures, it is accelerated if the pH increases to ≥ 11.6 resulting in strand separation and unwinding of the twisted helix.

Plasmid DNA commonly exists in 3 distinct physical conformations with unique structures and biological functions. Plasmids naturally assume a supercoiled (SC) state to relieve stress from the helical arrangements of nucleotides. When a strand breaks in response to a stressor, the supercoiled plasmid relaxes and assumes a nicked or open circular state. Disruption of both strands converts the plasmid to the linear form. While plasmids in the SC conformation are reported to have superior transfection efficiency over those in the nicked and linear state, results can vary with respect to cell line, tissue and delivery method. As disclosed herein, the presence of different plasmid conformations can significantly impact key stability indicating assays such as transfection efficiency and plasmid copy number as determined by quantitative PCR **(FIG. 35)** and not be detected in others **(FIG. 41).** Considering that nicked and linear forms are removed during clinical manufacturing due to the risk of recombination and integration into genomic DNA and that the US FDA Guidance for Industry recommends SC content be more than 80% of the bulk material, the presence of the SC conformation was monitored in the formulations disclosed herein by agarose gel electrophoresis and paired with transfection efficiency. An optimal formulation was defined as one that could support ≥ 80% of the SC conformation when stored at ambient temperature over time.

The primary component of all film-based dosage forms is a polymer base capable of forming an amorphous solid during the drying process. As disclosed herein, the polymer that stabilized recombinant adenovirus and AAV at ambient temperature significantly impaired transfection efficiency **(FIG. 25A).** Data from screening other polymers suggests that it and polymers of similar composition may not fully release plasmid during the dissolution process **(FIG. 28A).** A zwitterionic surfactant, known to stabilize viruses within the film matrix, condensed plasmid DNA so tightly that it could not transfect cells and migrate out of wells during agarose gel electrophoresis **(FIGs. 25A-25C).** EDTA, a known stabilizer of recombinant DNA in solution and a molecule also capable of holding a dual charge did maintain plasmid stability in solution at 40 °C **(FIG. 27C),** but failed to preserve the SC form within the film matrix for 8 weeks **(FIG. 28C).** During this time, pH within the film matrix shifted from 6.5 to 5.5 suggesting that acid-mediated hydrolysis could be responsible for this effect. The ionization state of EDTA within the film may be different from that observed in solution which may disrupt interactions between the polymer, the plasmid and Tris buffer, facilitating degradation of the plasmid and/or interfering with the ability of the buffer to maintain pH.

While sugars are often included in oral film formulations for taste-masking properties, they are also utilized to stabilize biologic drugs due to their unique ability to replace water and maintain structural conformation in an amorphous, glassy state. All sugars tested formed amorphous solids **(FIG. 32C, FIG. 40),** however, reducing sugars had the poorest stability profiles and most likely supported acid hydrolysis of plasmid as indicated by a significant drop in pH within the film matrix **(FIG. 40B).** In contrast, films prepared with trehalose did not experience a drop in pH during storage at 25 °C and maintained transfection efficiency when stored under extreme conditions **(FIG. 37B).** Although another sugar in the panel was also able to maintain pH within the film during storage, it significantly changed the overall Tg of the preparation, suggesting that interactions between it and the polymer base were not supportive of plasmid stability **(FIG. 28C, FIG. 40B).**

As shown herein, amino acids stabilized plasmids in solution and within a film matrix. Various amino acids have been included in pharmaceutical formulations to increase solubility, decrease viscosity and prevent aggregation during drying and rehydration, however, there is a limited understanding of how they contribute to the stabilization of nucleic acids. The amino acid in the formulations herein provided a stability profile superior to EDTA, a compound which has been shown to stabilize DNA in solution and the frozen state. According to solution thermodynamics, plasmids are highly charged molecules that expend a significant amount of energy to remain distant from each other. Certain concentrations of positively charged counterions in a liquid formulation reduces electrostatic repulsion between plasmids and facilitates folding of nucleic acids while other concentrations may support relaxation of plasmid and conformational transition. This effect was observed in liquid formulations utilized in the pre-screening process **(FIGs. 27C-27D).** Combining ETDA with the amino acid did not impact the stability offered by the amino acid alone, however, data generated from those studies did suggest that the amino acid could prevent the significant change in pH caused by ETDA alone as it was uniquely capable of maintaining an alkaline pH within the film matrix **(FIG. 26C** and **FIG. 40D).** Taken together, this suggests that the manner in which this amino acid stabilizes plasmid DNA within the film matrix is twofold. During the drying process, the positively charged compound aligns along electron dense regions of the plasmid to preserve the supercoiled conformation during the drying process **(FIG. 33B).** In the solid state, this interaction prevents degradation by hydrolysis and depurination. Amino acid molecules not involved in the protective shell can retain excess hydroxyl ions to prevent disruption of phosphodiester bonds formed between the double DNA strands and within the plasmid backbone.

To date, most plasmid-based products are formulated and stored as frozen or refrigerated liquid solutions. Recent strategies to deliver plasmids to the lungs or through the skin fostered the use of advanced drying technologies to preserve high quality plasmids in solid formulations. These include freeze drying, spray freeze drying and dry cooling with supercritical carbon dioxide. In one report, it was shown that a dry powder containing naked plasmid lyophilized in a formulation containing hyaluronic acid had a significantly higher long-term stability profile at 25 °C with improved gene expression in the lung with respect to the same plasmid lyophilized in mannitol or in a solution complexed with the transfection reagent, PEI. Other reports demonstrate that complex formation with biopolymers can prevent degradation of plasmid DNA during the drying process. For example, spray dried powders containing plasmid-chitosan nanoparticles retain a higher transfection efficiency with minimal aggregation compared to the same particles stored as a liquid suspension after 3 months at 4 °C. Powders created by supercritical $CO_2$ drying exhibit high levels of transgene expression 24 hours after intratracheal administration in mice, however, the acidic environment created by water and $CO_2$ during the drying process does not support long-term plasmid stability.

There are a limited number of reports in the literature in which naked plasmid DNA has been stabilized in a single layer film matrix as described herein. Most reports include a complexing agent such as PEI or liposome with the plasmid before adding it to the surface of a preformed film to create a multi-layer substrate. These multi-layer films have been incorporated into implantable stents for therapeutic applications. In one report, bilayer films containing a mucoadhesive polymer and naked plasmid encoding beta-galactosidase placed over an impermeable backing layer were evaluated for their ability to induce an immune response after buccal administration in rabbits. After releasing 60-80% of the plasmid within 2 hours, a systemic immune response similar to that achieved by subcutaneous injection was found in animals given the films.

Disclosed herein is a formulation that preserves transfection efficiency of naked plasmid DNA within a single layer film matrix for up to 9 months. It is important to note that the plasmid utilized herein was purified from bacterial stocks using laboratory grade kits and that 20% of the starting material was in the nicked and linear conformation. Thus, the stability profile may have been improved further with a highly purified stock that did not contain compromised plasmid. The present disclosure is the first in which the stability of naked plasmid DNA at ambient temperature within a single layer film matrix was characterized by several stability indicating assays. The impact of different conformations of plasmid DNA on data generated by was assessed, and inclusion of compounds capable of maintaining a positive charge while supporting an alkaline environment within the film matrix were identified.

**N. Exemplary Materials & Methods (Examples 1 and 2)**

**Materials.** Dulbecco's phosphate buffer solution (PBS), Trizma base [2-amino-2-(hydroxymethyl)-1,3-propanediol] (Tris), mannitol, melezitose, trehalose, sucrose, raffinose, poly(vinyl alcohol) MW 9,000-10,000, 80% hydrolyzed, ortho-nitrophenyl-β-galactopyranoside (ONPG) and β-mercaptoethanol were purchased from Sigma-Aldrich (St. Louis, MO). Poly (maleic anhydride-alt-1-octadecene) substituted with 3-(dimethylamino)propylamine (PMAL) was obtained from Anatrace (Maumee, OH). Polyethyleneimine (PEI, Linear, MW 25000, Transfection Grade) was purchased from Polysciences (Warrington, PA). Glycerol and Aqualine Complete 5 were purchased from Fisher Chemical (Fairlawn, NJ). Dulbecco's modified eagle media (DMEM), penicillin (10,000 IU) and streptomycin (10,000 $\mu$g/ml) and 0.25% Trypsin EDTA were bought from Mediatech (Manassas, VA). Fetal bovine serum was purchased from Mediatech (Corning, Woodlands, CA). Hydranal Formamide Dry was purchased from Honeywell (Charlotte, NC). Methanol, 99.8% extra dry was provided from Acros Organics (Fair Lawn, NJ). CellTiter-Glo® Luminescent Cell Viability Assay Kit was ordered from Promega (Madison, WI). All other chemicals were purchased from Thermo Fisher Scientific (Pittsburgh, PA).

**Plasmid Preparation.** pAAV-LacZ (AAV Helper-Free System, Stratagene, La Jolla, CA) was used in these studies as a model plasmid. Transfection-grade plasmids were amplified in *E. coli* (HB101 Competent cells, Promega, Madison, WI) and purified using Qiagen Maxi Plasmid kits (Qiagen, Hilden, Germany). Purified plasmids had OD260/280 ratios of 1.7-1.9 as determined by UV spectrophotometric analysis (Beckman Coulter, Brea, CA). Plasmid stock solutions were prepared in 10 mM Tris buffer pH 8.0 at a concentration of 1-2 mg/mL and stored at -20 °C. These stocks served as controls for all formulations evaluated in these studies.

**Film Preparation and Storage.** Film formulations were prepared in bulk and homogenized using a rotary mixer prior to addition of pAAV-LacZ to a final concentration of 0.1 mg/ml **(FIG. 34)**. Formulations were dispensed into 100 $\mu$l silicon molds (Bold Maker, Amesbury, MA) and films formed under aseptic conditions at 20 °C. Upon drying, films were reconstituted in Tris buffer for analysis or packed in Ziploc-like particle-free bags (American Cleanstat, Irvine, CA) inside a heat-sealed foil bag (Ted Pella Inc., Redding, CA). For most stability studies summarized in this manuscript packaged films were stored in a stability chamber (Binder, Tuttlingen, Germany) at 25 °C, 60% RH or at 4 °C, 40-50% RH.

**Establishment of Stability Indicating Assays.** Prior to the initiation of formulation screening studies, methods to detect different conformations of plasmid DNA in solution were established and their impact on several different assays evaluated **(FIG. 35)**. Standards representing the nicked and linear forms were created by enzymatic digestion with enzymes Bsal and Nb.BsrDI (New England Biolabs, Ipswich, MA) at 37 °C and 65 °C respectively. Digestion products were purified individually with Qiagen DNA Mini kits (Qiagen, Germany). Efficiency of the digestion reaction was confirmed by visualization of discrete bands of nicked and linear DNA via gel electrophoresis.

**Gel Electrophoresis.** Plasmid (0.25 $\mu$g) from rehydrated films was mixed with 6X loading dye before addition to individual wells of 0.8% agarose gels prepared in Tris-Borate-EDTA (TBE) buffer and cast using the OWL B1 Horizontal Electrophoresis System (Owl Separation Systems, Portsmouth, NH). DNA standards (1 kb Plus DNA ladder, New England Biolabs) were included in each gel for quality control. Gels were run at 105V for 90 minutes and stained with SYBR Safe dye (Thermo Fisher Scientific, Pittsburgh, PA). Bands were visualized and images captured using an Axygen Gel Documentation System (Corning, NY). The density of supercoiled and nicked bands was determined using ImageJ (U. S. National Institutes of Health, Bethesda, MD). Supercoiled plasmid content was calculated as the ratio of the fluorescence intensity of the supercoiled DNA band to the sum of the intensities of bands representing the three DNA conformations.

**Cell Culture and Transfection.** HEK293 cells (CRL-1573, ATCC, Manassas, VA, passage 12-26) were maintained in DMEM, 10% FBS and 1% penicillin (10,000 IU)/streptomycin (10,000 $\mu$g/ml). Cells ($7 \times 10^5$/well) were seeded in 12-well plates (Falcon, Corning, Durham, NC) to obtain 80% confluence in 24 hours. Culture media was replaced 1 hour before transfection. Plasmid (1 $\mu$g) in 50 $\mu$l of Opti-MEM (Life Technologies, Grand Island, NY) was mixed with 4 $\mu$g PEI in 50 $\mu$l of Opti-MEM and placed at room temperature for 20 minutes prior to addition to cells. Cells were then incubated at 37 °C, 5% $CO_2$ with culture medium replaced every 24 hours. Forty-eight hours after transfection, cells were harvested for assessment of transgene expression.

**Transfection Efficiency.** Analysis of beta-galactosidase expression was performed using a colorimetric assay based upon the reaction with ortho-nitrophenyl-P-galactopyranoside (ONPG). Forty-eight hours after transfection, cells were washed with PBS prior to treatment with Reporter Lysis buffer (Promega, Madison, WI). After a freeze/thaw cycle and centrifugation at 14,000 rpm for 1 min, 10 $\mu$l of diluted supernatant was mixed with 150 $\mu$l of 14.3 M β-mercaptoethanol in 150 mM phosphate buffer at pH 7.5 and held at 37 °C, 5% $CO_2$ for 5 min. At that time, 50 $\mu$l of 4 mg/ml ONPG was added to the sample. After 2.5 minutes, color development was stopped by adding 90 $\mu$l of 1M $Na_2CO_3$. Absorbance at 420 nm for each sample was recorded using a Glomax Multi-detection Plate Reader (Promega, Madison, WI). Protein content of cell lysates was determined by a standard Lowry assay (Bio-Rad, Hercules, California). Transfection efficiency is expressed as the amount beta-galactosidase present per mg cell protein in a given cell population for each formulation with respect to cells transfected with plasmid stock stored frozen at -20 °C in Tris buffer pH 8.

**Plasmid Copy Number.** The number of intact plasmids in a given formulation was determined by quantitated PCR. Samples were prepared by diluting plasmid formulated in Tris buffer or film matrix (0.1 mg/ml) 100,000-fold. Primers and TaqMan probe targeting the ITR regions of the pAAVlacZ plasmid were as follows: Forward Primer, 5'GGAACCCCTAGTGATGGAGTT-3', Reverse Primer, 5'-CGGCCTCAGTGAGCGA-3' (Sigma-Geneosys, The Woodlands, TX), TaqMan Probe 5'-/6 FAM/CACTCCCTCTCTGCGCGCTCG/3BHQ_1/-3' (Applied Biosystems, Foster City, CA). Reactions for each sample (25 $\mu$l) consisted of 5 $\mu$l sample, 20 $\mu$l of mixture of forward primer (0.1 $\mu$M), reverse primer (0.3 $\mu$M), probe (0.1 $\mu$M), and 12.5 $\mu$l of 2$\times$ TaqMan Fast Advanced MasterMix (Thermo Fisher Scientific Baltics Vilnius, Lithuania) in MicroAmp Fast 96-well optical reaction plates (Applied Biosystems, Foster City, CA). Reactions were run on an Applied Biosystems *ViiA 7* system (Thermo Fisher Scientific, Pittsburgh, PA) according to the following protocol: 95 °C for 30s, followed by 40 cycles of 95 °C for 5s and 60 °C for 30s. Standard curve samples were prepared from the same plasmid in the range of 40 - 4$\times$10$^6$ copies/$\mu$l. The amount of intact plasmid recovered from each formulation was determined by dividing the copy number obtained from each formulation by that obtained from plasmid stock stored frozen at -20 °C in Tris buffer pH 8 and reporting this value as % plasmid copy number.

**Cell Viability.** Solutions of individual excipients were prepared in Tris buffer (pH 8.0). Ten microliters of each solution were added to HEK 293 cells (7$\times$10$^4$ cells/well) in a 96 well plate. Twenty-four hours after treatment, cell viability was assessed using the CellTiter-Glo Luminescent Cell Viability Assay kit according to the manufacturer's instructions (Promega, Madison, WI). Viabilities of greater than 80% were considered acceptable for test formulations.

**Physical Characterization: Dissolution.** Films containing 100 $\mu$g plasmid DNA were placed in sterile scintillation vials (Kimble Glass, Vineland, NJ) containing 1 ml nuclease free 10 mM Tris buffer pH 8 (Invitrogen, Grand Island, NY). Vials were kept at 37 °C and solutions stirred at 60 rpm for 15 minutes. Samples (15 $\mu$l) were collected and replaced by an equal volume of buffer to maintain a constant volume throughout the study. DNA concentration in each sample was determined by measuring absorbance at 260 nm. Release rate was calculated as accumulative percentage of plasmid released from the film with respect to plasmid in liquid film formulation.

**Physical Characterization: Moisture Content.** Films were dissolved in 1 ml extraction solvent (1:1 volume ratio anhydrous formamide:extra dry methanol) at 50 °C. The weight of dry film (m1) and extraction solvent (m2) were recorded. Moisture content of blank extraction solvent (B%) and film containing sample (C%) was determined by Karl Fischer Titration using a V10S Volumetric Karl Fischer Titrator (Mettler Toledo, Columbus, OH). Specifically, 0.3 ml of sample was injected to the titration chamber and the sample mixed with extra dry methanol for 3 minutes before titration was initiated. Moisture content in film was calculated using the following equation:

$$Moisture\ content\ (\%) = C\% \times \left(\frac{m1 + m2}{m1}\right) - \left(\frac{B\% \times m2}{m1}\right)$$

**Physical Characterization: Tensile Strength.** Films (2.5 $\times$ 2.5 cm) were immobilized by tensile grips (TA-108S-5) on a TA XT Plus Texture Analyzer (TA Instruments, New Castle, Delaware). The pre-test speed was set at 2 mm/sec; test speed at 1 mm/sec and post-test speed at 10 mm/sec. The distance for the probe to travel through puncture site was 12 mm and the trigger force was 5.0 g. The maximum force required to break films indicates the strength of film while distance traveled correlates to elasticity of the film.

**Physical Characterization: Differential Scanning Calorimetry (DSC).** Films were cut into 5-10 mg pieces, hermetically sealed in aluminum sample pans (DSC Consumables, Austin, MN) and placed in a TA Q20 differential scanning calorimeter (TA Instruments; New Castle, Delaware). A conventional modulated method was followed with 1 °C amplitude and 60 seconds modulation. The ramp rate was set at 3 °C/min during the run from 30 °C to 180 °C. The flow rate of dry nitrogen gas was 50 mL/min. Data was analyzed using TA instruments Trios v.5.1.1.46572 analysis software.

**Physical Characterization: X-Ray Diffraction (XRD).** A Rigaku MiniFlex 600 II was employed to study the crystallinity of films and film components. Films were fixed on a hollow aluminum frame as a sample holder prior to inserting into the diffractometer. Measurements were taken with the scanning step of 0.04°, the 20 scanning range of 5-70° and generator operating voltage and current 40 kV and 15 mA, respectively.

**Physical Characterization: Fourier Transform Infrared Spectroscopy (FTIR).** FTIR spectroscopic analysis of films was performed using a Nicolet™ iS50 FT-IR Spectrometer (Thermo Fisher Scientific; Waltham, Massachusetts). Films were placed in a Smart Omni-Sampler (Thermo Fisher Scientific; Waltham, Massachusetts) and scans conducted in the 4000-800 cm$^{-1}$ region using an ATR (attenuated total reflectance) method with a diamond/ZnSe prism.

**Statistical Analysis.** Statistical analysis of data was conducted using Prism software (GraphPad Prism v.9.5.1, San Diego, CA). Paired t-tests were used to compare significant differences between values obtained from two treatment groups. For studies containing more than two unique treatments, one-way or two-way ANOVA were used to assess statistical significance. Dunnett's multiple comparison tests were used to compare individual treatment groups to a control group while Tukey post-hoc tests were utilized to compare every two groups to an entire set of data.

**Example 3 - Optimization of Transfection Reagent-Complexed Plasmid DNA Film Formulations**

In addition to optimizing film formulations for naked pDNA, formulations were also optimized for transfection reagent-complexed pDNA. Polyethyleneimine (PEI), a common transfection reagent which condenses DNA into positively charged complexes, can be added to solutions prepared from rehydrated film for efficient cell entry. Thus, to simplify the transfection process for use in large scale production of recombinant proteins and virus, the stability of pre-formed DNA complexes using either PEI or lipofectamine (LFP), a common lipid-based transfection reagent, was evaluated within the film matrix.

**A. PEI-Complexed pDNA**

In a first experiment, pDNA complexed with PEI was prepared for use with four optimized formulations (F1-F4) to assess the impact of the formulations on transfection efficiency. pDNA-PEI complexes were prepared by mixing plasmid DNA (2.5 $\mu$g DNA/40 $\mu$l) and PEI (10 $\mu$g/40 $\mu$l) in Tris buffer (pH 8.5) and waiting for 20 min for complexes to form. Complexes were then stored for 4 hours at room temperature in either OPTI-MEM™ or PBS as a solvent, neither of which affected transfection efficiency of the pDNA-PEI complexes **(FIG. 16A)**.

pDNA complexed with PEI was then added to four optimized formulations F1-F4, and the impact of the formulations on pDNA transfection efficiency assessed. Complexes were added so that each 100 $\mu$l film contained 2.5 $\mu$g DNA with a DNA:PEI ratio of 1:4. Formulations F1-F4 for PEI-complexed pDNA were as follows:

F1: 2% w/v PVA
F2: 0.8% w/v HPMC A4C
F3: 2% w/v Gelatin (pH 5)
F4: 2% w/v Gelatin (pH 8)

Gelatin-based film formulations F3 and F4 comprising pDNA complexed with PEI in OPTI-MEM™ or PBS as a solvent maintained transfection efficiency of pDNA after drying of the films **(FIG. 16B)**.

Additional results of films including the pDNA complex after storage for up to 11 days at 25 °C and 60% RH were obtained with the following gelatin-based film formulations **(FIG. 16C)**:

F1: 2% w/v Gelatin (pH 5) in OPTI-MEM™
F2: 2% w/v Gelatin (pH 5) in PBS
F3: 2% w/v Gelatin (pH 8) in OPTI-MEM™
F4: 2% w/v Gelatin (pH 8) in PBS

As before, pDNA-PEI complexes were added to the film matrix so that each 100 $\mu$l film contained 2.5 $\mu$g DNA with a DNA:PEI ratio of 1:4. These results demonstrate that results gelatin-based film formulations can significantly improve pDNA complex stability and performance after storage for up to 11 days at 25 °C and 60% RH.

In a second experiment, complexes were again formed in several aqueous media (water, Opti-MEM, PBS, Tris buffer) by three different methods. Method 1 involved mixing PEI and plasmid DNA in equal volumes together prior to addition to the film formulation. This approach supported a high level of transfection (79.0 $\pm$ 0.5% Opti-MEM, 96.1 $\pm$ 1.0% PBS, **FIG. 45A**). Method 2, where PEI was added dropwise to a solution of plasmid, produced somewhat improved results (81.5 $\pm$ 2.1% Opti-MEM, 107.8 $\pm$ 2.3% PBS). In contrast, Method 3, where plasmid was added dropwise to a solution of PEI, induced rapid precipitation of the plasmid which significantly compromised transfection efficiency. Water and Tris buffer, which did not have the pH and ionic strength to support proper condensation of the plasmid also failed to transfect cells **(FIG. 45A)**. Thus Method 1 was selected to form plasmid-PEI complexes prior to drying within the film matrix for the remainder of the study. While preparations made in PBS supported higher transfection efficiencies than Opti-MEM during the screening process, there was no statistical difference between transfection efficiency of plasmid in films prepared with either solution ($p > 0.18$ in all polymers, **FIG. 45B**). Films prepared with gelatin were able to maintain full transfection efficiency of PEI-DNA complexes (103.2 - 111.6% with respect to freshly made complexes in media). Complexes prepared in media and left at room temperature during the drying process retained only 12.2-14.1% of the original transfection capacity (RT control in **FIG. 45B**). Gelatin films prepared in PBS were able to maintain a high level of transfection efficiency after 7 (pH 5, 83.2 $\pm$ 1.2%; pH 8, 93.4 $\pm$ 4.5%) and 14 (pH 5, 73.3 $\pm$ 1.9%; pH 8, 71.1 $\pm$ 7.1%) days at 25 °C **(FIG. 45C)**. The pH of the film matrix significantly impacted the stability of PEI-DNA complexes prepared in Opti-MEM after 14 days at 25 °C (pH 5, 64.9 $\pm$ 3.6% and pH 8, 34.1 $\pm$ 3.0% respectively).

**B. LIPOFECTAMINE™-Complexed pDNA**

In a first experiment, pDNA complexed with LIPOFECTAMINE™ was prepared in four optimized formulations (F1 Liq, F1-F3 Film), and the impact of the formulations on pDNA transfection efficiency was assessed. Formulations F1 Liq and F1-F3 Film for LIPOFECTAMINE™-complexed pDNA were as follows:

> F1 Liq: 2% w/v HPMC A4C (viscosity 400 cP) liquid formulation, 10 mM Tris (pH 8.5)
> F1 Film: 2% w/v HPMC A4C (viscosity 400 cP) film formulation, 10 mM Tris (pH 8.5)
> F2 Film: 1% w/v HPMC A4C (viscosity 100 cP) film formulation, 10 mM Tris (pH 8.5)
> F3 Film: 2% w/v Gelatin

Formulations contained 2 μg plasmid DNA complexed with LIPOFECTAMINE™ 2000 CD REAGENT, and films were dried in 100 μl silicon molds.

**FIG. 15** shows the impact of optimized liquid (liq) or film formulations F1-F3 on the transfection efficiency of lipofectamine-complexed plasmid DNA prepared in the liquid or film formulations after storage for 2 weeks at 25 °C and 60% relative humidity (RH). The transfection efficiency of plasmid DNA prepared in the F3 formulation was statistically higher ($p < 0.001$) than that of the plasmid DNA prepared in a buffer-only control (Buffer (Liq)), F1 Liquid (F1 Liq), and F2 Film on days 4-21. The transfection efficiency of plasmid DNA prepared in the F3 Film formulation was also statistically higher ($p < 0.05$) than plasmid DNA prepared in the F1 Film formulation on day 21.

In a second experiment, formation of DNA-lipofectamine (LPF) complexes was optimized by altering the volume of Opti-Pro media utilized to dilute plasmid and lipofectamine individually prior to mixing **(FIG. 46A)**. The highest levels of transfection were achieved by placing 1 μg of DNA in 10-12.5 μl media. This was 15-fold higher than what is recommended by the manufacturer (1 μg DNA in 50 μl media). Films prepared using gelatin (pH 5, P3 and pH 8, P4) and different types of HPMC (P6 and P7) retained more than 80% of the original transfection efficiency during the film forming process while those made with chitosan (P1), pectin (P2) and alginate (P8) did not support transfection efficiency at all **(FIG. 46B)**. Formulations F2, F3 and F4 **(Table 4)** preserved the transfection efficiency of LPF-DNA complexes within the film matrix after 4 days at either 4 °C or 25 °C while transfection efficiency of complexes in media alone (Control) fell to $48.0 \pm 0.3\%$ at 4 °C and $3.2 \pm 0.2\%$ at 25 °C **(FIG. 46C)**. Formulations F3 and F4 retained $86.2 \pm 6.0\%$ and $92.0 \pm 3.7\%$ of their original transfection efficiency after 14 days at 25 °C while complexes in media alone fell to $12.6 \pm 0.1\%$ **(FIG. 46D)**. Taken together, these data demonstrate that embedding DNA complexes within the film matrix can preserve transfection efficiency during storage at 25 °C as complexes stored in buffer under the same conditions precipitated and failed to transfect cells after 24 hours at the same condition. It was also clear that LPF-DNA complexes were more stable than those formed with PEI as transfection efficiency never fell below 90% during the 14-day period. Thus, the stability of mRNA LNPs within a film matrix was evaluated.

**Example 4 - Optimization of LNP-Encapsulated RNA Film Formulation**

In addition to optimizing film formulations for naked or transfection reagent-complexed DNA, studies were also performed to optimize film formulations for lipid nanoparticle (LNP)-encapsulated RNA (e.g., mRNA). LNPs are fatty droplets with an aqueous core that contains a series of reversed micelles that bind, protect and deliver nucleotides like mRNA **(FIG. 43)**. Several different types of lipids are commonly used to make these particles. Ionizable lipids retain a positive charge within the acidic environment of the aqueous core to form micellular networks that bind mRNA through electrostatic interactions. When they reach physiological pH in solution or a living organism, they become neutral making them biocompatible. In the endosome, ionizable lipids revert to their original positively charged state where they interact with anionic phospholipids to disrupt the endosomal membrane and facilitate release of intact mRNA containing micelles into the cytoplasm **(FIG. 44)**. Neutral phospholipids and cholesterol are responsible for maintaining an organized lipid shell. Helper lipids such as distearoylphosphatidylcholine (DSPC) also contribute to endosomal escape by interacting with the endosomal membrane. Small amounts of lipids attached to polyethylene glycol (PEG) dispersed throughout the lipid phase of the particle can significantly impact particle size, surface charge and physical stability as they have been shown to prevent aggregation, extend circulation time and reduce particle clearance. PEG molecules on the outer shell of the particle create a hydration shell that circumvents opsonization and partially governs particle stability. PEG can also serve as a linker for attachment of targeting molecules to limit where particles are taken up and genes expressed.

LNPs containing mRNA are generally prepared by mixing single stranded ribonucleic acid molecules in an acidic aqueous buffer with lipids in an ethanolic solution in a microfluidic system where speed, volume ratio, and the flow pattern within mixing chamber are highly controlled **(FIG. 45)**. In practice, 40-80% of LNPs prepared in this manner do not contain any mRNA while each LNP carries 2-6 mRNA molecules. Unencapsulated materials are removed by dialysis or ultracentrifugation and particles are suspended in a buffer, often phosphate buffer saline (PBS, pH 7.4) or Tris (pH 8). Buffer selection has been shown to significantly impact the physical stability of LNPs. For example, Tris buffered self-replicating mRNA containing LNPs were larger than those stored in phosphate buffered saline at 4 and -20 °C. Tris buffer was also found to minimize changes in solution pH during freezing and adduct formation from aldehyde impurities from lipid components. Each of the current marketed COVID-19 vaccines are formulated as frozen buffered solutions containing sucrose (Table 3). While each of these products has been shown to be stable in solution at 4 °C for up to 10 weeks, they are discarded within 24 hours at 25 °C. It is interesting to note that an LNP-based product containing siRNA (ONPATTRO®) is formulated as a buffered solution that is stable at 4 °C for 3 years (Table 3). This improved stability profile may be due to the short double-stranded structure while mRNA and self-replicating RNAs are long single strands.

**Table 3 -- Stability of current RNA LNPs products in the market**

|  | COMIRNATY® (Pfizer, BioNTech) | SPIKEVAX™ (Moderna) | ONPATTRO® (Alnylam) |
|---|---|---|---|
| Vector | mRNA LNPs | mRNA LNPs | siRNA LNPs |
| Manufacturer | Covid-19 vaccine from Pfizer and BioNTech | Covid-19 vaccine from Modema | Polyneuropathy of hereditary transthyretin-mediated amyloidosis from Spark Therapeutics |
| Storage | -80°C for long term<br>-20 °C in 2 weeks<br>4 °C in 10 weeks<br>25 °C within 2 hours | -20 °C for long-term<br>4 °C in 30 days<br>25 °C within 24 hours | 4 °C for 3 years<br>25 °C for 14 days |
| Component | For each 0.3 ml dose, 0.01 mg potassium chloride, 0.01 mg monobasic potassium phosphate, 0.36 mg sodium chloride, 0.07 mg dibasic sodium phosphate dihydrate, and 6 mg sucrose | For each 0.5 ml dose, 0.31 mg tromethamine, 1.18 mg tromethamine hydrochloride, 0.043 mg acetic acid, 0.20 mg sodium acetate trihydrate, and 43.5 mg sucrose. | Disodium hydrogen phosphate heptahydrate, Potassium dihydrogen phosphate anhydrous, Sodium chloride |

To date, there is limited information available regarding the stability of mRNA LNPs in the solid state. Careful review of the literature revealed that most lyophilized products are stored frozen to maintain transfection efficiency. LNPs prepared in formulations containing more than 5% (w/v) of sugar demonstrated an increase in particle size prior to freeze-drying. This increased further once the process was complete. Storage at 4 or -20 °C was required to conserve the physicochemical properties of the particles. Several reports were conflicting as transfection efficiency or *in vivo* bioactivity of lyophilized LNPs were not compromised after the lyophilization process was complete despite the fact that notable drops in encapsulation efficiency and increases in particle size were detected once drying was complete. Taken together, this suggests that lyophilization cannot yet support storage of mRNA LNPs outside of the cold chain and that physiochemical properties of LNPs may not fully predict *in vitro* or *in vivo* performance.

Disclosed herein is a drying technique that avoids cold denaturation and dramatic temperature fluctuations during freeze-drying for its ability to stabilize mRNA LNPs under ambient conditions within a film matrix. Also disclosed is use of data collected from commonly accepted stability indicating assays for LNP products to predict bioactivity. Successful identification of a film-based formulation that eliminates the need for ultra-low cold chain storage and transport and identifying predictors of LNP performance will significantly improve access to these lifesaving medications and preparedness for the next pandemic.

**A. Summary of Formulations.**

Specific details of Example 3 and 4 formulations are summarized in **Table 4.**

**Table 4**

|  | Label | Formulation Components |
|---|---|---|
| **FIG. 45** | Gel 5 | 2% Gelatin type B in 50 mM Citrate buffer pH 5 + 0.5% glycerol |
|  | Gel 8 | 2% Gelatin type B in 10 mM Tris buffer pH 8 + 0.5% glycerol |

(continued)

|  | Label | Formulation Components |
|---|---|---|
| **FIG. 46** | P1 | 1% Chitosan in 1% acetic acid |
|  | P2 | 1.5% Pectin in Tris pH 8 |
|  | P3 | 2% Gelatin type B in 50 mM Citrate buffer pH 5 + 1% glycerol |
|  | P4 | 2% Gelatin type B in 10 mM Tris buffer pH 8 + 1% glycerol |
|  | P5 | 2% PVA in Tris pH 8 |
|  | P6 | 1% A15C HPMC in Tris pH 8 |
|  | P7 | 2% A4C HPMC in Tris pH 8 |
|  | P8 | 1% Sodium alginate in Tris pH 8 |
|  | F1 | 2% PVA + 1% Arg |
|  | F2 | 1% A15C HPMC |
|  | F3 | 2% A4C HPMC + 1% Arg + 1% Glycerol |
|  | F4 | 2% gelatin type B in 50 mM Citrate pH 5 + 1% glycerol |
|  | F5 | 2% gelatin type B in 10 mM Tris pH 8 + 1% glycerol |
| **FIG. 47** | Buf | 10 mM Tris pH 8 |
|  | P1 | 1.5% K100LV HPMC+ 2% Glycerol |
|  | P2 | 2% A15LV HPMC + 2% Glycerol |
|  | P3 | 3% E6LV HPMC + 2% Glycerol |
|  | P4 | 2% Hydroxyethyl cellulose |
|  | P5 | 1% Wallocel + 0.5% Glycerol |
|  | P6 | 4% Pullulan + 0.5% Glycerol |
|  | P7 | 2% PVA |
|  | P8 | 2% Gelatin type A + 0.1% Pluronic F68 |
|  | P9 | 5% Plasdone C17 |
|  | P10 | 5% Plasdone C12 |
| **FIG. 51** | High DMPE-PEG | 1.5% K100LV + 3% Glycerol + 0.01% Pluronic F127 + 0.04% DMPE-PEG |
|  | Low DMPE-PEG | 1.5% K100LV + 3% Glycerol + 0.01% Pluronic F127 + 0.008% DMPE-PEG |
|  | High DMG-PEG | 1.5% K100LV + 3% Glycerol + 0.01% Pluronic F127 + 0.04% DMG-PEG |
|  | Low DMG-PEG | 1.5% K100LV + 3% Glycerol + 0.01% Pluronic F127 + 0.008% DMG-PEG |
| **FIG. 52** | DMPE-PEG + K4M | 1% K4M + 3% Glycerol + 0.01% Pluronic F127 + 0.008% DMPE-PEG |
|  | DMG-PEG + K4M | 1% K4M + 3% Glycerol + 0.01% Pluronic F127 + 0.008% DMG-PEG |
|  | DMPC + K4M | 1% K4M + 3% Glycerol + 0.01% Pluronic F127 + 0.01% DMPC |
|  | DMPE-PEG + K100LV | 1.5% K100LV + 3% Glycerol + 0.01% Pluronic F127 + 0.008% DMPE-PEG |
|  | DMG-PEG + K100LV | 1.5% K100LV + 3% Glycerol + 0.01% Pluronic F127 + 0.008% DMG-PEG |

(continued)

|  | Label | Formulation Components |
|---|---|---|
|  | DMPC + K100LV | 1.5% K100LV + 3% Glycerol + 0.01% Pluronic F127 + 0.01% DMPC |
| **FIG. 53** | Pluronic F127 | 1% K4M + 3% Glycerol + 0.01% Pluronic F127 + 0.008% DMPE-PEG |
|  | Tween 80 + Pluronic F127 | 1% K4M + 3% Glycerol + 0.01% Pluronic F127 + 0.01% Tween 80 + 0.008% DMPE-PEG |
|  | Tween 80 + Pluronic F127 | 1% K4M + 3% Glycerol + 0.01% Pluronic F127 + 0.01% Tween 20 + 0.008% DMPE-PEG |
|  | Pluronic F68 + Pluronic F127 | 1% K4M + 3% Glycerol + 0.006% Pluronic F127 + 0.006% Pluronic F68 + 0.008% DMPE-PEG |
|  | Brij 58 + Pluronic F127 | 1% K4M + 3% Glycerol + 0.01% Pluronic F127 + 0.005% Brij 58 + 0.008% DMPE-PEG |
| **FIGs. 54 & 56** | Optimized formulation (OF) | 1% K4M + 3% Glycerol + 0.006% Pluronic F127 + 0.006% Pluronic F68 + 0.008% DMPE-PEG in Tris buffer pH 8 |
| **FIG. 60** | F20 | 1.5% K100LV + 3% Glycerol + 0.01% Pluronic F127 |
|  | F21 | 1.5% K100LV + 0.15% K4M + 3% Glycerol + 0.01% Pluronic F127 |
|  | F22 | 1.5% K100LV + 0.3% K4M + 3% Glycerol + 0.01% Pluronic F127 |
|  | F23 | 1% K100LV + 0.5% K4M + 3% Glycerol + 0.01% Pluronic F127 |
|  | F24 | 1% K4M + 3% Glycerol + 0.01% Pluronic F127 |
| **FIGs. 55 & 63** | Optimized formulation (OF in pH 8.5) | 1% K4M + 3% Glycerol + 0.006% Pluronic F127 + 0.006% Pluronic F68 + 0.008% DMPE-PEG in Tris pH 8.5 |
|  | OF + Arginine | 1% K4M + 3% Glycerol + 0.006% Pluronic F127 + 0.006% Pluronic F68 + 0.008% DMPE-PEG + 0.05% Arg in Tris pH 8.5 |
|  | OF + Cysteine | 1% K4M + 3% Glycerol + 0.006% Pluronic F127 + 0.006% Pluronic F68 + 0.008% DMPE-PEG + 0.005% Cysteine in Tris pH 8.5 |
|  | OF + EDTA | 1% K4M + 3% Glycerol + 0.006% Pluronic F127 + 0.006% Pluronic F68 + 0.008% DMPE-PEG + 1mM EDTA in Tris pH 8.5 |

**B. pH & Film Base Selection**

Films prepared with gelatin (Grade A, pI 7-9.5) were shown to improve transfection efficiency of mRNA LNPs containing the gene sequence for beta-galactosidase **(FIG. 17).** Solutions containing mRNA LNPs (100 μg/ml) were prepared with 2% gelatin in various buffers. Films formulated at pH 5 were prepared in 100 mM citrate buffer while films formulated at pH 7-9 were prepared in 10 mM Tris and pH adjusted accordingly. Transfection efficiency was assessed prior to drying (Liquid) and after films were formed (Film). The pH 9 preparation significantly enhanced transfection efficiency of the LNP stock and maintained enhanced transfection efficiency during the film forming process.

In a second experiment, since it was previously found that gelatin-based films prepared in buffers of pH 5 and pH 8 preserved transfection efficiency of DNA complexes within the film matrix, the impact of pH on mRNA-containing LNPs was evaluated **(FIG. 47).** Buffers of pH 6 and pH 6.5 increased average particle size and the particle distribution index (PDI) of the LNPs after 3 days at 4 °C **(FIG. 47A).** Encapsulation efficiency was also reduced, however, transfection efficiency remained high under these conditions **(FIG. 47B).** The average particle size and PDI of LNPs increased in formulations prepared with K100LV and buffers pH 7-9 during the film forming process **(FIG. 47C).** However, LNPs in formulations buffered at pH 8 and 9 had a transfection efficiency of 83.2 ± 10.6% and 165.8 ± 14.0% respectively despite a drop in encapsulation efficiency **(FIG. 47D).** Therefore, Tris buffer pH 8 was chosen for use in all formulations for screening studies.

Gelatin-based formulations that preserved transfection efficiency of DNA complexes did not support stability of LNPs containing mRNA **(FIG. 48).** LNPs placed in this formulation (P8) experienced a notable increase in particle size from 136 to 222 nm and associated particle distribution index from 0.055 to 0.338 (blue dots, **FIG. 48A).** Transfection efficiency also fell to 30.7 ± 7.8%. Screening of additional polymers revealed that formulations containing different types of HPMC (P1-P3) could maintain transfection efficiency with moderate increases in particle size and PDI during the drying process. Formulations containing hydroxyethyl cellulose (P4), wallocel (P5), pullulan (P6) and PVA (P7) formed large aggregates **(FIG. 48A)** that could not enter cells **(FIG. 48B)**. LNPs formulated with K100LV had properties (particle size 189 nm; PDI 0.297; transfection efficiency 96.7%,) very similar to those of the original stock solution **(FIG. 59)**.

## C. Sugar Selection

While gelatin-based films were shown to improve transfection efficiency of mRNA LNPs, the inclusion of sugars in the film formulations impaired stability of mRNA LNPs in the frozen and dried states **(FIG. 18A)**. Particle size analysis of LNPs (25 $\mu$g/ml) in phosphate buffered saline (PBS, pH 7.4) and 1% glycerol (Record 29, green line) or 1% sucrose (Record 62, blue line) after storage at -80 °C showed that inclusion of sucrose with the LNPs increased the size distribution of the LNPs. All data were compared to particle size data for LNPs stored in water at 4 °C (Record 58, red line), which remain unaggregated. In contrast to sugars, the inclusion of film-forming polymers in the film formulations preserved transfection efficiency during the film forming process **(FIG. 18B)**. Formulations tested were PBS-T: Trehalose 1%; Gel: Gelatin 2%; PVA: polyvinyl alcohol 2%; K4M: HPMC 2% (4000 cps); and A4C: HPMC 2% (400 cps). All formulations in were prepared in 10 mM Tris buffer pH 8.1.

As an alternative to sucrose, cyclodextrins were included in liquid and dried formulations, and the effect of the cyclodextrins on the transfection efficiency of LNP-encapsulated mRNA after storage in liquid film formulations or in dried films was tested. Cyclodextrins were demonstrated to both improve transfection efficiency in liquid formulations and maintain transfection efficiency in dried films. **FIG. 19A** shows the stability of mRNA LNPs (25 $\mu$g/ml) stored in liquid formulations for 5 hours (dark bars) and 24 hours (white bars) at 20 °C. Formulations tested included: Stock: Tris buffer (pH 9); PBS: phosphate buffered saline (pH 7.4); Alpha: alpha cyclodextrin 1% (w/v); Beta: beta cyclodextrin 0.1% (w/v); Gamma: gamma cyclodextrin 1% (w/v); HP-BCD: hydroxypropyl beta cyclodextrin 1% (w/v); and M-BCD: methoxy beta cyclodextrin 1% (w/v). **FIG. 19B** shows the transfection efficiency of mRNA LNPs (25 $\mu$g/ml) in films stored at 4 °C for 4 days. Data is normalized to transfection efficiency of fresh frozen stock of LNPs. Formulations tested included: Buff (Liq): LNPs stored in phosphate buffered saline (pH 7.4) in liquid form; G-HP-B: Gelatin 2%, hydroxypropyl beta cyclodextrin 1% (w/v); G-GCD: Gelatin 2%, gamma cyclodextrin 1% (w/v); and G-MBCD: Gelatin 2%, methoxy beta cyclodextrin 1% (w/v). All of the foregoing cyclodextrin formulations were prepared in PBS pH 7.4.

## D. Glycerol & Surfactants

Increasing glycerol from 2 to 4% significantly reduced particle size from 199 nm to 161 nm and PDI from 0.336 to 0.131 (p < 0.001, **FIG. 49A)** with no change in transfection efficiency (2%, 83.2 $\pm$ 10.6% vs 4%, 71.8 $\pm$ 5.9%, p=0.63, **FIG. 49B)**. Films made with 8% glycerol were wet like hydrogels despite longer drying time. Addition of the hydrophilic, non-ionic surfactant, Pluronic F127, to the film matrix produced smaller, more homogenous LNPs in the solution produced upon rehydration (F127 Base, **FIG. 50A)**. This also significantly improved transfection efficiency 189.1 $\pm$ 9.7%, **FIG. 50B)**. This effect was more pronounced when Pluronic F127 was mixed with the LNP stock prior to mixing with bulk film formulation (F127 LNP, particle size 159 nm; PDI 0.137; EE 83.0%; transfection efficiency 234.4%). While these results seemed promising, films prepared using the F127 LNP approach demonstrated a significant increase in particle size (152 $\pm$ 6 nm to 213 $\pm$ 7 nm) and PDI (0.122 $\pm$ 0.027 to 0.354 $\pm$ 0.018) after storage at 4 °C for a week with a corresponding drop in transfection efficiency (127.5 $\pm$ 5.4% to 33.5 $\pm$ 5.2%, No PEG lipid, **FIG. 51)**.

## E. PEG Lipids

Two PEG lipids commonly utilized for fabrication of LNPs, DMPE-PEG and DMG-PEG, were included as excipients in the film matrix at two concentrations: 0.008% (low) and 0.04% (high). Inclusion of these excipients in the film base did significantly improve particle size and PDI with respect to formulation that did not contain lipid (no PEG lipid 152 $\pm$ 6nm vs low DMPE-PEG 143 $\pm$ 1 nm, p = 0.61; vs high DMPE-PEG 131 $\pm$ 4 nm, p = 0.01, **FIG. 51A)**. The impact of the lipid was more obvious after 7 days at 4 °C as particle size increased in films without lipid (213 $\pm$7 nm) while that in low DMPE-PEG and high DMPE-PEG remained at 166 $\pm$ 1 nm and 126 $\pm$ 5 nm respectively, p < 0.001). Despite improvements in particle size, high concentrations of lipid made LNPs "leaky" with encapsulation efficiencies of 36.2 $\pm$ 3.3% (DMPE-PEG) and 56.2 $\pm$ 0.4% (DMG-PEG, **FIG. 51C)**. Transfection efficiency followed a similar trend in freshly prepared films (10.3 $\pm$ 1.7% (DMPE-PEG) and 37.9 $\pm$ 11.3% (DMG-PEG, **FIG. 51D)**. Low concentrations of PEG lipid did not compromise encapsulation and transfection efficiencies during the film forming process (Day 0, red and dark blue bars, **FIGs. 51C** and **51D)**. Despite these findings, transfection efficiency of LNPs in these preparations fell to 76.5 $\pm$ 10.6% (DMPE-PEG) and 66.9 $\pm$ 13.2% (DMG-PEG) after 7 days at 4 °C.

**F. Polymer Viscosity**

Initial screening of film forming polymers was conducted with low viscosity preparations for ease of dissolution and for use as injectable products. Since the most optimal polymer from those studies could not support long-term stability of mRNA containing LNPs, evaluated was stability in a high viscosity polymer shown to be superior to the low viscosity polymer used in studies for stabilization of AAV9 within a film matrix. Mixing the polymers (K4M and K100LV) at different ratios generated solutions with a range of viscosity from 56 to 169 cps **(Table 5)**.

**Table 5**

| | Film Viscosity | | |
| --- | --- | --- | --- |
| | Viscosity (cp) | Speed (rpm) | Torque (%) |
| **F20** | 56.3 | 20 | 24 |
| **F21** | 90.9 | 20 | 38.8 |
| **F22** | 124.7 | 20 | 53.3 |
| **F23** | 139.4 | 20 | 39.5 |
| **F24** | 168.8 | 20 | 71.9 |

LNPs in films prepared with these preparations had comparable particle size (162-193 nm), PDI (0.158-0.261) and encapsulation efficiency (80.0-87.5%) values **(FIGs. 60A-60C)**. Transfection efficiency was 2-4 fold higher than that seen from a LNP stock solution stored frozen **(FIG. 60D,** Buffer). After 4 weeks at 4 °C, a significant increase in particle size and drop in transfection efficiency was found in all formulations tested. Since formulations containing the low (F20) and high viscosity (F24) polymers maintained the smallest particles (268 $\pm$ 43 and 234 $\pm$ 14 respectively) and the best encapsulation efficiencies (82.6 $\pm$ 1.2% and 80.8 $\pm$ 2.3% respectively), additional studies with films containing each polymer and several different phospholipids were conducted **(FIG. 52)**.

LNPs in films prepared with K4M and PEG lipids demonstrated a significant increase in particle size (DMPE-PEG, 168 nm to 193 nm; DMG-PEG, 163 nm to 215 nm, **FIG. 52A)** as well as a significant decrease in encapsulation efficiency (DMPE-PEG, 80 to 63%; DMG-PEG 80 to 59%, **FIG. 52C)** after 28 days at 4 °C. Films containing K4M and the phospholipid, DMPC supported minimal changes in particle size (150 to 160 nm) and PDI (0.195 to 0.215, **FIG. 52B)** and encapsulation efficiency (85% to 79%) after 28 days at 4 °C. Despite this, transfection efficiency of LNPs in this formulation was very low after drying was complete (47.0 $\pm$ 4.5 %, D0) and after 28 days at 4 °C (14.4 $\pm$ 3.8%, **FIG. 52D)**. Degradation of LNPs in formulations prepared with the low viscosity polymer occurred at a much faster rate. Significant increases in particle size were found in these preparations after 14 and 28 days at 4 °C (on Day 14: DMPE PEG 171 $\pm$ 9 nm; DMG PEG 270 $\pm$ 26 nm; DMPC 275 $\pm$ 6 nm and on Day 28: DMPE PEG 309 $\pm$ 17 nm; DMG PEG 348 $\pm$ 37 nm; DMPC 288 $\pm$ 19 nm, p<0.001, **FIG. 52A)**. A similar trend was seen with respect to encapsulation efficiency (on Day 14: DMPE PEG 67.7 $\pm$ 4.6%; DMG PEG 44.8 $\pm$ 9.1%; DMPC 46.7 $\pm$ 4.0% and on Day 28: DMPE PEG 48.7 $\pm$ 6.2; DMG PEG 26.4 $\pm$ 4.9%; DMPC 47.2 $\pm$ 1.0%, **FIG. 52C)**. However, transfection efficiency of LNPs in these preparations were similar to those in formulations containing the K4M polymer **(FIG. 52D)**.

**G. Surfactant Combinations**

As shown in **FIG. 23,** the addition of a plasticizer (e.g., Pluronic F127) can significantly improve transfection efficiency of LNP-encapsulated mRNA compared to transfection efficiency of LNP-encapsulated mRNA prepared in films comprising other surfactants or plasticizers. Films containing 100 μg/mL of mRNA LNPs containing the gene sequence for firefly luciferase were prepared with 1.5% KLV100 film base, a surfactant and plasticizer comprising 0.5% pullulan, 0.1% PMAL, 0.5% PEG 1500, or 0.01% Pluronic F127, and glycerol 2%. Transfection efficiency represents the number of cells expressing the luciferase transgene normalized to that obtained with fresh unformulated LNP stock.

In order to improve LNP stability within the film matrix further, an additional study in which non-ionic surfactants, often used in pharmaceutical formulations to prevent aggregation and in suspension culture media to protect cells from hydrodynamic and bubble-induced shear, were combined with Pluronic F 127. Addition of surfactant combinations did not significantly impact particle size. PDI and transfection efficiency of LNPs during the film forming process (D0, **FIG. 53)**. However, encapsulation efficiency was significantly reduced by all formulations tested (D0, **FIG. 53C)**. After 2 weeks at 4 °C, LNPs in formulations containing surfactant combinations had significantly better physical characteristics than those with Pluronic F127 alone. Formulations containing Pluronic F68 and Brij 58 had the lowest particle size (151 $\pm$ 1 nm and 155 $\pm$ 4 nm) and PDI (0.150 $\pm$ 0.001 and 0.184 $\pm$ 0.001) of all formulations tested **(FIGs. 53A** and **53B)**. While encapsulation efficiency was significantly reduced in all formulations tested at the Day 14 timepoint, the formulation containing Pluronic F68 had the highest value (77.4 ± 0.5%). Despite these results, transfection efficiency was reduced by approximately 40% in all formulations tested (D14, **FIG. 53D).**

## H. pH of Film Base

Screening of several different film formulations revealed that the pH of films prepared with 10 mM Tris pH 8 dropped to 6.5 after drying and that sucrose (10%) in the same buffer reduced pH further to 6.0 and those prepared with PBS fell to 5.5 **(Table 6).**

**Table 6**

| pH of Dry Film | | |
|---|---|---|
| **Label** | **Buffer Type** | **pH of dry film** |
| Buffer | 10% Sucrose in 10 mM Tris pH 8 | 6 |
| pH 5.5 Film | 20 mM histidine buffer pH 5.5 | 5.5 |
| pH 7.4 Film | 150 mM PBS pH 7.4 | 5.5 |
| pH 8.0 Film | 10 mM Tris pH 8.0 | 6.5 |
| pH 8.5 Film | 10 mM Tris pH 8.5 | 7 |
| pH 9.0 Film | 10 mM Tris pH 9.0 | 7.5 |

Internal film pH did not impact the physical and biological characteristics of LNPs during the drying process (D0, **FIG. 54).** After 14 days at 4 °C, the particle size and PDI of LNPs in formulations prepared with pH 7.4 buffer increased to 242 ± 17 nm and 0.365 ± 0.030 respectively **(FIGs. 54A** and **54B).** Despite this, encapsulation efficiency remained high (82 ± 0.6%) and transfection efficiency was 62.8 ± 3.4% **(FIGs. 54C** and **54D).** LNPs stored in films prepared with buffer at pH 8.5 had optimal particle size (149 ± 1 nm), PDI (0.139 ± 0.009) encapsulation (82.6 ± 0.9%) and transfection (103.9± 6.3%) efficiencies. While those prepared with buffer at pH 9 had similar properties, transfection efficiency increased to 197 ± 19.2% after 2 weeks at 4 °C. Since this could be a sign of particle instability, a buffer of pH 8.5 was chosen to prepare additional optimized formulations.

## I. Amino Acids & EDTA in an Optimized Formulation

To further improve the stability of mRNA containing LNPs, compounds found to stabilize DNA within the film matrix for up to 9 months at room temperature were added to the optimized formulation of 1% K4M/ 3% Glycerol/0.006% Pluronic F127/0.006% Pluronic F68/0.008% DMPE-PEG made in 10 mM Tris buffer pH 8.5. The addition of arginine, a cationic alkaline amino acid, to the formulation significantly enhanced transfection efficiency of LNPs stored in the formulation for 14 days (241.5 ± 9.5%) and 28 days (268.5 ± 1.0%) **(FIG. 55D),** and up to 112 days **(FIG. 63D).** A similar effect was seen when EDTA, a chelating agent, was added to the optimized formulation (241.7 ± 8.4%, day 14; 265.4± 3.1%, day 28). Each of these formulations was also able to maintain particle size, PDI and encapsulation efficiency of LNPs for one month at 4 °C **(**FIGs. 55A-C) and up to 112 days at 4 °C **(**FIGs. 63A-C). It is also important to note that the optimized formulation alone was also capable of maintaining optimal physical and biological properties throughout the 28 day and 112 day periods at 4 °C. In contrast, incorporation of cysteine, known to prevent oxidation, did not significantly improve transfection efficiency at the day 14 timepoint (103 ± 7.7%) but did improve this value at the day 28 timepoint (210.3± 14.3%, **FIG. 55D).**

## J. mRNA LNP Concentration

**FIG. 20** shows that the concentration of mRNA LNPs in the film matrix can impact transfection efficiency upon rehydration. Films containing 40, 100, or 250 µg/mL amounts of mRNA LNPs were prepared in a base formulation of 1.5% KLV100, 0.5% Pullulan, and 3% glycerol. Films including the mRNA LNPs were dried, rehydrated, and transfection assessed. As shown, increasing concentrations of mRNA LNPs in the films corresponds to a reduction in transfection efficiency for the mRNA LNPs.

**K. Rehydration Solvent Selection**

**FIG. 21** shows that the solvent used to rehydrate film can impact transfection efficiency of mRNA LNPs containing the gene sequence for firefly luciferase. All films were prepared with 100 μg/mL of mRNA LNPs in a base formulation of 1.5% K100LV HPMC, 0.5% Pullulan, and 3% glycerol in Tris buffer (pH 8). Films were dried at 22 °C at 60% relative humidity. Once dried, films were rehydrated with the following solvents and transfection efficiency assessed: 1: Fresh Stock (Control for Comparison); 2: Water for Injection (WFI); 3: Buffered Saline; 4: Tris (pH 8); 5: 1% Pluronic F127 in Tris (pH 8); 6: 0.01% Pluronic F127 in Tris; 7: 0.001% Pluronic F127 in Tris; 8: 0.001% Pluronic F68 in Tris (pH 8); 9: 0.5% PEG 1500 in Tris (pH 8); and 10: 0.01% PEG 1500 in Tris.

**L. Manufacturing & Storage Conditions**

Films were dried at 20 °C in chambers set at 52.5 49, 45 and 40% relative humidity. For each condition, films were collected when dry by visual inspection (Sufficient Drying) and an hour later (Extended Drying, **FIG. 58**). The sufficient drying time was reduced by low humidity environments. LNPs in films prepared under the Sufficient Drying protocol were of similar particle size **(FIG. 58A)**. PDI increased as RH decreased (blue dots, **FIG. 58A**). Extending the drying time by 1 hour significantly increased LNP particle size (from 160 nm to 262 nm in the order of decreasing humidity) and PDI (from 0.293 to 0.462) while encapsulation (from 73.5% to 45.5%) and transfection (from 128.6% to 75.2%) efficiencies were significantly reduced **(FIG. 58B)**.

LNPs in films stored at 25 °C for 14 days demonstrated an increase in particle size (193 $\pm$ 29 nm) and PDI (0.199 $\pm$ 0.042) while encapsulation efficiency remained at 81.6 $\pm$ 1.7% and transfection efficiency fell to 2.7 $\pm$ 0.6% **(FIGs. 56A** and **56B)**. LNPs in films stored at - 20 °C did not experience a significant change in physical and biological properties over the 14 day period. The impact of environmental humidity on LNP stability within the film matrix was studied at 4 °C. Relative humidity did not significantly impact particle size, PDI or encapsulation efficiency of LNPs stored within the film matrix **(FIGs. 56C** and **56D)**. Transfection efficiency was compromised after 2 weeks at 4 °C regardless of the relative humidity of the storage chamber (102.9 $\pm$ 11.4%, Fresh Film vs. 24.3-40.0% in the order of decreasing humidity **FIG. 56D)**. Taken together, this data suggests that temperature not relative humidity significantly impacts the transfection efficiency of mRNA LNPs stored within the film matrix.

**M. Optimized LNP-Encapsulated RNA Film Formulations**

Based on the foregoing studies, six optimized film formulations F1-F4 were generated and their impact on LNP-encapsulated mRNA transfection efficiency assessed. Formulations GP, GPT, GPS, KP, KPT, and KPS were as follows:

GP: Gelatin 2% : Pluronic F68 0.01%
GPT: Gelatin 2% : Pluronic F68 0.1%, Trehalose 1%
GPS: Gelatin 2% : Pluronic F68 0.1%, Sucrose 1%
KP: K4M HPMC 2% : Pluronic F68 0.01%
KPT: K4M HPMC 2% : Pluronic F68 0.1%, Trehalose 1%
KPS: K4M HPMC 2% : Pluronic F68 0.1%, Sucrose 1%

All preparations were made in 10 mM Tris buffer pH 8. As shown in **FIG. 22,** all of the above complex formulations improved transfection efficiency of mRNA LNPs containing the gene sequence for beta-galactosidase during the film forming process.

Studies were also conducted to show that the order in which excipients are added to form the optimized films can significantly impact transfection efficiency of mRNA LNPs in the films **(FIG. 24)**. Films were prepared by two methods: bulk or mix. For the bulk method, excipients for film matrix were mixed to create a homogeneous solution. LNPs were then added to the solution, mixed, and formulation added to molds for drying. For the mix method, surfactant or plasticizer components of the film were mixed with LNPs. This mixture was then added to a homogeneous solution of film base polymer and glycerol, mixed, and added to molds for drying. Formulations tested included: 1. 1.5% KLV100 HPMC, 0.01% Pluronic F127, and 3% glycerol in Tris (pH 8), where the "mix" formulation was prepared by adding the Pluronic F127 to the LNPs and then adding that resulting solution to a mixture of KLV100 and glycerol; 2. 1.5% KLV100 HPMC, 0.5% PEG1500 and 3% glycerol in Tris (pH 8), where the "mix" formulation was prepared by adding the PEG1500 to the LNPs and then adding the resulting solution to a mixture of KLV100 and glycerol; 3. 1.5% KLV100 HPMC, 0.1% PMAL C16 and 2% glycerol in Tris (pH 8), where the "mix" formulation was prepared by adding the PMAL to the LNPs and then adding the resulting solution to a mixture of KLV100 and glycerol. For all formulations tested, the mix method, in which surfactant or plasticizer components of the film were mixed with LNPs, resulted in higher transfection efficiency for the mRNA LNPs.

### N. Summary

Described herein is the impact of a variety of parameters associated with incorporation of mRNA LNPs within a film matrix using current, gold standard metrics for LNP performance. Particle size, size distribution (reported as polydispersity index (PDI)) and encapsulation efficiency provide information about the physical characteristics of LNPs and provide insight into the structure and integrity of the particles. Specifications for these parameters can be found in **FIG. 59.**

One hurdle to stabilization of mRNA containing lipid nanoparticles in liquid formulations is keeping the lipid network of the particle in the lowest energy state. As lipid molecules flex, the energy associated with this molecular motion rises, slowly increasing particle size in a manner that supports aggregation, fusion or eventual disruption of the particle. Flex of the lipid molecules and the presence of electrophilic impurities associated with ionizable lipid stock can also initiate lipid degradation, the byproducts of which can accelerate RNA degradation within the particle often at a pace faster than free RNA in solution. This process accelerates as temperature rises and molecular motion increases or when stabilizing water molecules are removed from around the lipid shell during the film forming process. During this process, the phases of the particle can also begin to separate internally, shifting the RNA to the aqueous phase of the particle which accelerates degradation through hydrolysis and oxidation. This can also occur during freezing, however, molecular motion is then suspended in the frozen and/or solid states. When films of optimized formulation (OF at pH 8) were placed at -20 °C, physical and biological parameters remained stable for at least 5 weeks **(FIG. 56, FIG. 61),** however, transfection efficiency was significantly compromised after 2 weeks at 4 °C **(FIG. 56B** and **56D).** *In vivo* activity of LNPs containing self-replicating RNA has been shown to be reduced by 2-4 fold after storage at 4 °C for 7 days despite a superior stability profile at -20 °C. Taken together, this highlights the challenge of stabilizing LNPs at temperatures above freezing and the complex roles excipients fill to maintain LNP stability in the liquid and solid state.

Of the polymers assessed, HPMC did not compromise transfection efficiency during the film forming process **(FIG. 48).** HPMC is unique in that it is available in a variety of grades with different physical and thermal properties with respect to water absorption and water vapor and gas permeabilities. They also vary in molecular weight and hydroxypropyl/methoxy substituents that influence release of materials from films, hydration capacity and emulsion-stabilizing effects. Of the 3 grades tested (A, E, K), grade K HPMC was able to maintain particle shape and transfection efficiency of the LNPs to a higher degree **(FIG. 48).** Interestingly, the K grade contains the lowest methoxy content (19.0 - 24.0%) with a hydroxypropyl content similar to that of the grade E material (7-12%), suggesting that this hydroxypropyl/methoxy substitution ratio is optimal to form a protective matrix around the hydrophilic shells of the LNPs during the drying process and upon rehydration **(FIG. 60** and **FIG. 52).**

Because most mRNA LNP preparations are currently formulated as injectable products, initial formulation development focused on polymers with viscosities ranging from 56-169 cps **(FIG. 58).** Since the lowest viscosity polymer (K100LV) performed poorly alone **(FIG. 52),** it was mixed in various ratios with the highest viscosity polymer (K4M) in an effort to improve stability of the LNPs by preventing aggregation and maintaining particle integrity during the drying process. While all formulations tested improved transfection efficiency of LNPs in freshly prepared films, those containing each polymer alone (K100LV, F20 and K4M, F24) showed an increase in particle size and a drop in encapsulation and transfection efficiency during storage at 4 °C **(FIG. 60, Table 5).** An additional study was initiated to evaluate stability of LNPs in films prepared with the K100LV and K4M polymers individually. In these preparations, a PEG-conjugated lipid was added to the matrix to support particle integrity during the film forming process and long-term storage **(FIG. 52).** The lipid selected for this study is a common component of mRNA LNPs **(FIG. 42).** Various reports have found that the amount of PEG-conjugated lipid included in the LNP structure can significantly impact the bioactivity of a preparation without detectable change in physical properties. LNPs containing 0.5-3% DMPE-PEG lipid in the particle structure were previously evaluated, and it was found that particles containing 1.5% of the lipid had the highest transfection efficiency. It has been reported that particle size decreases as the amount of PEG-conjugated lipid included in the particle increases, however, *in vivo* gene expression is significantly reduced due to steric hinderance between the particles and cellular targets. Extraneous PEG-conjugated lipids added to LNP preparations at concentrations that far exceed the amount normally included in the particle structure (25-60% vs ≤ 15% lipid molar content) have been utilized to maintain particle size during nebulization and improve delivery to the lung. While those concentrations cannot be supported in a film-based dosage form nor an injectable product, a very small amount of the lipid (0.008%) maintained LNP stability while a higher concentration (0.04%) disrupted the particles during the film forming process **(FIG. 51).** One PEG-free lipid was also evaluated as an external excipient. While it could maintain the physical properties of LNPs, transfection efficiency was poor, suggesting that it formed a seal around the LNPs, making them too sturdy to release mRNA for transfection **(FIG. 52).** It is important to note that the concentration of PEG-conjugated lipid used as an external excipient in the formulations herein is significantly lower than 0.02-0.035% w/w, the concentration of PEG-lipid in marketed LNP particles. It does not inhibit cell entry and should not promote any toxic effects such as formation of anti-PEG antibodies as they were not detected in patients receiving multiple doses of LNP-based COVID-19 vaccines. Interestingly, the high viscosity polymer maintained the physical properties of the LNPs within the film matrix better than the low viscosity polymer **(FIG.**

**52).**

Surfactants have been used to produce and stabilize solid lipid nanoparticles to improve permeability and oral bioavailability of small molecule drugs. They are generally added in the aqueous phase of the particle prior to mixing with the lipid phase containing the drug so that they are present on the particle surface. Surfactants are also a common component of film-based dosage forms as they facilitate spreading across the mold surface and wetting of the film during dissolution. They also lower the surface tension of the film matrix which decreases water absorption properties of the HPMC base and makes hydrophilic groups less accessible to water molecules. They have also been shown to play a key role in the thermostabilization and delivery of biological drugs. However, when used as an external excipient, surfactants can easily dissolve mRNA LNPs. Adding Pluronic F68 and Pluronic F127 to the LNP stock to a concentration of 0.006% prior to mixing with the polymer base, though, did not alter the physical and biological properties of LNPs in fresh films **(FIG. 50)** and improved stability during storage at 4 °C **(FIG. 53).** This concentration was useful for maintaining LNP stability as lower concentrations (0.001-0.003%) facilitated LNP aggregation after 14 days at 4 °C (data not shown). Use of other amphipathic, nonionic surfactants such as Tween 20, Tween 80 and Brij 58 were not as effective. This suggests that the poly(ethylene oxide)(PEO)-poly(propylene oxide)(PPO)-poly(ethylene oxide)(PEO) triblock copolymers when used at a concentrations much lower than their critical micelle concentrations (F127, 0.26-0.8 wt%; F68, 0.033-0.4 wt%) form a molecular dispersion of monomers as they are added to the viscous film base and form a network of protective micelles around the particles during the drying process as water is removed from hydrophilic groups. Reconstitution of the film matrix allows the Pluronics to be dispersed as monomers that align themselves with the PEG-lipid and polymer base in a manner that maintains particle size and transfection efficiency of the LNP.

Glycerol is commonly included in film-based products to enhance the pliability and flexibility of the final product. Glycerol is also an established cryoprotectant for cells, tissues, viruses and liposomes through its ability to reduce ice crystal formation and changes in osmotic pressure during freezing. It has been shown to maintain the lipid bilayer of liposomes during drying and can form hydrogen bonds with water and free hydroxide groups on LNPs. Because addition of sugars (i.e. sucrose, trehalose, isomalt) did not impact LNP properties in fresh films nor enhance 4 °C stability of mRNA LNPs, glycerol may protect mRNA LNPs from dehydration and osmotic change during film forming process. It is important to note that the amount of glycerol needed to support LNP stability within the optimized film matrix was higher than that required to stabilize other biomolecules **(FIG. 49).** This paired with the fact that hydrogen bonding between plasticizers like glycerol and polymers like HPMC create pockets available for water molecules highlight the importance of packaging to prevent water absorption from the environment.

Even though a variety of excipients were identified that collectively improve the physical properties of LNPs during drying and storage, transfection efficiency was still compromised. As mentioned earlier, transfection efficiency depends on both intact lipid shells and mRNA. Realizing that the pH of the particle and the film matrix can influence lipid and mRNA degradation, as shown herein, a low pH caused a rapid increase in particle size and loss of transfection in both liquid (pH 6-6.5, **FIG. 45**) and dry formulations (pH 5.5-6.5, **FIG. 47** and **FIG. 54**) due to acid-mediated hydrolysis of lipids and mRNA. While it is known that strong bonds form between mRNA and ionizable lipids within the LNP, this could not prevent degradation during storage at 4 °C **(FIG. 54).** This also explains why mRNA based COVID-19 are formulated at pH 7.5-8 **(Table 3)** although there are some reports suggesting that keeping the pH below the pKa of the lipids is needed to maintain transfection and stability. Increasing the pH of the dry film to ≥7, or preparing the film base at pH 8.5-9.0, delayed acid mediated degradation and significantly improved mRNA LNP stability for 2 weeks at 4 °C **(FIG. 54).**

In addition to formulation design, operation parameters play a notable role in stabilizing mRNA LNPs within a film matrix. Relative humidity of 52.5% showed minimal impact on mRNA LNP characteristics during a normal and extended drying cycle **(FIG. 58).** Lower humidity and longer drying times damaged mRNA LNPs in fresh films as shown by increased particle size and reduced encapsulation and transfection efficiencies. mRNA LNPs contain a significant amount of internal water ($24 \pm 2\%$). Films prepared with the formulations herein contain 22-25% moisture content **(FIG. 57C),** suggesting that the drying process maintains equilibrium between the environment within the LNP and that within the film matrix. Low humidity and overdrying (or formulations with different tonicity) disrupts this balance, causing the LNPs to collapse or swell in response to the disruption. While environmental humidity was previously found to impact the long-term stability of adenovirus, AAV and plasmids at 25 °C and 40 °C within a film matrix, it did not significantly impact mRNA LNP stability at 4 °C **(FIGs. 56C** and **56D).**

The intricate structure of mRNA LNPs requires ultra-low temperature for storage. This posed a significant barrier to rapid and efficient distribution of vaccines during the COVID-19 pandemic. An optimized formulation containing a panel of hydrophilic excipients including HPMC, PEG-lipid, glycerol and surfactants created a preparation with a slightly increased particle size (< 10 nm), a decrease of 5% in encapsulation efficiency and no loss in transfection efficiency **(FIG. 55).** This paired with the fact that LNPs in the range of 60-150 nm retained immunogenicity in non-human primates suggests that a modest increase in LNP is indicative of poor LNP clinical performance. Triblock non-ionic surfactants at specific concentrations can be added to LNP stock prior to addition of film forming polymers to preserve physical properties of LNPs during the film forming process. The pH within the film matrix is important to maintain transfection efficiency.

The multi-component, optimized formulation disclosed herein can prevent damage to the LNP due to physical changes upon dehydration (osmotic stress, surface tension, spatial stress and water loss). They also form a network of hydrogen bonds that preserve the original structure of the LNP. This is the first report in which criteria for stabilization of mRNA LNPs within a film matrix has been extensively characterized.

## O. Exemplary Materials & Methods (Examples 3 and 4)

**Materials.** Dulbecco's phosphate buffer solution (DPBS), Trizma base [2-amino-2-(hydroxymethyl)-1,3-propanediol] (Tris), poly vinyl acetate (PVA), pluronic F127, sucrose and trehalose were purchased from Sigma-Aldrich (St. Louis, MO). Glycerol [USP grade], and Aqualine Complete 5 solvent were purchased from Thermo Fisher Scientific Chemicals (Fair lawn, NJ). Dulbecco's modified Eagle's medium (DMEM), penicillin (10,000 IU) and streptomycin (10,000 μg/ml) and 0.25% Trypsin EDTA sterile solution were purchased from Mediatech (Manassas, VA). Pluronic F68 was purchased from Gibco Life Technologies (Grand Island, NY). Fetal bovine serum was purchased from Mediatech (Corning, Woodlands, CA). Hydranal Formamide Dry was purchased from Honeywell (Charlotte, NC). Methanol, 99.8% extra dry was provided from Acros Organics (Fair Lawn, NJ). Pullulan was obtained from TCI chemicals (Tokyo, Japan). Gelatin powder type A, 300 bloom was purchased from Electron Microscopy Sciences (Hatfield, Pennsylvania). Compounds N-(Methylpolyoxyethylene oxycarbonyl)-1,2-dimyristoyl-sn-glycero-3-phosphoethanolamine sodium salt (DMPE-PEG; Sunbright PM-020CN) 1,2-dimyristoyl-rac-glycero-3-methoxypolyethylene glycol-2000 (DMG-PEG) and 1,2-dimyristoyl-sn-glycero-3-phosphocholine (DMPC) were purchased from NOF America (White Plains, NY) and Avanti Polar Lipids (Alabaster, AL) respectively. Polyethyleneimine (Linear, MW 25000, Transfection Grade) was purchased from Polysciences (Warrington, PA). Lipofectamine™ 2000 CD was purchased from Thermo Fisher Scientific Baltics (Vilnius, Lithuania). All other chemicals were of analytical reagent grade and purchased from Thermo Fisher Scientific (Pittsburgh, PA) unless specified otherwise.

**Plasmid Preparation.** Transfection-grade plasmid (pAAV-LacZ, AAV Helper-Free System Stratagene, La Jolla, CA) was amplified in *E. coli* (HB101 Competent cells, Promega, Madison, WI) and purified using Qiagen Maxi Plasmid kits (Qiagen, Germany). Plasmid stock (OD 260/280 ratio 1.7-1.9) was diluted to a concentration of 1-2 mg/ml with 10 mM Tris buffer pH 8 and stored at -20 °C.

**Preparation and Storage of Films Containing Plasmid Complexes.** PEI-DNA complexes were formed by adding 25 μg pAAV-LacZ in 400 μl to 100 μg PEI in 400 μl media. Lipofectamine (LPF)-DNA complexes were formed by adding 20 μg pAAV-LacZ in 250 μl (total volume) Opti-Pro™ SFM media (Gibco, Thermo Fisher Scientific) to 20 μl of LPF 250 μl (total volume) Opti-Pro™ SFM media. Samples remained at room temperature for 20 minutes. At this time, 800 μl solutions containing PEI-DNA complexes were mixed with 200 μl of film formulation for a final DNA concentration of 25 μg/ml. Solutions containing LPF-DNA complexes were mixed with film formulation at a v:v ratio of 1:1 for a final DNA concentration of 20 μg/ml. The resulting solutions were dispensed into 100 μl silicon molds (Bold Maker, Amesbury, MA). All films were dried under aseptic conditions at 20 °C. When drying was complete, a subset of films were reconstituted in transfection media for analysis of loss due to drying. Remaining films were peeled and placed in Ziploc-like particle-free bags (American Cleanstat, Irvine, CA) inside a heat-sealed foil bag (Ted Pella Inc., Redding, CA). For most stability studies summarized here, packaged films were stored in stability chambers (Binder, Tuttlingen, Germany) set at 25 °C, 60% RH or at 4 °C, 40-50% RH. Details of each formulation are summarized in Supplemental Table 1.

**Transfection (Plasmid).** HEK293 cells (CRL-1573, ATCC, Manassas, VA, passage 12-26) were seeded in 12-well plates (Falcon, Coming, Durham, NC) at a density of $7 \times 10^5$ cells/well. When they were 80% confluent, culture media was changed. One hour later, plasmid (1 μg) in 50 μl of Opti-MEM (Life Technologies, Grand Island, NY) was mixed with 4 μg PEI in 50 μl of Opti-MEM. The resulting solution was incubated at room temperature for 20 minutes prior to addition to cells. For LPF-mediated transfection, plasmid (1 μg) in 12.5 μl Opti-Pro was mixed well with 1 μl LPF in 12.5 μl Opti-Pro before dilution to a concentration of 1 μg pAAV-LacZ /100 μl media per well. Cells were then incubated at 37 °C, 5% $CO_2$ and culture medium replaced every 24 hours. Forty-eight hours after transfection, cells were harvested for assessment of transgene expression.

**Plasmid Transfection Efficiency.** Analysis of beta-galactosidase expression was performed using a colorimetric assay based upon an enzyme-mediated reaction with ortho-nitrophenyl-P-galactoside (ONPG). At the time of harvest, cells were washed with PBS prior to treatment with Reporter Lysis buffer (Promega, Madison, WI). After a freeze/thaw cycle and centrifugation at 14,000 rpm for 1 min, 10 μl of diluted supernatant was mixed with 150 μl of 14.3 M β-mercaptoethanol in 100 mM phosphate buffer at pH 7.5 and held at 37 °C, 5% $CO_2$ for 5 min. At that time, 50 μl of 4 mg/ml ONPG stock was added to the sample. After 2.5 minutes, color development was stopped by adding 90 μl of 1M $Na_2CO_3$. Absorbance at 420 nm for each sample was recorded using a Glomax Multi-detection Plate Reader (Promega, Madison, WI). Protein content of cell lysates was determined by a standard Lowry assay (Bio-Rad, Hercules, California). Transfection efficiency is expressed as the amount beta-galactosidase present per mg cell protein in a given cell population for each formulation with respect to cells transfected with freshly made PEI or LPF complexes prepared from plasmid stored frozen in Tris (pH 8).

**Preparation and Storage of Films Containing mRNA Lipid Nanoparticles** (LNPs). A stock solution of LNPs containing mRNA encoding luciferase in 10% sucrose/Tris buffer (pH 8) was diluted to a concentration of 0.4 mg mRNA per ml with additional Tris buffer. The concentration of mRNA was used to present the concentration of mRNA LNP in a solution. Bulk formulations were prepared by first adding PEG lipids (4% stock in water) to 10 mM Tris buffer to a final concentration of 0.008%. Then a volume of mRNA LNP stock equivalent to 25% of the total bulk formulation volume was added and gently mixed three times. Surfactants, if used in a formulation, were added to the resulting solution with gentle mixing. A mixture of polymer base prepared in 10 mM Tris buffer and glycerol (100%, USP grade) was incorporated last so that the final LNP concentration is 0.1 mg/ml. The complete formulations were dispensed into 100 μl silicone molds **(FIG. 57)** using an E3 Repeater pipette (Eppendorf, Hauppauge, NY) and dried under constant airflow, ambient (20 ± 1.5 °C, 1 atm, 52.5% RH, **FIG. 58)** and aseptic conditions. Temperature and humidity were monitored during the film forming process using an Ambient Weather WS-3000-X5 Wireless Thermo-Hygrometer (Chandler, AZ). Once dry, films were peeled and rehydrated in 100 μl sterile Tris buffer for 10 min at room temperature before gently mixing 10 times to ensure solutions were homogeneous. The resulting solution was then diluted 20-fold in DPBS for transfection, 40-fold in nuclease free Tris-EDTA for encapsulation efficiency and 500-fold in 0.1× DPBS for dynamic light scattering assays. Physical characteristics of the LNP stock are summarized in **FIG. 59.**

**Physical Characterization of LNPs. Dynamic Light Scattering.** Samples were placed in cuvettes and hydrodynamic particle size measured at 25 °C and a 173° backscatter angle using a Zetasizer ZS90 instrument (Malvern Instruments Ltd., Worcestershire, UK). Parameters were set for a particle refractive index of 1.45, absorption of 0.001, diluent viscosity of 0.888 cP and refractive index of 1.335. Data was analyzed with Malvern Zetasizer Software version 8.00.4813.

**Encapsulation efficiency** of LNPs in stock and various formulations was determined using a Quant-it™ RiboGreen Assay kit (Thermo Fisher Scientific, Eugene, OR). The total amount of mRNA in a sample was determined by treating an aliquot of that sample with 0.5% Triton X-100 for 14 minutes. The RiboGreen reagent was added to all samples and fluorescence intensity (excitation at 480nm and emission at 520 nm) measured 5 minutes later. Total and free mRNA concentrations in each samples were calculated using a mRNA standard curve in the range of 15 to 1000 ng/ml. Encapsulation efficiency was determined based on the following equation.

$$Encapsulation\ Efficiency\ (\%) = \frac{Total\ mRNA - Free\ mRNA}{Total\ mRNA} \times 100$$

**Transfection (mRNA LNP) Transgene Expression.** H1 Hela cells (ATCC CRL-1958, passage 6-30) were seeded in 96-well plates (Thermo Fisher Scientific, Waltham, MA) at a density of 2×10⁴ cells/well. Twenty-four hours later, media was replaced an hour before samples containing mRNA LNPs equivalent to 50 ng mRNA was added to each well **(FIG. 59)**. Twenty-four hours later, culture media was replaced with 100 μl of Glo Lysis Buffer (Promega, Madison, WI) and luciferase expression measured using the Pierce™ Firefly Luc One-Step Glow Assay Kit (Thermo Fisher, Rockford, IL) according to the manufacturer's instructions. A portion of the lysate was also used to determine the protein content in each sample by Pierce BCA Protein assay kit (Thermo Fisher, Rockford, IL). Transgene expression in each sample was reported as RLU/mg protein and transfection efficiency calculated using the following equation:

$$\%Transfection\ Efficiency = \frac{RLU\ sample/C_{protein}\ sample}{RLU\ control/C_{protein}\ control} \times 100$$

**Cytotoxicity.** Cytotoxicity of film formulations was evaluated using the CyQUANT™ LDH Cytotoxicity Assay kit (Thermo Fisher, Rockford, IL). Spontaneous LDH activity was determined from cells in standard culture media. Cells treated with lysis buffer were used to determine maximal LDH activity. Media (50 μl) collected from cells at the end of transfection was mixed with 50 μl of Reaction Mixture from the kit. Ten minutes later, the reaction was terminated by addition of Stop Solution and fluorescence (excitation of 560 nm, emission of 590 nm) analyzed immediately using a Glomax Multi-detection Plate Reader (Promega, Madison, WI). LDH activity was recorded as fluorescence intensity subtracting the background fluorescence. Background fluorescence was collected from wells containing culture media in the absence of cells. Cytotoxicity was calculated using the following formula:

$$\%\ Cytotoxicity = \frac{Film\ treated\ LDH\ activity - Spontaneous\ LDH\ activity}{Maximum\ LDH\ activity - Spontaneous\ LDH\ activity} \times 100$$

**Rheology.** Viscosity of formulations was measured using a LVDV Brookfield viscometer (Brookfield AMETEK, Middleboro, MA). The cylinder sample adapter spindle (SC4-21) was lowered into the sample chamber (SC4-13R) filled with 8 grams of formulation. The spindle was rotated at 20 rpm and torque (%) and viscosity (cP) recorded.

**Residual Moisture.** Films were completely dissolved in 1 ml extraction solvent (1: 1 v:v ratio anhydrous formamide: extra dry methanol) at 50 °C. Weight of dry film (m1) and extraction solvent used to dissolve film (m2) were recorded. Moisture content of blank extraction solvent (B%) and sample containing solution (C%) was determined by Karl Fischer Titration using a V10S Volumetric Karl Fischer Titrator (Mettler Toledo, Columbus, OH). Specifically, 0.3 ml of sample was injected to the titration chamber and the sample was mixed with extra dry methanol for 3 minutes before the titration reaction was initiated. The weight of the injected sample was used to calculate moisture content using the following equation and the values described above:

$$Moisture\ content\ (\%) = C\% \times \left(\frac{m1 + m2}{m1}\right) - \left(\frac{B\% \times m2}{m1}\right)$$

**Transmission Electron Microscopy (TEM).** LNPs (0.1 mg/ml) were prepared in Tris buffer, liquid formulations or dried film that was rehydrated prior to analysis. Carbon coated grids (CTU300-CU, Electron Microscopy Sciences, Hatfield, PA) pre-treated with 25 mA plasma for 2 min to make them hydrophilic. Samples (5 $\mu$l of a 1:1 dilution in Tris) were deposited on the grid for 2 minutes. Excess liquid was removed via blotting with filter paper. The sample was washed twice with 5 $\mu$l distilled water. For negative staining, 5 $\mu$l of 1% phosphotungstic acid (pH 7) with excess fluid removed by blotting. Images were collected on a Tecnai 80 kV transmission electron microscope.

## V. References

The following references, and any and all other references cited herein, including literature references, patent applications, patent publications, UniProtKB accession numbers, and GenBank accession numbers, to the extent that they provide exemplary procedural or other details supplementary to those set forth herein, are specifically and expressly incorporated herein by reference, as if each individual reference were specifically and individually indicated to be incorporated by reference in its entirety. When definitions of terms in documents that are incorporated by reference herein conflict with those used herein, the definitions used herein govern.

**Examples 1 & 2:**

1. Lederberg J, Cavalli LL, Lederberg EM. Sex Compatibility in Escherichia Coli. Genetics. 1952;37(6):720-730. doi:10.1093/genetics/37.6.720

2. Lederberg J. Cell genetics and hereditary symbiosis. Physiol Rev. 1952;32(4):403-430. doi:10.1152/physrev.1952.32.4.403

3. Rohwer F, Segall AM. In retrospect: A century of phage lessons. Nature. 2015;528(7580):46-48. doi:10.1038/528046a

4. Cohen SN, Chang AC, Boyer HW, Helling RB. Construction of biologically functional bacterial plasmids in vitro. Proc Natl Acad Sci USA. 1973;70(11):3240-3244. doi: 10.1 073/pnas. 70.11.3240

5. Cohen SN. DNA cloning: A personal view after 40 years. Proc Natl Acad Sci. 2013;110(39):15521-15529. doi:10.1073/pnas.1313397110

6. Chen C, Okayama H. High-efficiency transformation of mammalian cells by plasmid DNA. Mol Cell Biol. 1987;7(8):2745-2752. doi:10.1128/mcb.7.8.2745-2752.1987

7. Southern PJ, Berg P. Transformation of mammalian cells to antibiotic resistance with a bacterial gene under control of the SV40 early region promoter. J Mol Appl Genet. 1982;1(4):327-341.

8. Neumann E, Schaefer-Ridder M, Wang Y, Hofschneider PH. Gene transfer into mouse lyoma cells by electroporation in high electric fields. EMBO J. 1982;1(7):841-845. doi:10.1002/j.1460-2075.1982.tb01257.x

9. Nóbrega, C., Mendonca, L., Matos CA. Gene and Cell Therapy. In: A Handbook of Gene and Cell Therapy. Cham: Springer Nature Switzerland AB; 2020:1-22.

10. Scherman D. Basic Definitions and General Principles. In: Advanced Textbook on Gene Transfer, Gene Therapy and Genetic Pharmacology. Vol 2. World Scientific Publishing, Longdon, UK; 2019:7-16. doi:doi:10.1142/p820

11. Burgess-Brown NA, Mahajan P, Strain-Damerell C, Fernandez-Cid A, Gileadi O, Gräslund S. Screening and Production of Recombinant Human Proteins: Protein Production in E. coli. Methods Mol Biol. 2021;2199:45-66. doi:10.1007/978-1-0716-0892-0_4

12. Carlesso A, Delgado R, Ruiz Isant O, et al. Yeast as a tool for membrane protein production and structure determination. FEMS Yeast Res. 2022;22(1):foac047. doi:10.1093/femsyr/foac047

13. Kamle S, Li D, Lee CG, Elias JA. Chapter 3 - Methods for transient expression and purification of monoclonal antibodies in mammalian cells. In: Tripathi T, Dubey VKBT-A in PM and SBM, eds. Advances in Protein Molecular and Structural Biology Methods. Academic Press; 2022:31-39. doi.org/10.1016/B978-0-323-90264-9.00003-9

14. Wang T-Y, Guo X. Expression vector cassette engineering for recombinant therapeutic production in mammalian cell systems. Appl Microbiol Biotechnol. 2020;104(13):5673-5688. doi:10.1007/s00253-020-10640-w

15. Ramirez GA, Gasmi M. Manufacturing of Viral Gene Therapies. Int Ophthalmol Clin. 2021;61(3):91-112. doi:10.1097/IIO.0000000000000362

16. Dasgupta A, Tinch S, Szczur K, et al. Phase I/II Manufacture of Lentiviral Vectors Under GMP in an Academic Setting. Methods Mol Biol. 2020;2086:27-60. doi:10.1007/978-1-0716-0146-4_3

17. Nguyen TNT, Sha S, Hong MS, et al. Mechanistic model for production of recombinant adeno-associated virus via triple transfection of HEK293 cells. Mol Ther-Methods Clin Dev. 2021;21:642-655. doi:10.1016/j.omtm.2021.04.006

18. Volkova E, Sippert E, Liu M, et al. Validated Reference Panel from Renewable Source of Genomic DNA Available for Standardization of Blood Group Genotyping. J Mol Diagn. 2019;21(3):525-537. doi:10.1016/j.jmoldx.2019.02.003

19. Moldovan E, Moldovan V. Controls in Real-Time Polymerase Chain Reaction Based Techniques. Acta Marisiensis - Ser Medica. 2020;66(3):79-82. doi:doi:10.2478/amma-2020-0025

20. Artika IM, Dewi YP, Nainggolan IM, Siregar JE, Antonjaya U. Real-Time Polymerase Chain Reaction: Current Techniques, Applications, and Role in COVID-19 Diagnosis. Genes (Basel). 2022;13(12). doi:10.3390/genes13122387

21. Meiser LC, Nguyen BH, Chen Y-J, et al. Synthetic DNA applications in information technology. Nat Commun. 2022;13(1):352. doi:10.1038/s41467-021-27846-9

22. Matange K, Tuck JM, Keung AJ. DNA stability: a central design consideration for DNA data storage systems. Nat Commun. 2021;12(1):1358. doi:10.1038/s41467-021-21587-5

23. Park G, Park H, Park S-C, et al. Recent Developments in DNA-Nanotechnology-Powered Biosensors for Zika/Dengue Virus Molecular Diagnostics. Nanomaterials. 2023;13(2). doi:10.3390/nano13020361

24. Kulkarni JA, Witzigmann D, Thomson SB, et al. The current landscape of nucleic acid therapeutics. Nat Nanotechnol. 2021;16(6):630-643. doi:10.1038/s41565-021-00898-0

25. Mohammad R. Key considerations in formulation development for gene therapy products. Drug Discov Today. 2022;27(1):292-303. doi:10.1016/j.drudis.2021.08.013

26. The Journal of Gene Medicine. Gene Therapy Clinical Trials Worldwide. John Wiley and Sons LTD. world-wide-webt at abedia.com/wiley/vectors.php. Published 2021. Accessed March 23, 2023.

27. Ghattas M, Dwivedi G, Lavertu M, Alameh M-G. Vaccine Technologies and Platforms for Infectious Diseases: Current Progress, Challenges, and Opportunities. Vaccines. 2021;9(12). doi:10.3390/vaccines9121490

28. Martinez-Puente DH, Pérez-Trujillo JJ, Zavala-Flores LM, et al. Plasmid DNA for Therapeutic Applications in Cancer. Pharmaceutics. 2022;14(9). doi:10.3390/pharmaceutics14091861

29. Organization WH. COVID-19 - Landscape of Novel Coronavirus Candidate Vaccine Development Worldwide. world-wide-webt at who.int/publications/m/item/draft-landscape-of-covid-19-candidate-vaccines. Published 2022. Accessed March 23, 2023.

30. Pinczak W, Trzcinska S, Kaminski M. Characteristics and Outcomes of Clinical Trials on Gene Therapy in Noncongenital Cardiovascular Diseases: Cross-sectional Study of Three Clinical Trial Registries. JMIR Form Res. 2022;6(4):e33893. doi:10.2196/33893

31. Sergej L'vovich Kiselev, Roman Vadimovich Deev, Ivan Ivanovich Vorob'ev, Nadezhda Aleksandrovna Orlova AAI. Pharmaceutical composition for stimulation of angiogenesis. 2012. patents.google.com/patent/US20150335711A1/en.

32. Sheridan C. First COVID-19 DNA vaccine approved, others in hot pursuit. Nat Biotechnol. 2021;39(12):1479-1482. doi:10.1038/d41587-021-00023-5

33. Khobragade A, Bhate S, Ramaiah V, et al. Efficacy, safety, and immunogenicity of the DNA SARS-CoV-2 vaccine (ZyCoV-D): the interim efficacy results of a phase 3, randomised, double-blind, placebo-controlled study in India. Lancet. 2022;399(10332):1313-1321. doi:10.1016/S0140-6736(22)00151-9

34. Schmeer M, Schleef M, Shankar R, Kobelt D, Walther W. Capillary Gel Electrophoresis (CGE) for Quality Control of Plasmid DNA in Gene Therapy: Quality Control of 20 Years Stored GMP-Grade Plasmid DNA. Methods Mol Biol. 2022;2521:317-328. doi:10.1007/978-1-0716-2441-8-17

35. Thomson JG, Thilmony R. A simple and inexpensive method for sending binary vector plasmid DNA by mail. Plant Biotechnol. 2008;25(4):403-406. doi:10.5511/plantbiotechnology.25.403

36. Schmeer M, Buchholz T, Schleef M. Plasmid DNA Manufacturing for Indirect and Direct Clinical Applications. Hum Gene Ther. 2017;28(10):856-861. doi:10.1089/hum.2017.159

37. Evans RK, Xu Z, Bohannon KE, Wang B, Bruner MW, Volkin DB. Evaluation of degradation pathways for plasmid dna in pharmaceutical formulations via accelerated stability studies. JPharm Sci. 2000;89(1):76-87. doi:10.1002/(SICI)1520-6017(200001)89:1<76: :AID-JPS8>3.0.CO;2-U

38. Armstrong TK, Anchordoquy TJ. Immobilization of nonviral vectors during the freezing step of lyophilization. JPharm Sci. 2004;93(11):2698-2709. doi:10.1002/jps.20177

39. Anchordoquy TJ, Koe GS. Physical Stability of Nonviral Plasmid-Based Therapeutics. JPharm Sci. 2000;89(3):289-296. doi.org/10.1002/(SICI)1520-6017(200003)89:3<289::AID-JPS1>3.0.CO;2-N

40. Anchordoquy TJ, Armstrong TK, Molina M d C. Low molecular weight dextrans stabilize nonviral vectors during lyophilization at low osmolalities: concentrating suspensions by rehydration to reduced volumes. JPharm Sci. 2005;94(6):1226-1236. doi:10.1002/jps.20353

41. Quaak SGL, Haanen JBAG, Beijnen JH, Nuijen B. Naked plasmid DNA formulation: effect of different disaccharides on stability after lyophilisation. AAPS PharmSciTech. 2010;11(1):344-350. doi: 1 0.1208/s12249-01 0-9391-2

42. TechNavio. Global Cold Chain Logistics Market for Pharmaceuticals Industry 2023-2027. Research. world-wide-webt at technavio.com/report/cold-chain-logistics-market-for-pharmaceuticals-industry-analysis. Published 2022. Accessed April 1, 2023.

43. Bajrovic I, Schafer SC, Romanovicz DK, Croyle MA. Novel technology for storage and distribution of live vaccines and other biological medicines at ambient temperature. Sci Adv. 2023;6(10):eaau4819. doi:10.1126/sciadv.aau4819

44. Doan TNK, Le MD, Bajrovic I, et al. Thermostability and in vivo performance of AAV9 in a film matrix. Commun Med. 2022;2(1):148. doi:10.1038/s43856-022-00212-6

45. Choi JH, Jonsson-Schmunk K, Qiu X, et al. A Single Dose Respiratory Recombinant Adenovirus-Based Vaccine Provides Long-Term Protection for Non-Human Primates from Lethal Ebola Infection. Mol Pharm. 2015;12(8):2712-2731. doi:10.1021/mp500646d

46. Schleef M, Schmidt T. Animal-free production of ccc-supercoiled plasmids for research and clinical applications. J Gene Med. 2004;6(SUPPL. 1):45-54. doi:10.1002/jgm.511

47. Bonnet J, Colotte M, Coudy D, et al. Chain and conformation stability of solid-state DNA: implications for room temperature storage. Nucleic Acids Res. 2010;38(5): 1531-1546. doi:10.1093/nar/gkp1060

48. Agilent. Instruction mannual of b-galactosidase assay kit. chem-agilent.com/pdf/strata/200383. pdf.

49. International Organization for Standardization. ISO 10993-5:2009 Biological Evaluation of Medical Devices. Part 5: Tests for In Vitro Cytotoxicity. Geneva, Stwizerland; 2009:9. world-wide-webt at iso.org/standard/36406.html.

50. Fischer K. New procedure for the analytical determination of the water content of liquids and solid bodies. Angew Chem. 1935;48(26):394-396. 10.1002/ange. 19350482605.

51. Bajrovic I, Schafer SC, Romanovicz DK, Croyle MA. Novel technology for storage and distribution of live vaccines and other biological medicines at ambient temperature. Sci Adv. 2020;6(10):eaau4819. doi:10.1126/sciadv.aau4819

52. Lipfert J, Doniach S, Das R, Herschlag D. Understanding nucleic acid-ion interactions. Annu Rev Biochem. 2014;83:813-841. doi:10.1146/annurev-biochem-060409-092720

53. Soult A. Chapter 13: Amino Acids and Proteins. LibreTexts. chem. libretexts.org/Courses/University_of_Kentucky/UK%3A_CHE_103_-_Chemistry_for_Allied_Health_(Soult)/ Chapters/Chapter_13%3A_Amino_Acids_and _Proteins/13.1%3A_Amino_Acids. Published 2021. Accessed March 3, 2023.

54. Heikrujam J, Kishor R, Mazumder PB. The Chemistry Behind Plant DNA Isolation Protocols. In: Boldura O-M, Baltaa C, Awwad NS, eds. *Biochemical Analysis Tools-Methods for Biomolecules Studies.* Rijeka: IntechOpen; 2020 °Ch. 8. doi:10.5772/intechopen.92206

55. Derbyshire E, Obeid R. Choline, Neurological Development and Brain Function: A Systematic Review Focusing on the First 1000 Days. Nutrients. 2020;12(6). doi:10.3390/nu12061731

56. Massant J, Fleurime S, Batens M, Vanhaerents H, Van den Mooter G. Formulating monoclonal antibodies as powders for reconstitution at high concentration using spray-drying: Trehalose/amino acid combinations as reconstitution time reducing and stability improving formulations. Eur JPharm Biopharm. 2020;156:131-142. doi.org/10.1016/j.ejpb.2020.08.019

57. Zhang X, Zhang J, Quan G, Yang P, Pan X, Wu C. The Serum-Resistant Transfection Evaluation and Long-Term Stability of Gene Delivery Dry Powder Based on Mesoporous Silica Nanoparticles and Polyethyleneimine by Freezing-Drying. AAPS PharmSciTech. 2017;18(5):1536-1543. doi:10.1208/s12249-016-0617-9

58. Okamoto H, Nishida S, Todo H, Sakakura Y, Iida K, Danjo K. Pulmonary Gene Delivery by Chitosan-pDNA Complex Powder Prepared by a Supercritical Carbon Dioxide Process. JPharm Sci. 2003;92(2):371-380. doi.org/10.1002/jps. 10285

59. Bakker NAM, de Boer R, Marie C, et al. Small-scale GMP production of plasmid DNA using a simplified and fully disposable production method. J Biotechnol. 2019;306:100007. doi.org/10.1016/j.btecx.2019.100007

60. Li F, Li S, Guo X, et al. Chiral Carbon Dots Mimicking Topoisomerase I To Mediate the Topological Rearrangement of Supercoiled DNA Enantioselectively. Angew Chemie Int Ed. 2020;59(27):11087-11092. doi.org/10.1002/anie.202002904

61. Pogozelski WK, Tullius TD. Oxidative Strand Scission of Nucleic Acids: Routes Initiated by Hydrogen Abstraction from the Sugar Moiety. Chem Rev. 1998;98(3):1089-1108. doi:10.1021/cr960437i

62. An R, Dong P, Komiyama M, Pan X, Liang X. Inhibition of nonenzymatic depurination of nucleic acids by polycations. FEBS Open Bio. 2017;7(11):1707-1714. doi:10.1002/2211-5463.12308

63. An R, Jia Y, Wan B, et al. Non-Enzymatic Depurination of Nucleic Acids: Factors and Mechanisms. PLoS One. 2015;9(12):e115950. doi.org/10.1371/joumal.pone.0115950.

64. Ageno M, Dore E, Frontali C. The alkaline denaturation of DNA. Biophys J. 1969;9(11):1281-1311. doi:10.1016/S0006-3495(69)86452-0

65. Santos T, Pereira P, Queiroz JA, Cruz C, Sousa F. Plasmid production and purification: An integrated experiment-based biochemistry and biotechnology laboratory course. Biochem Mol Biol Educ a Bimon Publ Int Union Biochem Mol Biol. 2019;47(6):638-643. doi:10.1002/bmb.21290

66. Higgins NP, Vologodskii A V. Topological Behavior of Plasmid DNA. Microbiol Spectr. 2015;3(2). doi:10.1128/microbiolspec.PLAS-0036-2014

67. Sousa F, Prazeres DMF, Queiroz JA. Improvement of transfection efficiency by using supercoiled plasmid DNA purified with arginine affinity chromatography. J Gene Med. 2009;11(1):79-88. doi:10.1002/jgm.1272

68. Cherng J-Y, Schuurmans-Nieuwenbroek NME, Jiskoot W, et al. Effect of DNA topology on the transfection efficiency of poly((2-dimethylamino)ethyl methacrylate)-plasmid complexes. J Control Release. 1999;60(2):343-353. doi.org/10.1016/S0168-3659(99)00089-9

69. Cupillard L, Juillard V, Latour S, et al. Impact of plasmid supercoiling on the efficacy of a rabies DNA vaccine to protect cats. Vaccine. 2005;23(16):1910-1916. doi:10.1016/j.vaccine.2004.10.018

70. Contente S, Dubnau D. Characterization of plasmid transformation in Bacillus subtilis: kinetic properties and the effect of DNA conformation. Mol Gen Genet. 1979;167(3):251-258. doi:10.1007/BF00267416

71. Barreira M, Kerridge C, Jorda S, et al. Enzymatically amplified linear dbDNATM as a rapid and scalable solution to industrial lentiviral vector manufacturing. Gene Ther. 2023;30(1):122-131. doi:10.1038/s41434-022-00343-4

72. Maucksch C, Connor B, Rudolph C. Plasmid DNA Concatemers: Influence of Plasmid Structure on Transfection Efficiency. In: Minicircle and Miniplasmid DNA Vectors. ; 2013:59-69. doi.org/10.1002/9783527670420.ch5

73. Adie T, Orefo I, Kysh D, et al. dbDNA™: An advanced platform for genetic medicines. Drug Discov Today. 2022;27(2):374-377. doi:10.1016/j.drudis.2021.09.018

74. Ghanem A, Healey R, Adly FG. Current trends in separation of plasmid DNA vaccines: A review. Anal Chim Acta. 2013;760:1-15. doi.org/10.1016/j.aca.2012.11.006

75. FDA. Guidance for industry: Considerations for plasmid DNA vaccines for infectious disease indications. In: FDA Guidance Documents. ; 2007:2-4. doi:10.1089/blr.2007.9905

76. Olechno K, Basa A, Winnicka K. "Success Depends on Your Backbone"-About the Use of Polymers as Essential Materials Forming Orodispersible Films. Materials (Basel). 2021;14(17):4872. doi:10.3390/ma14174872

77. Whiteman M. Oral Films. In: Specialized Pharmaceutical Formulation: The Science and Technology of Dosage Forms. Rolyal Society of Chemistry, Cambridge, UK; 2022:151-174.

78. Bajrovic I, Le MD, Davis MM, Croyle MA. Evaluation of intermolecular interactions required for thermostability of a recombinant adenovirus within a film matrix. J Control Release. 2021;341:118-131. doi: 10.1016/j.jconrel.2021.11.012

79. Khadir, A., Muthu S. Polymer Technology in Dye-containing Wastewater. Sustainable Textiles: Production, Processing, Manufacturing & Chemistry. In: Environmental Issues Concerned with Poly (Vinyl Alcohol) (PVA) in Textile Wastewater. Springer Singapore; 2022:225-236.

80. Holland BJ, Hay JN. The thermal degradation of poly(vinyl alcohol). Polymer (Guildf). 2001;42(16):6775-6783. doi.org/10.1016/S0032-3861(01)00166-5

81. Lee JC, Timasheff SN. The stabilization of proteins by sucrose. J Biol Chem. 1981;256(14):7193-7201.

82. Ajito S, Iwase H, Takata S, Hirai M. Sugar-Mediated Stabilization of Protein against Chemical or Thermal Denaturation. J Phys Chem B. 2018;122(37):8685-8697. doi:10.1021/acs.jpcb.8b06572

83. Izutsu K-I. Applications of Freezing and Freeze-Drying in Pharmaceutical Formulations. Adv Exp Med Biol. 2018;1081:371-383. doi:10.1007/978-981-13-1244-1_20

84. Castañeda Ruiz AJ, Shetab Boushehri MA, Phan T, et al. Alternative Excipients for Protein Stabilization in Protein Therapeutics: Overcoming the Limitations of Polysorbates. Pharmaceutics. 2022;14(12). doi:10.3390/pharmaceutics14122575

85. Strickley RG, Lambert WJ. A review of Formulations of Commercially Available Antibodies. J Pharm Sci. 2021;110(7):2590-2608.e56. doi.org/10.1016/j.xphs.2021.03.017

86. Kim NA, Hada S, Thapa R, Jeong SH. Arginine as a protein stabilizer and destabilizer in liquid formulations. Int J Pharm. 2016;513(1-2):26-37. doi:10.1016/j.ijpharm.2016.09.003

87. Arakawa T, Kita Y. Multi-faceted arginine: mechanism of the effects of arginine on protein. Curr Protein Pept Sci. 2014;15(6):608-620. doi:10.2174/1389203715061408181130015

88. Chaurasiya B, Zhao Y-Y. Dry Powder for Pulmonary Delivery: A Comprehensive Review. Pharmaceutics. 2021;13(1). doi:10.3390/pharmaceutics13010031

89. Ito T, Okuda T, Takashima Y, Okamoto H. Naked pDNA Inhalation Powder Composed of Hyaluronic Acid Exhibits High Gene Expression in the Lungs. Mol Pharm. 2019;16(2):489-497. doi:10.1021/acs.molpharmaceut.8b00502

90. Schulze J, Kuhn S, Hendrikx S, Schulz-Siegmund M, Polte T, Aigner A. Spray-Dried Nanoparticle-in-Microparticle Delivery Systems (NiMDS) for Gene Delivery, Comprising Polyethylenimine (PEI)-Based Nanoparticles in a Poly(Vinyl Alcohol) Matrix. Small. 2018;14(12): 1701810. doi.org/10.1002/smll.201701810

91. Liang W, Chan AYL, Chow MYT, et al. Spray freeze drying of small nucleic acids as inhaled powder for pulmonary delivery. Asian JPharm Sci. 2018;13(2):163-172. doi.org/10.1016/j.ajps.2017.10.002

92. van der Heijden I, Beijnen JH, Nuijen B. Long term stability of lyophilized plasmid DNA pDERMATT. Int J Pharm. 2013;453(2):648-650. doi.org/10.1016/j.ijpharm.2013.06.010

93. Arya JM, Dewitt K, Scott-Garrard M, Chiang Y-W, Prausnitz MR. Rabies vaccination in dogs using a dissolving microneedle patch. J Control Release. 2016;239:19-26. doi.org/10.1016/j.jconrel.2016.08.012

94. Cui Z, Mumper RJ. Bilayer Films for Mucosal (Genetic) Immunization via the Buccal Route in Rabbits. Pharm Res. 2002;19(7):947-953. doi:10.1023/A:1016454003450

95. Zhang J, Chua LS, Lynn DM. Multilayered Thin Films that Sustain the Release of Functional DNA under Physiological Conditions. Langmuir. 2004;20(19):8015-8021. doi:10.1021/la048888i

96. Yu Y, Si Y, Bechler SL, Liu B, Lynn DM. Polymer Multilayers that Promote the Rapid Release and Contact Transfer of DNA. Biomacromolecules. 2015;16(9):2998-3007. doi:10.1021/acs.biomac.5b00905

97. Xie L, Ding X, Budry R, Mao G. Layer-by-layer DNA films incorporating highly transfecting bioreducible poly(amido amine) and polyethylenimine for sequential gene delivery. Int J Nanomedicine. 2018;13:4943-4960. doi:10.2147/IJN.S162353

98. Jewell CM, Zhang J, Fredin NJ, Wolff MR, Hacker TA, Lynn DM. Release of plasmid DNA from intravascular stents coated with ultrathin multilayered polyelectrolyte films. Biomacromolecules. 2006;7(9):2483-2491. doi:10.1021/bm0604808

99. Teng W, Wang Q, Chen Y, Huang H. Controllably local gene delivery mediated by polyelectrolyte multilayer films assembled from gene-loaded nanopolymersomes and hyaluronic acid. Int J Nanomedicine. 2014;9:5013-5024. doi:10.2147/IJN.S70952

100. Zhang J, Montañez SI, Jewell CM, Lynn DM. Multilayered Films Fabricated from Plasmid DNA and a Side-Chain Functionalized Poly(β-amino Ester): Surface-Type Erosion and Sequential Release of Multiple Plasmid Constructs from Surfaces. Langmuir. 2007;23(22):11139-11146. doi:10.1021/la702021s

**Examples 3 & 4:**

1. Hou X, Zaks T, Langer R, Dong Y. Lipid nanoparticles for mRNA delivery. Nat Rev Mater. 2021;6(12):1078-1094. doi:10.1038/s41578-021-00358-0

2. Guerrini G, Gioria S, Sauer A V, et al. Monitoring Anti-PEG Antibodies Level upon Repeated Lipid Nanoparticle-Based COVID-19 Vaccine Administration. Int J Mol Sci. 2022;23(16). doi:10.3390/ijms23168838

3. Pacouret S, Bouzelha M, Shelke R, et al. AAV-ID: A Rapid and Robust Assay for Batch-to-Batch Consistency Evaluation of AAV Preparations. Mol Ther. 2017;25(6):1375-1386.doi:10.1016/j.ymthe.2017.04.001

4. Qin S, Tang X, Chen Y, et al. mRNA-based therapeutics: powerful and versatile tools to combat diseases. Signal Transduct Target Ther. 2022;7(1):166. doi:10.1038/s41392-022-01007-w

5. Tam YYC, Chen S, Cullis PR. Advances in lipid nanoparticles for siRNA delivery. Pharmaceutics. 2013;5(3):498-507. doi:10.3390/pharmaceutics5030498

6. Han X, Zhang H, Butowska K, et al. An ionizable lipid toolbox for RNA delivery. Nat Commun. 2021;12(1):7233. doi:10.1038/s41467-021-27493-0

7. Sfera A, Hazan S, Anton JJ, et al. Psychotropic drugs interaction with the lipid nanoparticle of COVID-19 mRNA therapeutics. Front Pharmacol. 2022;13:995481. doi:10.3389/fphar.2022.995481

8. Boafo GF, Magar KT, Ekpo MD, Qian W, Tan S, Chen C. The Role of Cryoprotective Agents in Liposome Stabilization and Preservation. Int J Mol Sci. 2022;23(20). doi:10.3390/ijms232012487

9. Zhang H, Leal J, Soto MR, Smyth HDC, Ghosh D. Aerosolizable Lipid Nanoparticles for Pulmonary Delivery of mRNA through Design of Experiments. Pharmaceutics. 2020;12(11). doi:10.3390/pharmaceutics12111042

10. Yanez Arteta M, Kjellman T, Bartesaghi S, et al. Successful reprogramming of cellular protein production through mRNA delivered by functionalized lipid nanoparticles. Proc Natl Acad Sci. 2018;115(15):E3351-E3360. doi:10.1073/pnas.1720542115

11. Klimek L, Novak N, Cabanillas B, Jutel M, Bousquet J, Akdis CA. Allergenic components of the mRNA-1273 vaccine for COVID-19: Possible involvement of polyethylene glycol and IgG-mediated complement activation. Allergy. 2021;76(11):3307-3313. doi.org/10.1111/all.14794

12. Cullis PR, Hope MJ. Lipid Nanoparticle Systems for Enabling Gene Therapies. Mol Ther. 2017;25(7):1467-1475. doi:10.1016/j.ymthe.2017.03.013

13. Li S, Hu Y, Li A, et al. Payload distribution and capacity of mRNA lipid nanoparticles. Nat Commun. 2022;13(1):5561. doi:10.1038/s41467-022-33157-4

14. Kim B, Hosn RR, Remba T, et al. Optimization of storage conditions for lipid nanoparticle-formulated self-replicating RNA vaccines. J Control Release. 2023;353:241-253. doi.org/10.1016/j.jconrel.2022.11.022

15. Oude Blenke E, Ornskov E, Schöneich C, et al. The Storage and In-Use Stability of mRNA Vaccines and Therapeutics: Not A Cold Case. JPharm Sci. 2022. doi.org/10.1016/j.xphs.2022.11.001

16. Schoenmaker L, Witzigmann D, Kulkarni JA, et al. mRNA-lipid nanoparticle COVID-19 vaccines: Structure and stability. Int J Pharm. 2021;601:120586. doi:10.1016/j.ijpharm.2021.120586

17. Moderna COVID-19 Vaccine: Storage and Handling Summary. CDC. world-wide-webt at cdc.gov/vaccines/covid-19/info-by-product/moderna/downloads/storage-summary.pdf. Published 2022.

18. Pfizer-BioNTech COVID-19 Vaccine: Storage and Handling Summary. CDC. world-wide-webt at cdc.gov/vaccines/covid-19/info-by-product/pfizer/downloads/storage-summary.pdf. Published 2022.

19. Muramatsu H, Lam K, Bajusz C, et al. Lyophilization provides long-term stability for a lipid nanoparticle-formulated, nucleoside-modified mRNA vaccine. Mol Ther. 2022;30(5):1941-1951. doi:10.1016/j.ymthe.2022.02.001

20. Ai L, Li Y, Zhou L, et al. Lyophilized mRNA-lipid nanoparticle vaccines with long-term stability and high antigenicity against SARS-CoV-2. Cell Discov. 2023;9(1):9. doi:10.1038/s41421-022-00517-9

21. Czyz MP. Thermostability of Freeze-Dried Plant-Made VLP-Based Vaccines. In: Olvera TPE-J del R, ed. Sustainable Drying Technologies. Rijeka: IntechOpen; 2016:7-36. doi:10.5772/63503

22. Bajrovic I, Schafer SC, Romanovicz DK, Croyle MA. Novel technology for storage and distribution of live vaccines and other biological medicines at ambient temperature. Sci Adv. 2020;6(10):eaau4819. doi:10.1126/sciadv.aau4819

23. Doan TNK, Le MD, Bajrovic I, et al. Thermostability and in vivo performance of AAV9 in a film matrix. Commun Med. 2022;2(1):148. doi:10.1038/s43856-022-00212-6

24. Agilent. Instruction mannual of b-galactosidase assay kit. chem-agilent.com/pdf/strata/200383. pdf.

25. Fischer K. New procedure for the analytical determination of the water content of liquids and solid bodies. Angew Chem. 1935;48(26):394-396. 10.1002/ange. 19350482605.

26. Yang S, Zhou X, Li R, Fu X, Sun P. Optimized PEI-based Transfection Method for Transient Transfection and Lentiviral Production. Curr Protoc Chem Biol. 2017;9(3):147-157. doi:10.1002/cpch.25

27. Cardarelli F, Digiacomo L, Marchini C, et al. The intracellular trafficking mechanism of Lipofectamine-based transfection reagents and its implication for gene delivery. Sci Rep. 2016;6(1):25879. doi:10.1038/srep25879

28. Young ATL, Moore RB, Murray AG, Mullen JC, Lakey JRT. Assessment of Different Transfection Parameters in Efficiency Optimization. Cell Transplant. 2004;13(2):179-185. doi:10.3727/000000004773301861

29. Bajrovic I, Le MD, Davis MM, Croyle MA. Evaluation of intermolecular interactions required for thermostability of a recombinant adenovirus within a film matrix. J Control Release. 2022;341:118-131. doi.org/10.1016/j.jconrel.2021.11.012

30. Tharmalingam T, Goudar CT. Evaluating the impact of high Pluronic® F68 concentrations on antibody producing CHO cell lines. Biotechnol Bioeng. 2015;112(4):832-837. doi:10.1002/bit.25491

31. Kerwin BA. Polysorbates 20 and 80 used in the formulation of protein biotherapeutics: structure and degradation pathways. JPharm Sci. 2008;97(8):2924-2935. doi:10.1002/jps.21190

32. Liu T, Tian Y, Zheng A, Cui C. Design Strategies for and Stability of mRNA-Lipid Nanoparticle COVID-19 Vaccines. Polymers (Basel). 2022;14(19). doi:10.3390/polym14194195

33. Packer M, Gyawali D, Yerabolu R, Schariter J, White P. A novel mechanism for the loss of mRNA activity in lipid nanoparticle delivery systems. Nat Commun. 2021;12(1):6777. doi:10.1038/s41467-021-26926-0

34. Ghadermazi R, Hamdipour S, Sadeghi K, Ghadermazi R, Khosrowshahi Asl A. Effect of various additives on the properties of the films and coatings derived from hydroxypropyl methylcellulose-A review. Food Sci Nutr. 2019;7(11):3363-3377. doi:10.1002/fsn3.1206

35. Olechno K, Basa A, Winnicka K. "Success Depends on Your Backbone"-About the Use of Polymers as Essential Materials Forming Orodispersible Films. Materials (Basel). 2021;14(17):4872. doi:10.3390/ma14174872

36. Panraksa P, Udomsom S, Rachtanapun P, Chittasupho C, Ruksiriwanich W, Jantrawut P. Hydroxypropyl Methylcellulose E15: A Hydrophilic Polymer for Fabrication of Orodispersible Film Using Syringe Extrusion 3D Printer. Polymers (Basel). 2020;12(11). doi:10.3390/polym12112666

37. Krull SM, Ammirata J, Bawa S, Li M, Bilgili E, Davé RN. Critical Material Attributes of Strip Films Loaded With Poorly Water-Soluble Drug Nanoparticles: II. Impact of Polymer Molecular Weight. J Pharm Sci. 2017;106(2):619-628. doi.org/10.1016/j.xphs.2016.10.009

38. Otoni CG, Lorevice M V, Moura MR de, Mattoso LHC. On the effects of hydroxyl substitution degree and molecular weight on mechanical and water barrier properties of hydroxypropyl methylcellulose films. Carbohydr Polym. 2018;185:105-111. doi.org/10.1016/j.carbpol.2018.01.016

39. Ryals RC, Patel S, Acosta C, McKinney M, Pennesi ME, Sahay G. The effects of PEGylation on LNP based mRNA delivery to the eye. PLoS One. 2020;15(10):e0241006. doi:10.1371/journal.pone.0241006

40. Hassett KJ, Higgins J, Woods A, et al. Impact of lipid nanoparticle size on mRNA vaccine immunogenicity. J Control Release. 2021;335:237-246. doi.org/10.1016/j.jconrel.2021.05.021

41. Duong V-A, Nguyen T-T-L, Maeng H-J. Preparation of Solid Lipid Nanoparticles and Nanostructured Lipid Carriers for Drug Delivery and the Effects of Preparation Parameters of Solvent Injection Method. Molecules. 2020;25(20). doi:10.3390/molecules25204781

42. Bajrovic I, Le MD, Davis MM, Croyle MA. Evaluation of intermolecular interactions required for thermostability of a recombinant adenovirus within a film matrix. J Control Release. 2021;341:118-131. doi:10.1016/j.jconrel.2021.11.012

43. Spark Therapeutics Inc. Luxterna (voretigene neparvovec-rzyl) [Package Insert]. 2017. www.fda.gov/medwatch.

44. Novartis Gene Therapies Inc. Zolgensma (onasemnogene abeparvovec-xioi) [Package Insert]. 2021. www.fda.gov/medwatch.

45. Gao Q, Liang Q, Yu F, Xu J, Zhao Q, Sun B. Synthesis and characterization of novel amphiphilic copolymer stearic acid-coupled F127 nanoparticles for nano-technology based drug delivery system. Colloids Surf B Biointerfaces. 2011;88(2):741-748. doi:10.1016/j.colsurfb.2011.08.010

46. Bąk A, Pilarek M, Podgórska W, Markowska-Radomska A, Hubacz R. Surface properties ofperfluorodecalin-containing liquid/liquid systems: The influence of Pluronic F-68 dissolved in the aqueous phase. J Fluor Chem. 2018;215:36-43. doi.org/10.1016/j.j fluchem. 2018.09.002

47. Song Y, Tian Q, Huang Z, et al. Self-assembled micelles of novel amphiphilic copolymer cholesterol-coupled F68 containing cabazitaxel as a drug delivery system. Int J Nanomedicine. 2014;9:2307-2317. doi:10.2147/IJN.S61220

48. Zhang P-Q, Tan P-C, Gao Y-M, et al. The effect of glycerol as a cryoprotective agent in the cryopreservation of adipose tissue. Stem Cell Res Ther. 2022;13(1):152. doi:10.1186/s13287-022-02817-z

49. Larson NR, Hu G, Wei Y, Tuesca AD, Forrest ML, Middaugh CR. pH-Dependent Phase Behavior and Stability of Cationic Lipid-mRNA Nanoparticles. J Pharm Sci. 2022;111(3):690-698. doi.org/10.1016/j.xphs.2021.11.004

All of the methods disclosed and claimed herein can be made and executed without undue experimentation in light of the present disclosure. While the compositions and methods of this disclosure have been described in terms of certain aspects, it will be apparent to those of skill in the art that variations may be applied to the methods and in the steps or in the sequence of steps of the method described herein without departing from the concept, spirit and scope of the disclosure. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the disclosure as defined by the appended claims.

## Claims

1. A composition comprising an agent and a carrier, wherein the carrier comprises a sugar, a zwitterionic compound, and a base polymer.

2. The composition of claim 1, wherein:

   (a) the sugar is melezatose, trehalose, raffinose, sucrose, dextrose, mannitol, sorbitol, cyclodextrin, or a combination thereof and/or the carrier comprises between 0.1% and 3.0% of the sugar;
   (b) the zwitterionic compound is arginine and/or ethylenediaminetetraacetic acid (EDTA) and/or the carrier comprises between 0.1% and 2.0% of the zwitterionic compound;
   (c) the base polymer is hydroxypropyl methylcellulose (HPMC), polyvinyl pyrrolidone (PVP), polyvinyl alcohol (PVA), gelatin, or a combination thereof and/or the carrier comprises between 0.5% and 3.0% of the base polymer;
   (d) the composition comprises:

      i. about 1% trehalose and about 0.5% sucrose, about 1% arginine, and about 2% PVA, or
      ii. about 1% sorbitol, about 1% arginine, and about 2% PVA;

(e) the composition comprises between 0.001 mg/mL and 1 mg/mL of the agent;
(f) the agent is a therapeutic agent;
(g) the agent is a nucleic acid, and optionally:

    i. the nucleic acid is DNA;
    ii. the nucleic acid is DNA at a concentration of between 0.05 mg/mL and 2 g/mL;
    iii.. the nucleic acid is DNA, optionally at a concentration of between 0.05 mg/mL and 2 g/mL, and the DNA is plasmid DNA;
    iv. the nucleic acid is DNA, optionally at a concentration of between 0.05 mg/mL and 2 g/mL, and the DNA is genomic DNA; or
    v. the composition is a composition as defined by of any one of part (g), options i-iv of this claim, and the DNA is complexed with lipofectamine or polyethylenimine;

(h) the carrier further comprises one or more additional excipients, optionally wherein the one or more additional excipients comprise surfactants, plasticizers, additional sugars, and/or additional polymers:
(i) the composition has a pH from 7.0 to 9.0; and/or
(j) the composition:

    i. is a liquid; or
    ii. is a substantially solid film.

3.    A method for storing an agent comprising formulating the agent in a composition of claim 1 or 2, the method comprising storing the agent in the composition for up to 1 year at a temperature of at least 0 °C,
optionally wherein the agent is a nucleic acid, and wherein, after storing, the nucleic acid is preserved by at least 80% as measured by transduction efficiency and/or transfection efficiency.

4.    A therapeutic agent in the composition of claim 1 or 2 for use in medicine, for example by delivering the therapeutic agent to a subject by a method that comprises administering to the subject an effective amount of the composition of claim 1 or 2,
optionally wherein the composition is administered to the subject intravenously, intramuscularly, intranasally, sublingually, or buccally.

5.    A method for making the composition of claim 1 or 2, the method comprising forming an aqueous solution comprising the agent, the sugar, the zwitterionic compound, and the base polymer, and optionally:

(a) wherein the agent and the zwitterionic compound are mixed to form a first mixture, the sugar and the base polymer are mixed to form a second mixture, and the second mixture is added to the first mixture to obtain the aqueous solution, for example wherein the second mixture further comprises a surfactant and a plasticizer mixed with the sugar and the base polymer;
(b) wherein the aqueous solution comprises about 2% PVA, about 1% arginine, about 1% trehalose, and about 0.5% sorbitol, and wherein the aqueous solution has a pH between 8.0 and 9.0;
(c) wherein the aqueous solution comprises about 22% PVA, about 1% arginine, about 1% trehalose, about 0.5% sorbitol, and about 1.5% glycerol, wherein the aqueous solution has a pH between 8.0 and 9.0; and/or
(d) wherein the method further comprises drying the aqueous solution to form a substantially solid film, for example wherein the method further comprises: i. storing the substantially solid film for up to one year at a temperature of at least 0 °C; and ii. dissolving the substantially solid film in an appropriately buffered aqueous solution.

6.    A composition comprising an agent and a carrier, wherein the carrier comprises a plasticizer and/or a surfactant and a base polymer.

7.    The composition of claim 6, wherein:

(a) the plasticizer is glycerol and/or the carrier comprises between 1% and 5% of the plasticizer;
(b) the surfactant is a poloxamer and/or the carrier comprises between 0.0001% and 3.0% of the surfactant, optionally wherein the poloxamer is poloxamer 188 and/or poloxamer 407;
(c) the base polymer is hydroxypropyl methylcellulose (HPMC), polyvinyl pyrrolidone (PVP), polyvinyl alcohol (PVA), gelatin, or a combination thereof and/or the carrier comprises between 0.5% and 3.0% of the base polymer, optionally wherein the HPMC has a molecular weight (MW) that produces a viscosity that is about 4000 cp or less at a concentration of 2% in water, and/or optionally wherein the HPMC is A4C, A4M, A15C, A15LV, E4M, E6LV, F4M, K4M, or K100LV;

(d) the composition further comprises a pegylated lipid, optionally wherein the pegylated lipid comprises DMPE-PEG and/or DMG-PEG;

(e) the composition comprises about 1.5% HPMC, about 0.01% poloxamer, and about 3% glycerol, optionally wherein the HPMC is HPMC K100LV and/or optionally the poloxamer is poloxamer 407;

(f) the composition comprises about 1% HPMC, about 0.012% poloxamer, about 3% glycerol, and about 0.008% pegylated lipid, optionally wherein the HPMC is K4M, the poloxamer is poloxamer 188 and poloxamer 407, and/or optionally the pegylated lipid is DMPE-PEG;

(g) the composition comprises between 0.001 mg/mL and 1 mg/mL of the agent;

(h) the agent is a therapeutic agent;

(i) the agent is a nucleic acid, and optionally:

    i. the nucleic acid is RNA, for example wherein the RNA is mRNA;
    ii. the nucleic acid is RNA, for example wherein the RNA is mRNA, and the RNA is at a concentration of between 0.001 mg/mL and 1 mg/mL; or
    iii. the composition is a composition as defined by of any one of part (i), option i or ii of this claim, and wherein the RNA is encapsulated in a lipid nanoparticle (LNP);

(j) the carrier further comprises one or more additional excipients, optionally wherein the one or more additional excipients are sugars, choline, phosphocholine, calcium D-heptagluconate dihydrate, additional surfactants, additional plasticizers, and/or additional polymers;

(k) the composition has a pH from 7.0 to 9.0; and/or

(l) the composition is:

    i. a liquid; or
    ii. is a substantially solid film.

8. A method for storing an agent comprising formulating the agent in a composition of claim 6 or 7, the method comprising storing the agent in the composition for up to 12 weeks at a temperature of at least 0 °C, optionally wherein the agent is a nucleic acid, and wherein, after storing, the nucleic acid is preserved by at least 80% as measured by transduction efficiency and/or transfection efficiency.

9. The composition of claim 6 or 7 for use in medicine, for example by delivering an agent to a subject by a method comprising administering to the subject an effective amount of the composition of claim 6 or 7, optionally wherein the composition is administered to the subject intravenously, intramuscularly, intranasally, sublingually, or buccally.

10. A method for making the composition of claim 6 or 7, the method comprising forming an aqueous solution comprising the agent, the plasticizer, the surfactant, and the base polymer by:

mixing the agent with the surfactant to form a first mixture;
mixing the plasticizer with the base polymer to form a second mixture; and
adding the second mixture to the first mixture to obtain the aqueous solution.

11. The method of claim 10, wherein:

(a) the aqueous solution comprises about 1.5% HPMC, about 0.01% poloxamer, and about 3% glycerol, and wherein the aqueous solution has a pH between 7.5 and 8.5; and/or

(b) the method further comprises mixing the agent with a pegylated lipid before mixing the agent with the surfactant to form the first mixture,

optionally wherein the aqueous solution comprises about 1% HPMC, about 0.012% poloxamer, about 3% glycerol, about 0.008% pegylated lipid, and wherein the aqueous solution has a pH between 7.5 and 9.5.

12. The method of claim 10 or 11, further comprising:

drying the aqueous solution, optionally, under constant airflow, to form a substantially solid film; and
storing the substantially solid film up to 16 weeks at a temperature of at least 0 °C; and
rehydrating the substantially solid film in an appropriately buffered aqueous solution.

13. The method of claim 12, wherein the appropriately buffered aqueous solution comprises buffered saline or a poloxamer, and optionally wherein the poloxamer is poloxamer 188 and/or poloxamer 407.

14. The method of claim 13, wherein the poloxamer is buffered in Tris at about pH 8.

15. The method of claim 13 or 14, wherein the poloxamer is at a concentration of between 0.0001% and 1.0%.

## FIG. 1

## FIGs. 2A-C

**A**

**B**

**C**

## FIG. 3

## FIG. 4

## FIGs. 5A-D

## FIGs. 6A-C

## FIG. 7

## FIG. 8

## FIGs. 9A-B

## FIGs. 10A-B

## FIG. 11

## FIGs. 12A-B

## FIGs. 13A-B

## FIG. 14

## FIG. 15

## FIGs. 16A-C

## FIG. 17

## FIGs. 18A-B

## FIGs. 19A-B

## FIG. 20

## FIG. 21

## FIG. 22

## FIG. 23

## FIG. 24

# FIGs. 25A-E

# FIGs. 26A-C

**a.**

| | | Basic amino acids | | | | | | Acidic amino acids | | | | Neutral amino acids | | | | | |
|---|---|---|---|---|---|---|---|---|---|---|---|---|---|---|---|---|---|
| | Control | aa1 | | aa2 | | aa3 | | aa4 | | aa5 | | aa6 | | aa7 | | aa8 | |
| | -20°C | 50°C | 1% | 0.1% | 1% | 0.1% | 1% | 0.1% | 1% | 0.1% | 1% | 0.1% | 1% | 0.1% | 1% | 0.1% | 1% | 0.1% |

**b.**

| Standard | | Control | | aa1 | | aa2 | | aa3 | |
|---|---|---|---|---|---|---|---|---|---|
| Linear | Nicked | -20°C | 50°C | 1% | 0.1% | 1% | 0.1% | 1% | 0.1% |

**c.**

| Group | Amino acid | pI | Charge | pH |
|---|---|---|---|---|
| Basic aa | aa1 | 9.47 | - | 9.92 |
| | aa2 | 10.76 | + | 10.14 |
| | aa3 | 7.64 | - | 8.07 |
| Acidic aa | aa4 | 3.08 | - | 3.45 |
| | aa5 | 2.98 | - | 3.37 |
| Neutral aa | aa6 | 5.65 | - | 7.66 |
| | aa7 | 6.11 | - | 8.04 |
| | aa8 | 5.15 | + | 1.77 |

## FIGs. 27A-F

## FIGs. 28A-F

## FIGs. 29A-D

# FIGs. 30A-C

# FIGs. 31A-D

## FIGs. 32A-D

a.

b.

c.

d.

## FIGs. 33A-B

## FIGs. 34A-D

## FIGs. 35A-C

## FIGs. 36A-D

## FIGs. 37A-C

## FIGs. 38A-B

a.

| | | | | | | | | | | | | | |
|---|---|---|---|---|---|---|---|---|---|---|---|---|---|
| Nicked Linear SC | | | | | | | | | | | | | |
| Tris | F1 | F2 | F3 | F4 | F1 | F2 | F3 | F4 | F1 | F2 | F3 | F4 | Tris |
| -20C | 4 weeks | | | | 8 weeks | | | | 12 weeks | | | | |

b.

## FIG. 39

## FIGs. 40A-D

### a.

### b.

| Formulation | Tg (°C) | Heat capacity (J/g.C) | pH fresh film | pH 25°C 8 weeks |
|---|---|---|---|---|
| Film P2 | 143.74 | 1.346 | 6.5 | 6.5 |
| Film P2 + Sug1 | 142.12 | 1.745 | 6.5 | 6.5 |
| Film P2 + Sug2 | 142.66 | 1.463 | 7 | 7 |
| Film P2 + Sug3 | 143.64 | 0.9988 | 6.5 | 6.0 |
| Film P2 + Sug4 | 149.40 | 1.68 | 6.5 | 6.5 |
| Film P2 + Sug5 | 143.61 | 2.552 | 6.5 | 5.5 |
| Film P2 + Sug6 | 95.98 | 0.9297 | 7 | 7 |
| Film P2 + Sug7 | 147.03 | 1.164 | 6.5 | 6.5 |
| PAST | 130.45 | 0.3694 | 8 | 8 |

### c.

### d.

| Formulation | Tg (°C) | Heat capacity (J/g.C) | pH fresh film | pH 25°C 8 weeks |
|---|---|---|---|---|
| P | 143.74 | 1.346 | 6.5 | 6.5 |
| PT | 142.66 | 1.463 | 6.5 | 7 |
| PS | 147.03 | 1.164 | 6.5 | 6 |
| PA | 131.47 | 0.916 | 8 | 8 |
| PAST | 130.45 | 0.3694 | 8 | 8 |
| PAST + pDNA | 128.77 | 0.3493 | 8 | 8 |

## FIGs. 41A-B

### a. UV-Vis

### b. Nanodrop

## FIG. 42

Ionizable amino acid

DSPC

PEG-lipid

Cholesterol

Targeting ligand

## FIG. 43

## FIG. 44

## FIGs. 45A-C

## FIGs. 46A-D

## FIGs. 47A-D

## FIGs. 48A-B

# FIGs. 49A-B

**a.**

**b.**

# FIGs. 50A-B

**a.**

**b.**

## FIGs. 51A-D

## FIGs. 52A-D

# FIGs. 53A-D

## FIGs. 54A-D

## FIGs. 55A-D

## FIGs. 56A-D

## FIGs. 57A-F

# FIGs. 58A-B

## a.

## b.

# FIGs. 59A-B

## a.

| Characteristics | Value |
|---|---|
| Size | 136 ± 2.1 nm |
| Charge | -11.5 ± 1.1 mV |
| PDI | 0.055 ± 0.007 |
| Encapsulation efficiency | 88.8 − 91.4% |

## b.

## FIGs. 60A-D

## FIGs. 61A-B

## FIGs. 62A-C

## FIGs. 63A-D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63466053
- US 63411460
- WO 2012018628 A
- US 20190298836

**Non-patent literature cited in the description**

- Remington's Pharmaceutical Sciences. Mack Printing Company, 1990, 1289-1329
- Remington's Pharmaceutical Sciences. Mack Printing Company, 1990
- **LEDERBERG J ; CAVALLI LL ; LEDERBERG EM.** Sex Compatibility in Escherichia Coli. *Genetics,* 1952, vol. 37 (6), 720-730
- **LEDERBERG J.** Cell genetics and hereditary symbiosis. *Physiol Rev.,* 1952, vol. 32 (4), 403-430
- **ROHWER F ; SEGALL AM.** In retrospect: A century of phage lessons. *Nature,* 2015, vol. 528 (7580), 46-48
- **COHEN SN ; CHANG AC ; BOYER HW ; HELLING RB.** Construction of biologically functional bacterial plasmids in vitro. *Proc Natl Acad Sci USA.,* 1973, vol. 70 (11), 3240-3244
- **COHEN SN.** DNA cloning: A personal view after 40 years. *Proc Natl Acad Sci.,* 2013, vol. 110 (39), 15521-15529
- **CHEN C ; OKAYAMA H.** High-efficiency transformation of mammalian cells by plasmid DNA. *Mol Cell Biol.,* 1987, vol. 7 (8), 2745-2752
- **SOUTHERN PJ ; BERG P.** Transformation of mammalian cells to antibiotic resistance with a bacterial gene under control of the SV40 early region promoter. *J Mol Appl Genet.,* 1982, vol. 1 (4), 327-341
- **NEUMANN E ; SCHAEFER-RIDDER M ; WANG Y ; HOFSCHNEIDER PH.** Gene transfer into mouse lyoma cells by electroporation in high electric fields. *EMBO J.,* 1982, vol. 1 (7), 841-845
- Gene and Cell Therapy. **NÓBREGA, C. ; MENDONCA, L. ; MATOS CA.** A Handbook of Gene and Cell Therapy. Springer Nature, 2020, 1-22
- Basic Definitions and General Principles. **SCHERMAN D.** Advanced Textbook on Gene Transfer, Gene Therapy and Genetic Pharmacology. World Scientific Publishing, 2019, vol. 2, 7-16
- **BURGESS-BROWN NA ; MAHAJAN P ; STRAIN-DAMERELL C ; FERNANDEZ-CID A ; GILEADI O ; GRÄSLUND S.** Screening and Production of Recombinant Human Proteins: Protein Production in E. coli. *Methods Mol Biol.,* 2021, vol. 2199, 45-66
- **CARLESSO A ; DELGADO R ; RUIZ ISANT O et al.** Yeast as a tool for membrane protein production and structure determination. *FEMS Yeast Res.,* 2022, vol. 22 (1), foac047
- Methods for transient expression and purification of monoclonal antibodies in mammalian cells. **KAMLE S ; LI D ; LEE CG ; ELIAS JA.** Advances in Protein Molecular and Structural Biology Methods. Academic Press, 2022, 31-39
- **WANG T-Y ; GUO X.** Expression vector cassette engineering for recombinant therapeutic production in mammalian cell systems. *Appl Microbiol Biotechnol.,* 2020, vol. 104 (13), 5673-5688
- **RAMIREZ GA ; GASMI M.** Manufacturing of Viral Gene Therapies. *Int Ophthalmol Clin.,* 2021, vol. 61 (3), 91-112
- **DASGUPTA A ; TINCH S ; SZCZUR K et al.** Phase I/II Manufacture of Lentiviral Vectors Under GMP in an Academic Setting. *Methods Mol Biol.,* 2020, vol. 2086, 27-60
- **NGUYEN TNT ; SHA S ; HONG MS et al.** Mechanistic model for production of recombinant adeno-associated virus via triple transfection of HEK293 cells. *Mol Ther-Methods Clin Dev.,* 2021, vol. 21, 642-655
- **VOLKOVA E ; SIPPERT E ; LIU M et al.** Validated Reference Panel from Renewable Source of Genomic DNA Available for Standardization of Blood Group Genotyping. *J Mol Diagn.,* 2019, vol. 21 (3), 525-537
- **MOLDOVAN E ; MOLDOVAN V.** Controls in Real-Time Polymerase Chain Reaction Based Techniques. *Acta Marisiensis - Ser Medica.,* 2020, vol. 66 (3), 79-82
- **ARTIKA IM ; DEWI YP ; NAINGGOLAN IM ; SIREGAR JE ; ANTONJAYA U.** Real-Time Polymerase Chain Reaction: Current Techniques, Applications, and Role in COVID-19 Diagnosis. *Genes (Basel).,* 2022, vol. 13 (12
- **MEISER LC ; NGUYEN BH ; CHEN Y-J et al.** Synthetic DNA applications in information technology. *Nat Commun.,* 2022, vol. 13 (1), 352

- **MATANGE K ; TUCK JM ; KEUNG AJ.** DNA stability: a central design consideration for DNA data storage systems. *Nat Commun,* 2021, vol. 12 (1), 1358
- **PARK G ; PARK H ; PARK S-C et al.** Recent Developments in DNA-Nanotechnology-Powered Biosensors for Zika/Dengue Virus Molecular Diagnostics. *Nanomaterials,* 2023, vol. 13 (2
- **KULKARNI JA ; WITZIGMANN D ; THOMSON SB et al.** The current landscape of nucleic acid therapeutics. *Nat Nanotechnol.,* 2021, vol. 16 (6), 630-643
- **MOHAMMAD R.** Key considerations in formulation development for gene therapy products. *Drug Discov Today.,* 2022, vol. 27 (1), 292-303
- Gene Therapy Clinical Trials Worldwide. The Journal of Gene Medicine. John Wiley and Sons LTD, 2021
- **GHATTAS M ; DWIVEDI G ; LAVERTU M ; ALAMEH M-G.** Vaccine Technologies and Platforms for Infectious Diseases: Current Progress, Challenges, and Opportunities. *Vaccines,* 2021, vol. 9 (12
- **MARTINEZ-PUENTE DH ; PÉREZ-TRUJILLO JJ ; ZAVALA-FLORES LM et al.** Plasmid DNA for Therapeutic Applications in Cancer. *Pharmaceutics,* 2022, vol. 14 (9
- *COVID-19 - Landscape of Novel Coronavirus Candidate Vaccine Development Worldwide,* 2022
- **PINCZAK W ; TRZCINSKA S ; KAMINSKI M.** Characteristics and Outcomes of Clinical Trials on Gene Therapy in Noncongenital Cardiovascular Diseases: Cross-sectional Study of Three Clinical Trial Registries. *JMIR Form Res.,* 2022, vol. 6 (4), e33893
- **SERGEJ L'VOVICH KISELEV ; ROMAN VADI-MOVICH DEEV ; IVAN IVANOVICH VOROB'EV ; NADEZHDA ALEKSANDROVNA ORLOVA AAI.** *Pharmaceutical composition for stimulation of angiogenesis,* 2012
- **SHERIDAN C.** First COVID-19 DNA vaccine approved, others in hot pursuit. *Nat Biotechnol.,* 2021, vol. 39 (12), 1479-1482
- **KHOBRAGADE A ; BHATE S ; RAMAIAH V et al.** Efficacy, safety, and immunogenicity of the DNA SARS-CoV-2 vaccine (ZyCoV-D): the interim efficacy results of a phase 3, randomised, double-blind, placebo-controlled study in India. *Lancet.,* 2022, vol. 399 (10332), 1313-1321
- **SCHMEER M ; SCHLEEF M ; SHANKAR R ; KOBELT D ; WALTHER W.** Capillary Gel Electrophoresis (CGE) for Quality Control of Plasmid DNA in Gene Therapy: Quality Control of 20 Years Stored GMP-Grade Plasmid DNA. *Methods Mol Biol.,* 2022, vol. 2521, 317-328
- **THOMSON JG ; THILMONY R.** A simple and inexpensive method for sending binary vector plasmid DNA by mail. *Plant Biotechnol.,* 2008, vol. 25 (4), 403-406
- **SCHMEER M ; BUCHHOLZ T ; SCHLEEF M.** Plasmid DNA Manufacturing for Indirect and Direct Clinical Applications. *Hum Gene Ther.,* 2017, vol. 28 (10), 856-861
- **EVANS RK ; XU Z ; BOHANNON KE ; WANG B ; BRUNER MW ; VOLKIN DB.** Evaluation of degradation pathways for plasmid dna in pharmaceutical formulations via accelerated stability studies. *JPharm Sci.,* 2000, vol. 89 (1), 76-87
- **ARMSTRONG TK ; ANCHORDOQUY TJ.** Immobilization of nonviral vectors during the freezing step of lyophilization. *JPharm Sci.,* 2004, vol. 93 (11), 2698-2709
- **ANCHORDOQUY TJ ; KOE GS.** Physical Stability of Nonviral Plasmid-Based Therapeutics. *JPharm Sci.,* 2000, vol. 89 (3), 289-296
- **ANCHORDOQUY TJ ; ARMSTRONG TK ; MOLINA M D C.** Low molecular weight dextrans stabilize nonviral vectors during lyophilization at low osmolalities: concentrating suspensions by rehydration to reduced volumes. *JPharm Sci.,* 2005, vol. 94 (6), 1226-1236
- **QUAAK SGL ; HAANEN JBAG ; BEIJNEN JH ; NU-IJEN B.** Naked plasmid DNA formulation: effect of different disaccharides on stability after lyophilisation. *AAPS PharmSciTech.,* 2010, vol. 11 (1), 344-350
- TechNavio. Global Cold Chain Logistics Market for Pharmaceuticals Industry 2023-2027. *Research,* 2022
- **BAJROVIC I ; SCHAFER SC ; ROMANOVICZ DK ; CROYLE MA.** Novel technology for storage and distribution of live vaccines and other biological medicines at ambient temperature. *Sci Adv,* 2023, vol. 6 (10), eaau4819
- **DOAN TNK ; LE MD ; BAJROVIC I et al.** Thermostability and in vivo performance of AAV9 in a film matrix. *Commun Med.,* 2022, vol. 2 (1), 148
- **CHOI JH ; JONSSON-SCHMUNK K ; QIU X et al.** A Single Dose Respiratory Recombinant Adenovirus-Based Vaccine Provides Long-Term Protection for Non-Human Primates from Lethal Ebola Infection. *Mol Pharm.,* 2015, vol. 12 (8), 2712-2731
- **SCHLEEF M ; SCHMIDT T.** Animal-free production of ccc-supercoiled plasmids for research and clinical applications. *J Gene Med.,* 2004, vol. 6 (1), 45-54
- **BONNET J ; COLOTTE M ; COUDY D et al.** Chain and conformation stability of solid-state DNA: implications for room temperature storage. *Nucleic Acids Res.,* 2010, vol. 38 (5), 1531-1546
- Agilent. Instruction mannual of b-galactosidase assay kit
- *ISO 10993-5:2009 Biological Evaluation of Medical Devices. Part 5: Tests for In Vitro Cytotoxicity. Geneva, Stwizerland,* 2009, vol. 9

- **FISCHER K.** New procedure for the analytical determination of the water content of liquids and solid bodies. *Angew Chem.,* 1935, vol. 48 (26), 394-396
- **BAJROVIC I ; SCHAFER SC ; ROMANOVICZ DK ; CROYLE MA.** Novel technology for storage and distribution of live vaccines and other biological medicines at ambient temperature. *Sci Adv.,* 2020, vol. 6 (10), eaau4819
- **LIPFERT J ; DONIACH S ; DAS R ; HERSCHLAG D.** Understanding nucleic acid-ion interactions. *Annu Rev Biochem.,* 2014, vol. 83, 813-841
- **SOULT A.** Amino Acids and Proteins. 2021
- **DERBYSHIRE E ; OBEID R. CHOLINE.** Neurological Development and Brain Function: A Systematic Review Focusing on the First 1000 Days. *Nutrients,* 2020, vol. 12 (6
- **MASSANT J ; FLEURIME S ; BATENS M ; VANHAERENTS H ; VAN DEN MOOTER G.** Formulating monoclonal antibodies as powders for reconstitution at high concentration using spray-drying: Trehalose/amino acid combinations as reconstitution time reducing and stability improving formulations. *Eur JPharm Biopharm.,* 2020, vol. 156, 131-142
- **ZHANG X ; ZHANG J ; QUAN G ; YANG P ; PAN X ; WU C.** The Serum-Resistant Transfection Evaluation and Long-Term Stability of Gene Delivery Dry Powder Based on Mesoporous Silica Nanoparticles and Polyethyleneimine by Freezing-Drying. *AAPS PharmSciTech.,* 2017, vol. 18 (5), 1536-1543
- **OKAMOTO H ; NISHIDA S ; TODO H ; SAKAKURA Y ; IIDA K ; DANJO K.** Pulmonary Gene Delivery by Chitosan-pDNA Complex Powder Prepared by a Supercritical Carbon Dioxide Process. *JPharm Sci.,* 2003, vol. 92 (2), 371-380
- **BAKKER NAM ; DE BOER R ; MARIE C et al.** Small-scale GMP production of plasmid DNA using a simplified and fully disposable production method. *J Biotechnol.,* 2019, vol. 306, 100007
- **LI F ; LI S ; GUO X et al.** Chiral Carbon Dots Mimicking Topoisomerase I To Mediate the Topological Rearrangement of Supercoiled DNA Enantioselectively. *Angew Chemie Int Ed.,* 2020, vol. 59 (27), 11087-11092
- **POGOZELSKI WK ; TULLIUS TD.** Oxidative Strand Scission of Nucleic Acids: Routes Initiated by Hydrogen Abstraction from the Sugar Moiety. *Chem Rev.,* 1998, vol. 98 (3), 1089-1108
- **AN R ; DONG P ; KOMIYAMA M ; PAN X ; LIANG X.** Inhibition of nonenzymatic depurination of nucleic acids by polycations. *FEBS Open Bio.,* 2017, vol. 7 (11), 1707-1714
- **AN R ; JIA Y ; WAN B et al.** Non-Enzymatic Depurination of Nucleic Acids: Factors and Mechanisms. *PLoS One.,* 2015, vol. 9 (12), e115950
- **AGENO M ; DORE E ; FRONTALI C.** The alkaline denaturation of DNA. *Biophys J.,* 1969, vol. 9 (11), 1281-1311
- **SANTOS T ; PEREIRA P ; QUEIROZ JA ; CRUZ C ; SOUSA F.** Plasmid production and purification: An integrated experiment-based biochemistry and biotechnology laboratory course. *Biochem Mol Biol Educ a Bimon Publ Int Union Biochem Mol Biol.,* 2019, vol. 47 (6), 638-643
- **HIGGINS NP ; VOLOGODSKII A V.** Topological Behavior of Plasmid DNA. *Microbiol Spectr.,* 2015, vol. 3 (2
- **SOUSA F ; PRAZERES DMF ; QUEIROZ JA.** Improvement of transfection efficiency by using supercoiled plasmid DNA purified with arginine affinity chromatography. *J Gene Med.,* 2009, vol. 11 (1), 79-88
- **CHERNG J-Y ; SCHUURMANS-NIEUWENBROEK NME ; JISKOOT W et al.** Effect of DNA topology on the transfection efficiency of poly((2-dimethylamino)ethyl methacrylate)-plasmid complexes. *J Control Release.,* 1999, vol. 60 (2), 343-353
- **CUPILLARD L ; JUILLARD V ; LATOUR S et al.** Impact of plasmid supercoiling on the efficacy of a rabies DNA vaccine to protect cats. *Vaccine.,* 2005, vol. 23 (16), 1910-1916
- **CONTENTE S ; DUBNAU D.** Characterization of plasmid transformation in Bacillus subtilis: kinetic properties and the effect of DNA conformation. *Mol Gen Genet.,* 1979, vol. 167 (3), 251-258
- **BARREIRA M ; KERRIDGE C ; JORDA S et al.** Enzymatically amplified linear dbDNATM as a rapid and scalable solution to industrial lentiviral vector manufacturing. *Gene Ther.,* 2023, vol. 30 (1), 122-131
- **MAUCKSCH C ; CONNOR B ; RUDOLPH C.** Plasmid DNA Concatemers: Influence of Plasmid Structure on Transfection Efficiency. *Minicircle and Miniplasmid DNA Vectors,* 2013, 59-69
- **ADIE T ; OREFO I ; KYSH D et al.** dbDNA™: An advanced platform for genetic medicines. *Drug Discov Today,* 2022, vol. 27 (2), 374-377
- **GHANEM A ; HEALEY R ; ADLY FG.** Current trends in separation of plasmid DNA vaccines: A review. *Anal Chim Acta.,* 2013, vol. 760, 1-15
- Guidance for industry: Considerations for plasmid DNA vaccines for infectious disease indications. *FDA Guidance Documents,* 2007, 2-4
- **OLECHNO K ; BASA A ; WINNICKA K.** Success Depends on Your Backbone''-About the Use of Polymers as Essential Materials Forming Orodispersible Films. *Materials (Basel).,* 2021, vol. 14 (17), 4872
- Oral Films. **WHITEMAN M.** Specialized Pharmaceutical Formulation: The Science and Technology of Dosage Forms. Rolyal Society of Chemistry, 2022, 151-174

- **BAJROVIC I ; LE MD ; DAVIS MM ; CROYLE MA.** Evaluation of intermolecular interactions required for thermostability of a recombinant adenovirus within a film matrix. *J Control Release.,* 2021, vol. 341, 118-131
- Polymer Technology in Dye-containing Wastewater. Sustainable Textiles: Production, Processing, Manufacturing & Chemistry. **KHADIR, A. ; MUTHU S.** Environmental Issues Concerned with Poly (Vinyl Alcohol) (PVA) in Textile Wastewater. Springer, 2022, 225-236
- **HOLLAND BJ ; HAY JN.** The thermal degradation of poly(vinyl alcohol). *Polymer (Guildf).,* 2001, vol. 42 (16), 6775-6783
- **LEE JC ; TIMASHEFF SN.** The stabilization of proteins by sucrose. *J Biol Chem.,* 1981, vol. 256 (14), 7193-7201
- **AJITO S ; IWASE H ; TAKATA S ; HIRAI M.** Sugar-Mediated Stabilization of Protein against Chemical or Thermal Denaturation. *J Phys Chem B.,* 2018, vol. 122 (37), 8685-8697
- **IZUTSU K-I.** Applications of Freezing and Freeze-Drying in Pharmaceutical Formulations. *Adv Exp Med Biol.,* 2018, vol. 1081, 371-383
- **CASTAÑEDA RUIZ AJ ; SHETAB BOUSHEHRI MA ; PHAN T et al.** Alternative Excipients for Protein Stabilization in Protein Therapeutics: Overcoming the Limitations of Polysorbates. *Pharmaceutics,* 2022, vol. 14 (12
- **STRICKLEY RG ; LAMBERT WJ.** A review of Formulations of Commercially Available Antibodies. *JPharm Sci.,* 2021, vol. 110 (7), 2590-2608.e56
- **KIM NA ; HADA S ; THAPA R ; JEONG SH.** Arginine as a protein stabilizer and destabilizer in liquid formulations. *Int J Pharm.,* 2016, vol. 513 (1-2), 26-37
- **ARAKAWA T ; KITA Y.** Multi-faceted arginine: mechanism of the effects of arginine on protein. *Curr Protein Pept Sci.,* 2014, vol. 15 (6), 608-620
- **CHAURASIYA B ; ZHAO Y-Y.** Dry Powder for Pulmonary Delivery: A Comprehensive Review. *Pharmaceutics,* 2021, vol. 13 (1
- **ITO T ; OKUDA T ; TAKASHIMA Y ; OKAMOTO H.** Naked pDNA Inhalation Powder Composed of Hyaluronic Acid Exhibits High Gene Expression in the Lungs. *Mol Pharm.,* 2019, vol. 16 (2), 489-497
- **SCHULZE J ; KUHN S ; HENDRIKX S ; SCHULZ-SIEGMUND M ; POLTE T ; AIGNER A.** Spray-Dried Nanoparticle-in-Microparticle Delivery Systems (NiMDS) for Gene Delivery, Comprising Polyethylenimine (PEI)-Based Nanoparticles in a Poly(Vinyl Alcohol) Matrix. *Small,* 2018, vol. 14 (12), 1701810
- **LIANG W ; CHAN AYL ; CHOW MYT et al.** Spray freeze drying of small nucleic acids as inhaled powder for pulmonary delivery. *Asian JPharm Sci.,* 2018, vol. 13 (2), 163-172
- **VAN DER HEIJDEN I ; BEIJNEN JH ; NUIJEN B.** Long term stability of lyophilized plasmid DNA pDERMATT. *Int J Pharm.,* 2013, vol. 453 (2), 648-650
- **ARYA JM ; DEWITT K ; SCOTT-GARRARD M ; CHIANG Y-W ; PRAUSNITZ MR.** Rabies vaccination in dogs using a dissolving microneedle patch. *J Control Release.,* 2016, vol. 239, 19-26
- **CUI Z ; MUMPER RJ.** Bilayer Films for Mucosal (Genetic) Immunization via the Buccal Route in Rabbits. *Pharm Res.,* 2002, vol. 19 (7), 947-953
- **ZHANG J ; CHUA LS ; LYNN DM.** Multilayered Thin Films that Sustain the Release of Functional DNA under Physiological Conditions. *Langmuir.,* 2004, vol. 20 (19), 8015-8021
- **YU Y ; SI Y ; BECHLER SL ; LIU B ; LYNN DM.** Polymer Multilayers that Promote the Rapid Release and Contact Transfer of DNA. *Biomacromolecules.,* 2015, vol. 16 (9), 2998-3007
- **XIE L ; DING X ; BUDRY R ; MAO G.** Layer-by-layer DNA films incorporating highly transfecting bioreducible poly(amido amine) and polyethylenimine for sequential gene delivery. *Int J Nanomedicine.,* 2018, vol. 13, 4943-4960
- **JEWELL CM ; ZHANG J ; FREDIN NJ ; WOLFF MR ; HACKER TA ; LYNN DM.** Release of plasmid DNA from intravascular stents coated with ultrathin multilayered polyelectrolyte films. *Biomacromolecules.,* 2006, vol. 7 (9), 2483-2491
- **TENG W ; WANG Q ; CHEN Y ; HUANG H.** Controllably local gene delivery mediated by polyelectrolyte multilayer films assembled from gene-loaded nanopolymersomes and hyaluronic acid. *Int J Nanomedicine.,* 2014, vol. 9, 5013-5024
- **ZHANG J ; MONTAÑEZ SI ; JEWELL CM ; LYNN DM.** Multilayered Films Fabricated from Plasmid DNA and a Side-Chain Functionalized Poly(β-amino Ester): Surface-Type Erosion and Sequential Release of Multiple Plasmid Constructs from Surfaces. *Langmuir.,* 2007, vol. 23 (22), 11139-11146
- **HOU X ; ZAKS T ; LANGER R ; DONG Y.** Lipid nanoparticles for mRNA delivery. *Nat Rev Mater.,* 2021, vol. 6 (12), 1078-1094
- **GUERRINI G ; GIORIA S ; SAUER A V et al.** Monitoring Anti-PEG Antibodies Level upon Repeated Lipid Nanoparticle-Based COVID-19 Vaccine Administration. *Int J Mol Sci.,* 2022, vol. 23 (16
- **PACOURET S ; BOUZELHA M ; SHELKE R et al.** AAV-ID: A Rapid and Robust Assay for Batch-to-Batch Consistency Evaluation of AAV Preparations. *Mol Ther.,* 2017, vol. 25 (6), 1375-1386
- **QIN S ; TANG X ; CHEN Y et al.** mRNA-based therapeutics: powerful and versatile tools to combat diseases. *Signal Transduct Target Ther.,* 2022, vol. 7 (1), 166

- **TAM YYC ; CHEN S ; CULLIS PR.** Advances in lipid nanoparticles for siRNA delivery. *Pharmaceutics.,* 2013, vol. 5 (3), 498-507
- **HAN X ; ZHANG H ; BUTOWSKA K et al.** An ionizable lipid toolbox for RNA delivery. *Nat Commun.,* 2021, vol. 12 (1), 7233
- **SFERA A ; HAZAN S ; ANTON JJ et al.** Psychotropic drugs interaction with the lipid nanoparticle of COVID-19 mRNA therapeutics. *Front Pharmacol.,* 2022, vol. 13, 995481
- **BOAFO GF ; MAGAR KT ; EKPO MD ; QIAN W ; TAN S ; CHEN C.** The Role of Cryoprotective Agents in Liposome Stabilization and Preservation. *Int J Mol Sci.,* 2022, vol. 23 (20
- **ZHANG H ; LEAL J ; SOTO MR ; SMYTH HDC ; GHOSH D.** Aerosolizable Lipid Nanoparticles for Pulmonary Delivery of mRNA through Design of Experiments. *Pharmaceutics,* 2020, vol. 12 (11
- **YANEZ ARTETA M ; KJELLMAN T ; BARTESAGHI S et al.** Successful reprogramming of cellular protein production through mRNA delivered by functionalized lipid nanoparticles. *Proc Natl Acad Sci.,* 2018, vol. 115 (15), E3351-E3360
- **KLIMEK L ; NOVAK N ; CABANILLAS B ; JUTEL M ; BOUSQUET J ; AKDIS CA.** Allergenic components of the mRNA-1273 vaccine for COVID-19: Possible involvement of polyethylene glycol and IgG-mediated complement activation. *Allergy.,* 2021, vol. 76 (11), 3307-3313
- **CULLIS PR ; HOPE MJ.** Lipid Nanoparticle Systems for Enabling Gene Therapies. *Mol Ther.,* 2017, vol. 25 (7), 1467-1475
- **LI S ; HU Y ; LI A et al.** Payload distribution and capacity of mRNA lipid nanoparticles. *Nat Commun.,* 2022, vol. 13 (1), 5561
- **KIM B ; HOSN RR ; REMBA T et al.** Optimization of storage conditions for lipid nanoparticle-formulated self-replicating RNA vaccines. *J Control Release.,* 2023, vol. 353, 241-253
- **OUDE BLENKE E ; ORNSKOV E ; SCHÖNEICH C et al.** The Storage and In-Use Stability of mRNA Vaccines and Therapeutics: Not A Cold Case. *JPharm Sci.,* 2022
- **SCHOENMAKER L ; WITZIGMANN D ; KULKARNI JA et al.** mRNA-lipid nanoparticle COVID-19 vaccines: Structure and stability. *Int J Pharm.,* 2021, vol. 601, 120586
- Moderna COVID-19 Vaccine: Storage and Handling Summary. CDC, 2022
- Pfizer-BioNTech COVID-19 Vaccine: Storage and Handling Summary. CDC, 2022
- **MURAMATSU H ; LAM K ; BAJUSZ C et al.** Lyophilization provides long-term stability for a lipid nanoparticle-formulated, nucleoside-modified mRNA vaccine. *Mol Ther.,* 2022, vol. 30 (5), 1941-1951

- **AI L ; LI Y ; ZHOU L et al.** Lyophilized mRNA-lipid nanoparticle vaccines with long-term stability and high antigenicity against SARS-CoV-2. *Cell Discov,* 2023, vol. 9 (1), 9
- Thermostability of Freeze-Dried Plant-Made VLP-Based Vaccines. **CZYZ MP.** Sustainable Drying Technologies. IntechOpen, 2016, 7-36
- **DOAN TNK ; LE MD ; BAJROVIC I et al.** Thermostability and in vivo performance of AAV9 in a film matrix. *Commun Med.,* 2022, vol. 2 (1), 148
- **FISCHER K.** New procedure for the analytical determination of the water content of liquids and solid bodies. *Angew Chem.,* 1935, vol. 48 (26), 394-396
- **YANG S ; ZHOU X ; LI R ; FU X ; SUN P.** Optimized PEI-based Transfection Method for Transient Transfection and Lentiviral Production. *Curr Protoc Chem Biol.,* 2017, vol. 9 (3), 147-157
- **CARDARELLI F ; DIGIACOMO L ; MARCHINI C et al.** The intracellular trafficking mechanism of Lipofectamine-based transfection reagents and its implication for gene delivery. *Sci Rep.,* 2016, vol. 6 (1), 25879
- **YOUNG ATL ; MOORE RB ; MURRAY AG ; MULLEN JC ; LAKEY JRT.** Assessment of Different Transfection Parameters in Efficiency Optimization. *Cell Transplant.,* 2004, vol. 13 (2), 179-185
- **BAJROVIC I ; LE MD ; DAVIS MM ; CROYLE MA.** Evaluation of intermolecular interactions required for thermostability of a recombinant adenovirus within a film matrix. *J Control Release.,* 2022, vol. 341, 118-131
- **THARMALINGAM T ; GOUDAR CT.** Evaluating the impact of high Pluronic® F68 concentrations on antibody producing CHO cell lines. *Biotechnol Bioeng,* 2015, vol. 112 (4), 832-837
- **KERWIN BA.** Polysorbates 20 and 80 used in the formulation of protein biotherapeutics: structure and degradation pathways. *JPharm Sci.,* 2008, vol. 97 (8), 2924-2935
- **LIU T ; TIAN Y ; ZHENG A ; CUI C.** Design Strategies for and Stability of mRNA-Lipid Nanoparticle COVID-19 Vaccines. *Polymers (Basel).,* 2022, vol. 14 (19
- **PACKER M ; GYAWALI D ; YERABOLU R ; SCHARITER J ; WHITE P.** A novel mechanism for the loss of mRNA activity in lipid nanoparticle delivery systems. *Nat Commun.,* 2021, vol. 12 (1), 6777
- **GHADERMAZI R ; HAMDIPOUR S ; SADEGHI K ; GHADERMAZI R ; KHOSROWSHAHI ASL A.** Effect of various additives on the properties of the films and coatings derived from hydroxypropyl methylcellulose-A review. *Food Sci Nutr.,* 2019, vol. 7 (11), 3363-3377

- **PANRAKSA P ; UDOMSOM S ; RACHTANAPUN P ; CHITTASUPHO C ; RUKSIRIWANICH W ; JANTRAWUT P.** Hydroxypropyl Methylcellulose E15: A Hydrophilic Polymer for Fabrication of Orodispersible Film Using Syringe Extrusion 3D Printer. *Polymers (Basel).,* 2020, vol. 12 (11
- **KRULL SM ; AMMIRATA J ; BAWA S ; LI M ; BILGILI E ; DAVÉ RN.** Critical Material Attributes of Strip Films Loaded With Poorly Water-Soluble Drug Nanoparticles: II. Impact of Polymer Molecular Weight. *J Pharm Sci.,* 2017, vol. 106 (2), 619-628
- **OTONI CG ; LOREVICE M V ; MOURA MR DE ; MATTOSO LHC.** On the effects of hydroxyl substitution degree and molecular weight on mechanical and water barrier properties of hydroxypropyl methylcellulose films. *Carbohydr Polym.,* 2018, vol. 185, 105-111
- **RYALS RC ; PATEL S ; ACOSTA C ; MCKINNEY M ; PENNESI ME ; SAHAY G.** The effects of PEGylation on LNP based mRNA delivery to the eye. *PLoS One.,* 2020, vol. 15 (10), e0241006
- **HASSETT KJ ; HIGGINS J ; WOODS A et al.** Impact of lipid nanoparticle size on mRNA vaccine immunogenicity. *J Control Release.,* 2021, vol. 335, 237-246
- **DUONG V-A ; NGUYEN T-T-L ; MAENG H-J.** Preparation of Solid Lipid Nanoparticles and Nanostructured Lipid Carriers for Drug Delivery and the Effects of Preparation Parameters of Solvent Injection Method. *Molecules,* 2020, vol. 25 (20
- *Luxterna (voretigene neparvovec-rzyl),* 2017, www.fda.gov/medwatch.
- *Zolgensma (onasemnogene abeparvovec-xioi),* 2021, www.fda.gov/medwatch
- **GAO Q ; LIANG Q ; YU F ; XU J ; ZHAO Q ; SUN B.** Synthesis and characterization of novel amphiphilic copolymer stearic acid-coupled F127 nanoparticles for nano-technology based drug delivery system. *Colloids Surf B Biointerfaces.,* 2011, vol. 88 (2), 741-748
- **SONG Y ; TIAN Q ; HUANG Z et al.** Self-assembled micelles of novel amphiphilic copolymer cholesterol-coupled F68 containing cabazitaxel as a drug delivery system. *Int J Nanomedicine.,* 2014, vol. 9, 2307-2317
- **ZHANG P-Q ; TAN P-C ; GAO Y-M et al.** The effect of glycerol as a cryoprotective agent in the cryopreservation of adipose tissue. *Stem Cell Res Ther.,* 2022, vol. 13 (1), 152
- **LARSON NR ; HU G ; WEI Y ; TUESCA AD ; FORREST ML ; MIDDAUGH CR.** pH-Dependent Phase Behavior and Stability of Cationic Lipid-mRNA Nanoparticles. *J Pharm Sci.,* 2022, vol. 111 (3), 690-698